# EUROPEAN PATENT APPLICATION

(11) **EP 4 219 469 A1**
(43) Date of publication of application: **02.08.2023**
(21) Application number: 21872232.0
(22) Date of filing: 13.09.2021
(51) Int. Cl.: C07D 403/04, C08G 61/02, C09K 11/06, C07D 251/24, H05B 33/12, H01L 51/50

(54) **LIGHT-EMITTING ELEMENT AND COMPOSITION**

(30) Priority: 24.09.2020 JP 2020160083
(71) Applicant: SUMITOMO CHEMICAL COMPANY, LIMITED, Tokyo 103-6020 (JP)
(72) Inventor: SASADA, Toshiaki, Tsukuba-shi, Ibaraki 300-3294 (JP); ASANO, Atsushi, Tokyo 103-6020 (JP)
(74) Representative: J A Kemp LLP
(86) International application number: PCT/JP2021/033490
(87) International publication number: WO 2022/065102

(57) **Abstract**

To provide a light emitting device excellent in maximum light emission efficiency.

A light emitting device having an anode and a cathode, and a first layer and a second layer disposed between the anode and the cathode, in which the first layer is a layer containing at least one compound (B-1) selected from compounds represented by the formula (B), the second layer is a layer containing at least one compound (B-2) selected from compounds represented by the formula (B), and a crosslinked body of a compound having a cross-linkable group, and at least one of the compound (B-1) and the compound (B-2) is a compound represented by the formula (B) in which n^{B1} is 1 or more.

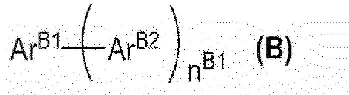

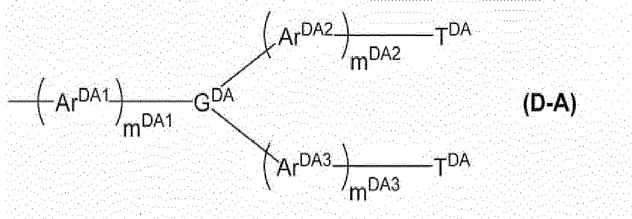

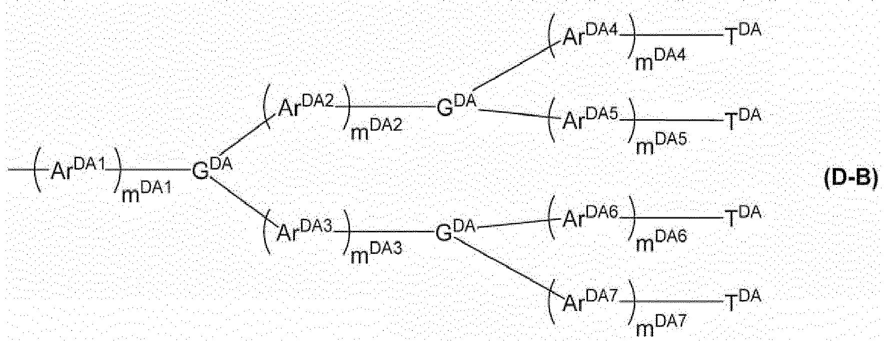

## Description

### Technical Field

The present invention relates to a light emitting device and a composition.

### Background Art

Light emitting devices such as an organic electroluminescent device and the like can be suitably used for applications of display and illumination.

For example, Patent Document 1 describes a light emitting device having a hole transporting layer composed only of a crosslinked body of a cross-linkable material, and a light emitting layer containing a compound B-H1 and a metal complex B-G1.

For example, Patent Document 2 describes a light emitting device having a layer composed only of a crosslinked body of a cross-linkable material, and a layer containing a compound B-H2 and a metal complex B-G2.

For example, Patent Document 3 describes a light emitting device having a layer containing a crosslinked body of a cross-linkable material and a metal complex B-G1, and a layer containing a metal complex B-G1. Note that the metal complex B-G1 is a compound not corresponding to a compound (B') described later.

For example, Patent Document 4 describes a light emitting device having a layer containing a crosslinked body of a cross-linkable material and a compound B-H8, and a layer containing a compound B-H8. Note that the compound B-H8 is a compound not corresponding to a compound (B') described later.

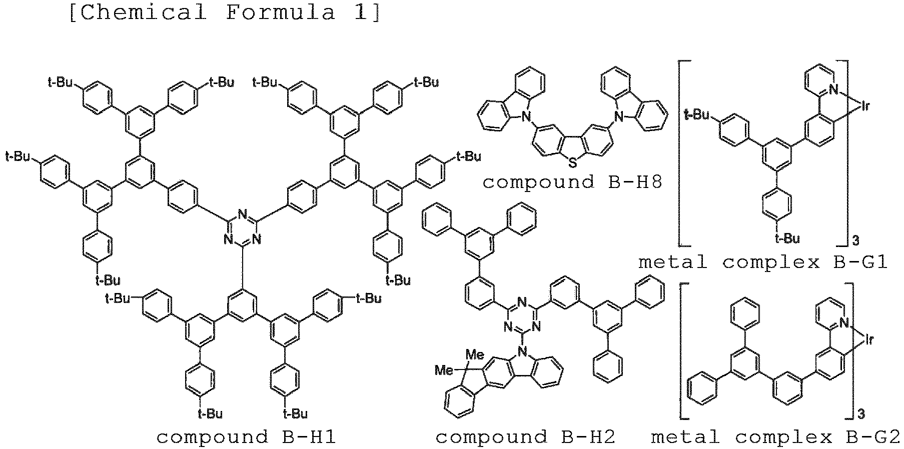

### [Prior Art Document]

### [Patent Document]

[Patent Document 1] International Publication WO2016/170671
[Patent Document 2] International Publication WO2019/049225
[Patent Document 3] International Publication WO2015/186539
[Patent Document 4] International Publication WO2018/198975

### Summary of the Invention

### Problem to be Solved by the Invention

However, the light emitting device described above is not necessarily sufficient in maximum light emission efficiency.

Then, a main object of the present invention is to provide a light emitting device excellent in maximum light emission efficiency.

### Means for Solving the Problem

The present invention provides the following [1] to [25] .
[1] A light emitting device having an anode and a cathode, and a first layer and a second layer disposed between the above-described anode and the above-described cathode, wherein
   the above-described first layer is a layer containing at least one compound (B-1) selected from compounds represented by the formula (B),
   the above-described second layer is a layer containing at least one compound (B-2) selected from compounds represented by the formula (B), and a crosslinked body of a compound having a cross-linkable group, and
   at least one of the above-described compound (B-1) and the above-described compound (B-2) is a compound represented by the formula (B) in which n^{B1} is 1 or more: 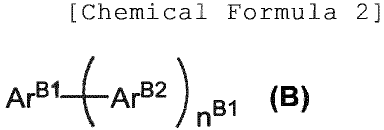 [wherein,
      n^{B1} represents an integer of 0 or more.
      Ar^{B1} represents a group obtained by removing from an aromatic hydrocarbon n^{B1} hydrogen atoms bonding directly to carbon atoms constituting the ring, a group obtained by removing from a heterocyclic compound n^{B1} hydrogen atoms bonding directly to atoms constituting the ring or a group obtained by removing n^{B1} hydrogen atoms from a metal complex represented by the formula (1), and these groups optionally have a substituent. When a plurality of the substituents are present, they may be the same or different and may be combined together to form a ring together with atoms to which they are attached.
      Ar^{B2} represents a group represented by the formula (D-A) or a group represented by the formula (D-B). When a plurality of Ar^{B2} are present, they may be the same or different.] 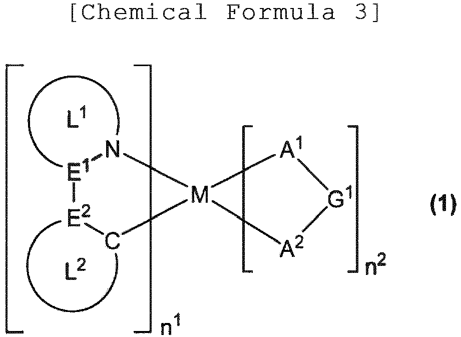
   [wherein,
   M represents a rhodium atom, a palladium atom, an iridium atom or a platinum atom.
   n¹ represents an integer of 1 or more, and n² represents an integer of 0 or more. Under this condition, n¹+n² is 3 when M is a rhodium atom or an iridium atom, while n¹+n² is 2 when M is a palladium atom or a platinum atom.
   E¹ and E² each independently represent a carbon atom or a nitrogen atom. When a plurality of E¹ and E² are present, they may be the same or different at each occurrence.
   Ring L¹ represents an aromatic hetero ring, and the ring optionally has a substituent. When a plurality of the substituents are present, they may be the same or different and may be combined together to form a ring together with atoms to which they are attached. When a plurality of Ring L¹ are present, they may be the same or different.
   Ring L² represents an aromatic hydrocarbon ring or an aromatic hetero ring, and these rings optionally have a substituent. When a plurality of the substituents are present, they may be the same or different and may be combined together to form a ring together with atoms to which they are attached. When a plurality of Ring L² are present, they may be the same or different.
   A substituent which Ring L¹ optionally has and a substituent which Ring L² optionally has may be the same or different and may be combined together to form a ring together with atoms to which they are attached.
   A¹-G¹-A² represents an anionic bidentate ligand. A¹ and A² each independently represent a carbon atom, an oxygen atom or a nitrogen atom, and these atoms may be ring-constituent atoms. G¹ represents a single bond, or an atomic group constituting a bidentate ligand together with A¹ and A². When a plurality of A¹-G¹-A² are present, they may be the same or different.] 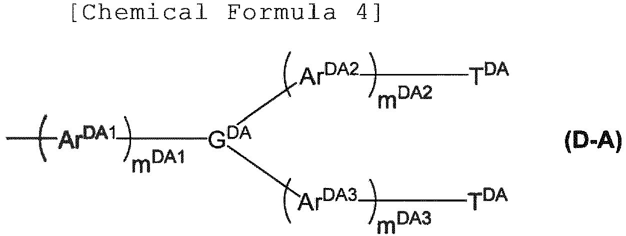
   [wherein,
   m^{DA1} represents an integer of 1 or more.
   m^{DA2} and m^{DA3} each independently represent an integer of 0 or more.
   G^{DA} represents a nitrogen atom, an aromatic hydrocarbon group or a hetero ring group, and these groups optionally have a substituent. When a plurality of the substituents are present, they may be the same or different and may be combined together to form a ring together with atoms to which they are attached.
   Ar^{DA1}, Ar^{DA2} and Ar^{DA3} each independently represent an arylene group or a divalent hetero ring group, and these groups optionally have a substituent. When a plurality of the substituents are present, they may be the same or different and may be combined together to form a ring together with atoms to which they are attached. When a plurality of Ar^{DA1}, Ar^{DA2} and Ar^{DA3} are present, they may be the same or different at each occurrence.
   T^{DA} represents an aryl group or a monovalent hetero ring group, and these groups optionally have a substituent. When a plurality of the substituents are present, they may be the same or different and may be combined together to form a ring together with atoms to which they are attached. A plurality of T^{DA} may be the same or different.] 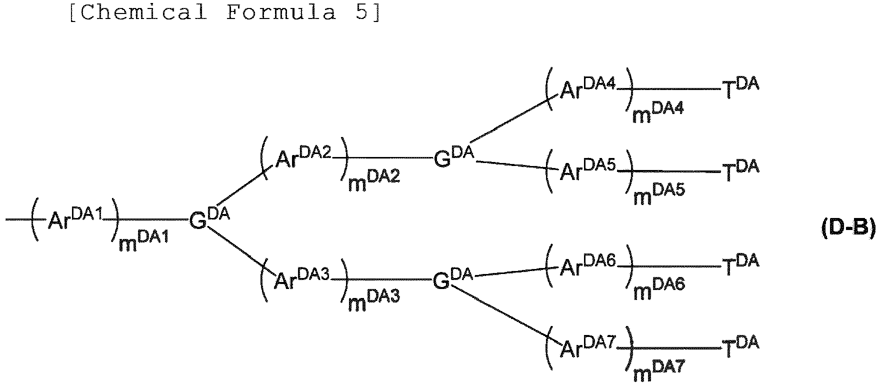
   [wherein,
   m^{DA1} represents an integer of 1 or more.
   m^{DA2} , m^{DA3}, m^{DA4}, m^{DA5}, m^{DA6} and m^{DA7} each independently represent an integer of 0 or more.
   G^{DA} represents a nitrogen atom, an aromatic hydrocarbon group or a hetero ring group, and these groups optionally have a substituent. When a plurality of the substituents are present, they may be the same or different and may be combined together to form a ring together with atoms to which they are attached. A plurality of G^{DA} may be the same or different.
   Ar^{DA1}, Ar^{DA2}, Ar^{DA3}, Ar^{DA4}, Ar^{DA5}, Ar^{DA6} and Ar^{DA7} each independently represent an arylene group or a divalent hetero ring group, and these groups optionally have a substituent. When a plurality of the substituents are present, they may be the same or different and may be combined together to form a ring together with atoms to which they are attached. When a plurality of Ar^{DA1}, Ar^{DA2}, Ar^{DA3}, Ar^{DA4}, Ar^{DA5}, Ar^{DA6} and Ar^{DA7} are present, they may be the same or different at each occurrence.
   T^{DA} represents an aryl group or a monovalent hetero ring group, and these groups optionally have a substituent. When a plurality of the substituents are present, they may be the same or different and may be combined together to form a ring together with atoms to which they are attached. A plurality of T^{DA} may be the same or different.].
[2] The light emitting device according to [1], wherein the above-described G^{DA} is an aromatic hydrocarbon group or a hetero ring group, and these groups optionally have a substituent.
[3] The light emitting device according to [2], wherein the above-described G^{DA} is a group obtained by removing from a monocyclic, bicyclic or a tricyclic aromatic hydrocarbon three hydrogen atoms bonding directly to carbon atoms constituting the ring or a group obtained by removing from a monocyclic, bicyclic or a tricyclic heterocyclic compound three hydrogen atoms bonding directly to atoms constituting the ring, and these groups optionally have a substituent.
[4] The light emitting device according to [3], wherein the above-described G^{DA} is a group obtained by removing from benzene, pyridine, diazabenzene, triazine or carbazole three hydrogen atoms bonding directly to atoms constituting the ring, and the group optionally has a substituent.
[5] The light emitting device according to any one of [1] to [4], wherein the above-described Ar^{DA1} is a group obtained by removing from a monocyclic, bicyclic or a tricyclic aromatic hydrocarbon two hydrogen atoms bonding directly to carbon atoms constituting the ring or a group obtained by removing from a monocyclic, bicyclic or a tricyclic heterocyclic compound two hydrogen atoms bonding directly to atoms constituting the ring, and these groups optionally have a substituent.
[6] The light emitting device according to any one of [1] to [4], wherein the above-described Ar^{DA1} is an arylene group, and the group optionally has a substituent.
[7] The light emitting device according to [6], wherein the above-described Ar^{DA1} is a phenylene group optionally having a substituent.
[8] The light emitting device according to any one of [1] to [7], wherein the above-described Ar^{B1} is a group obtained by removing from a monocyclic or bicyclic to pentacyclic aromatic hydrocarbon n^{B1} hydrogen atoms bonding directly to carbon atoms constituting the ring or a group obtained by removing from a monocyclic or bicyclic to pentacyclic heterocyclic compound n^{B1} hydrogen atoms bonding directly to atoms constituting the ring, and these groups optionally have a substituent.
[9] The light emitting device according to [8], wherein the above-described Ar^{B1} is a group obtained by removing from a monocyclic or bicyclic to pentacyclic heterocyclic compound n^{B1} hydrogen atoms bonding directly to atoms constituting the ring, and the group optionally has a substituent.
[10] The light emitting device according to any one of [1] to [7], wherein the above-described Ar^{B1} is a group obtained by removing n^{B1} hydrogen atoms from the above-described metal complex represented by the formula (1), and the group optionally has a substituent.
[11] The light emitting device according to [10], wherein the above-described Ring L¹ is an aromatic hetero ring containing a 5-membered ring or an aromatic hetero ring containing a 6-membered ring, and the ring optionally has a substituent, and, the above-described Ring L² is an aromatic hydrocarbon ring containing a 5-membered ring or 6-membered ring or an aromatic hetero ring containing a 5-membered ring or 6-membered ring, and these rings optionally have a substituent.
[12] The light emitting device according to [10] or [11], wherein the above-described Ring L¹ is a pyridine ring, a diazabenzene ring, an azanaphthalene ring, a diazanaphthalene ring, a diazole ring or a triazole ring, and these rings optionally have a substituent, and, the above-described Ring L² is a benzene ring, a pyridine ring or a diazabenzene ring, and these rings optionally have a substituent.
[13] The light emitting device according to any one of [1] to [12], wherein the above-described compound having a cross-linkable group is a polymer compound containing a constitutional unit having a cross-linkable group.
[14] The light emitting device according to [13], wherein the above-described constitutional unit having a cross-linkable group is a constitutional unit represented by the formula (Z) or a constitutional unit represented by the formula (Z'): 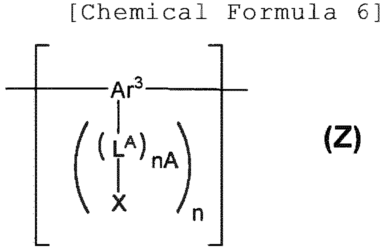 [wherein,
   n represents an integer of 1 or more.
   nA represents an integer of 0 or more. When a plurality of nA are present, they may be the same or different.
   Ar³ represents a hydrocarbon group, a hetero ring group, or a group in which at least one hydrocarbon group and at least one hetero ring group are bonded directly, and these groups optionally have a substituent. When a plurality of the substituents are present, they may be the same or different and may be combined together to form a ring together with atoms to which they are attached.
   L^{A} represents an alkylene group, a cycloalkylene group, an arylene group, a divalent hetero ring group, a group represented by -N(R')-, an oxygen atom or a sulfur atom, and these groups optionally have a substituent. When a plurality of the substituents are present, they may be the same or different and may be combined together to form a ring together with atoms to which they are attached. R' represents a hydrogen atom, an alkyl group, a cycloalkyl group, an aryl group or a monovalent hetero ring group, and these groups optionally have a substituent. When a plurality of the substituents are present, they may be the same or different and may be combined together to form a ring together with atoms to which they are attached. When a plurality of L^{A} are present, they may be the same or different.
   X represents a cross-linkable group. When a plurality of X are present, they may be the same or different.] 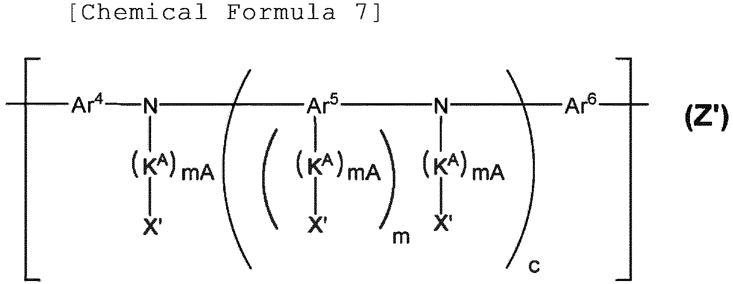
   [wherein,
   mA, m and c each independently represent an integer of 0 or more. When a plurality of mA are present, they may be the same or different. When a plurality of m are present, they may be the same or different.
   Ar⁵ represents a hydrocarbon group, a hetero ring group, or a group in which at least one hydrocarbon group and at least one hetero ring group are bonded directly, and these groups optionally have a substituent. When a plurality of the substituents are present, they may be the same or different and may be combined together to form a ring together with atoms to which they are attached. When a plurality of Ar⁵ are present, they may be the same or different.
   Ar⁴ and Ar⁶ each independently represent an arylene group or a divalent hetero ring group, and these groups optionally have a substituent. When a plurality of the substituents are present, they may be the same or different and may be combined together to form a ring together with atoms to which they are attached.
   K^{A} represents an alkylene group, a cycloalkylene group, an arylene group, a divalent hetero ring group, a group represented by -N(R")-, an oxygen atom or a sulfur atom, and these groups optionally have a substituent. When a plurality of the substituents are present, they may be the same or different and may be combined together to form a ring together with atoms to which they are attached. R" represents a hydrogen atom, an alkyl group, a cycloalkyl group, an aryl group or a monovalent hetero ring group, and these groups optionally have a substituent. When a plurality of the substituents are present, they may be the same or different and may be combined together to form a ring together with atoms to which they are attached. When a plurality of K^{A} are present, they may be the same or different.
   X' represents a hydrogen atom, a cross-linkable group, an alkyl group, a cycloalkyl group, an aryl group or a monovalent hetero ring group, and these groups optionally have a substituent. When a plurality of the substituents are present, they may be the same or different and may be combined together to form a ring together with atoms to which they are attached. When a plurality of X' are present, they may be the same or different. Under this condition, at least one X' is a cross-linkable group.].
[15] The light emitting device according to [13] or [14], wherein the above-described polymer compound further contains at least one constitutional unit selected from the group consisting of a constitutional unit represented by the formula (X) and a constitutional unit represented by the formula (Y): 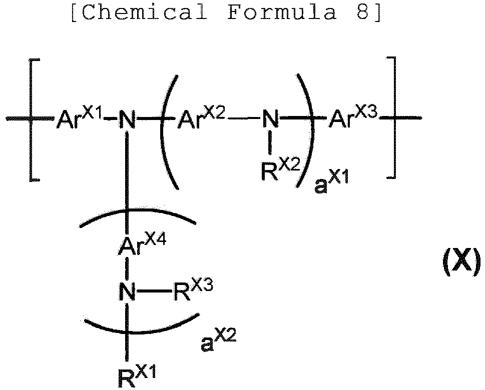 [wherein,
   a^{X1} and a^{X2} each independently represent an integer of 0 or more.
   Ar^{X1} and Ar^{X3} each independently represent an arylene group or a divalent hetero ring group, and these groups optionally have a substituent. When a plurality of the substituents are present, they may be the same or different and may be combined together to form a ring together with atoms to which they are attached.
   Ar^{X2} and Ar^{X4} each independently represent an arylene group, a divalent hetero ring group, or a divalent group in which at least one arylene group and at least one divalent hetero ring group are bonded directly, and these groups optionally have a substituent. When a plurality of the substituents are present, they may be the same or different and may be combined together to form a ring together with atoms to which they are attached. When a plurality of Ar^{X2} are present, they may be the same or different at each occurrence. When a plurality of Ar^{X4} are present, they may be the same or different at each occurrence.
   R^{X1}, R^{X2} and R^{X3} each independently represent a hydrogen atom, an alkyl group, a cycloalkyl group, an aryl group or a monovalent hetero ring group, and these groups optionally have a substituent. When a plurality of the substituents are present, they may be the same or different and may be combined together to form a ring together with atoms to which they are attached. When a plurality of R^{X2} are present, they may be the same or different. When a plurality of R^{X3} are present, they may be the same or different.] 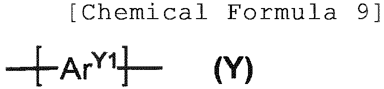
   [wherein, Ar^{Y1} represents an arylene group, a divalent hetero ring group, or a divalent group in which at least one arylene group and at least one divalent hetero ring group are bonded directly, and these groups optionally have a substituent. When a plurality of the substituents are present, they may be the same or different and may be combined together to form a ring together with atoms to which they are attached.].
[16] The light emitting device according to any one of [1] to [12], wherein the above-described compound having a cross-linkable group is a compound represented by the formula (Z"): 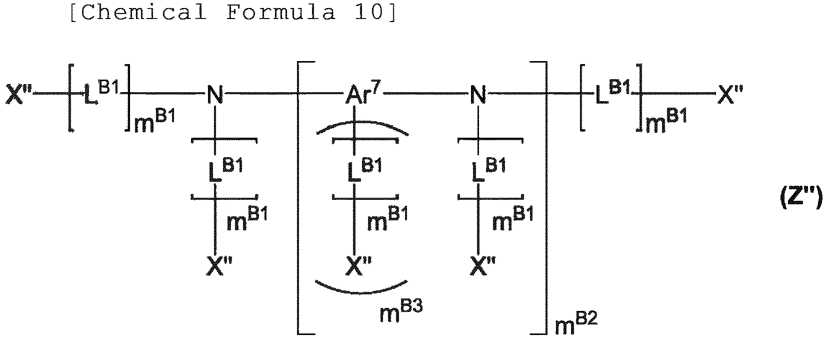 [wherein,
   m^{B1}, m^{B2} and m^{B3} each independently represent an integer of 0 or more. When a plurality of m^{B1} are present, they may be the same or different. When a plurality of m^{B3} are present, they may be the same or different.
   Ar⁷ represents a hydrocarbon group, a hetero ring group, or a group in which at least one hydrocarbon group and at least one hetero ring group are bonded directly, and these groups optionally have a substituent. When a plurality of the substituents are present, they may be the same or different and may be combined together to form a ring together with atoms to which they are attached. When a plurality of Ar⁷ are present, they may be the same or different.
   L^{B1} represents an alkylene group, a cycloalkylene group, an arylene group, a divalent hetero ring group, a group represented by -N(R‴)-, an oxygen atom or a sulfur atom, and these groups optionally have a substituent. When a plurality of the substituents are present, they may be the same or different and may be combined together to form a ring together with atoms to which they are attached. R‴ represents a hydrogen atom, an alkyl group, a cycloalkyl group, an aryl group or a monovalent hetero ring group, and these groups optionally have a substituent. When a plurality of the substituents are present, they may be the same or different and may be combined together to form a ring together with atoms to which they are attached. When a plurality of L^{B1} are present, they may be the same or different. X" represents a cross-linkable group, a hydrogen atom, an alkyl group, a cycloalkyl group, an aryl group or a monovalent hetero ring group, and these groups optionally have a substituent. When a plurality of the substituents are present, they may be the same or different and may be combined together to form a ring together with atoms to which they are attached. A plurality of X" may be the same or different. Under this condition, at least one of a plurality of X" is a cross-linkable group.].
[17] The light emitting device according to any one of [1] to [16], wherein the above-described cross-linkable group is at least one cross-linkable group selected from Group A of cross-linkable group:
   (Group A of cross-linkable group) 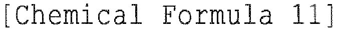 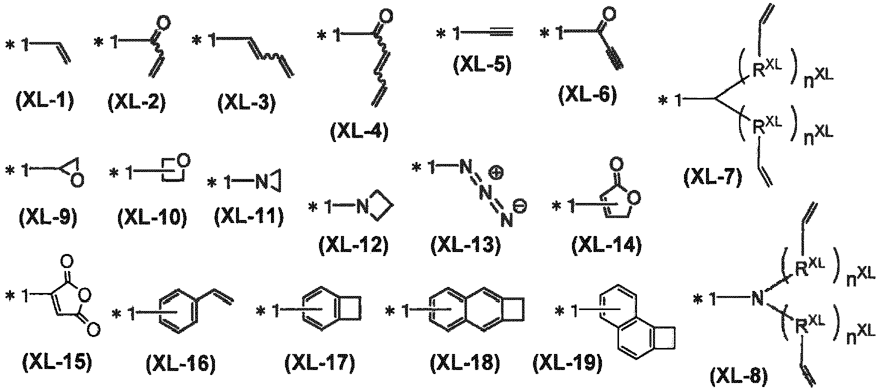 [wherein, R^{XL} represents a methylene group, an oxygen atom or a sulfur atom, and n^{XL} represents an integer of 0 to 5. When a plurality of R^{XL} are present, they may be the same or different. When a plurality of n^{XL} are present, they may be the same or different. *1 represents a bonding position. These cross-linkable groups optionally have a substituent, and when a plurality of the substituents are present, they may be the same or different and may be combined together to form a ring together with atoms to which they are attached.].
[18] The light emitting device according to any one of [1] to [17], wherein the above-described first layer contains two or more of the above-described compounds (B-1).
[19] The light emitting device according to any one of [1] to [18], wherein the above-described first layer further contains a polymer compound containing at least one constitutional unit selected from the group consisting of a constitutional unit represented by the formula (X) and a constitutional unit represented by the formula (Y): 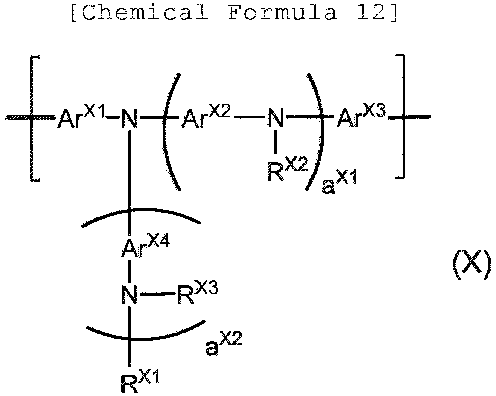 [wherein,
   a^{X1} and a^{X2} each independently represent an integer of 0 or more.
   Ar^{X1} and Ar^{X3} each independently represent an arylene group or a divalent hetero ring group, and these groups optionally have a substituent. When a plurality of the substituents are present, they may be the same or different and may be combined together to form a ring together with atoms to which they are attached.
   Ar^{X2} and Ar^{X4} each independently represent an arylene group, a divalent hetero ring group, or a divalent group in which at least one arylene group and at least one divalent hetero ring group are bonded directly, and these groups optionally have a substituent. When a plurality of the substituents are present, they may be the same or different and may be combined together to form a ring together with atoms to which they are attached. When a plurality of Ar^{X2} are present, they may be the same or different at each occurrence. When a plurality of Ar^{X4} are present, they may be the same or different at each occurrence.
   R^{X1}, R^{X2} and R^{X3} each independently represent a hydrogen atom, an alkyl group, a cycloalkyl group, an aryl group or a monovalent hetero ring group, and these groups optionally have a substituent. When a plurality of the substituents are present, they may be the same or different and may be combined together to form a ring together with atoms to which they are attached. When a plurality of R^{X2} are present, they may be the same or different. When a plurality of R^{X3} are present, they may be the same or different.] 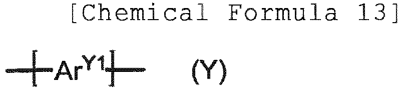
   [wherein, Ar^{Y1} represents an arylene group, a divalent hetero ring group, or a divalent group in which at least one arylene group and at least one divalent hetero ring group are bonded directly, and these groups optionally have a substituent. When a plurality of the substituents are present, they may be the same or different and may be combined together to form a ring together with atoms to which they are attached.].
[20] The light emitting device according to any one of [1] to [19], wherein at least one of the above-described compound (B-1) and at least one of the above-described compound (B-2) are the same.
[21] The light emitting device according to any one of [1] to [20], wherein at least one of the above-described compound (B-1) is the above-described compound in which n^{B1} is 1 or more.
[22] The light emitting device according to any one of [1] to [21], wherein the above-described first layer further contains at least one selected from the group consisting of a hole transporting material, a hole injection material, an electron transporting material, an electron injection material, a light emitting material and an antioxidant.
[23] The light emitting device according to any one of [1] to [22], wherein the above-described first layer and the above-described second layer are adjacent.
[24] The light emitting device according to any one of [1] to [23], wherein the above-described second layer is disposed between the above-described anode and the above-described first layer.
[25] A composition containing at least one compound (B-2) selected from compounds represented by the formula (B), and a crosslinked body of a compound having a cross-linkable group: 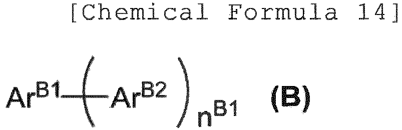 [wherein,
   n^{B1} represents an integer of 1 or more.
   Ar^{B1} represents a group obtained by removing from an aromatic hydrocarbon n^{B1} hydrogen atoms bonding directly to carbon atoms constituting the ring, a group obtained by removing from a heterocyclic compound n^{B1} hydrogen atoms bonding directly to atoms constituting the ring or a group obtained by removing n^{B1} hydrogen atoms from a metal complex represented by the formula (1), and these groups optionally have a substituent. When a plurality of the substituents are present, they may be the same or different and may be combined together to form a ring together with atoms to which they are attached.
   Ar^{B2} represents a group represented by the formula (D-A) or a group represented by the formula (D-B). When a plurality of Ar^{B2} are present, they may be the same or different.] 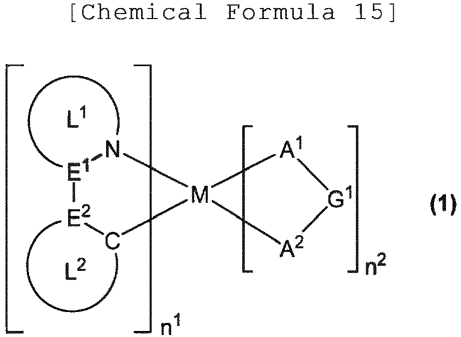
   [wherein,
   M represents a rhodium atom, a palladium atom, an iridium atom or a platinum atom.
   n¹ represents an integer of 1 or more, and n² represents an integer of 0 or more. Under this condition, n¹+n² is 3 when M is a rhodium atom or an iridium atom, while n¹+n² is 2 when M is a palladium atom or a platinum atom.
   E¹ and E² each independently represent a carbon atom or a nitrogen atom. When a plurality of E¹ and E² are present, they may be the same or different at each occurrence.
   Ring L¹ represents an aromatic hetero ring, and the ring optionally has a substituent. When a plurality of the substituents are present, they may be the same or different and may be combined together to form a ring together with atoms to which they are attached. When a plurality of Ring L¹ are present, they may be the same or different.
   Ring L² represents an aromatic hydrocarbon ring or an aromatic hetero ring, and these rings optionally have a substituent. When a plurality of the substituents are present, they may be the same or different and may be combined together to form a ring together with atoms to which they are attached. When a plurality of Ring L² are present, they may be the same or different.
   A substituent which Ring L¹ optionally has and a substituent which Ring L² optionally has may be the same or different and may be combined together to form a ring together with atoms to which they are attached.
   A¹-G¹-A² represents an anionic bidentate ligand. A¹ and A² each independently represent a carbon atom, an oxygen atom or a nitrogen atom, and these atoms may be ring-constituent atoms. G¹ represents a single bond, or an atomic group constituting a bidentate ligand together with A¹ and A². When a plurality of A¹-G¹-A² are present, they may be the same or different.] 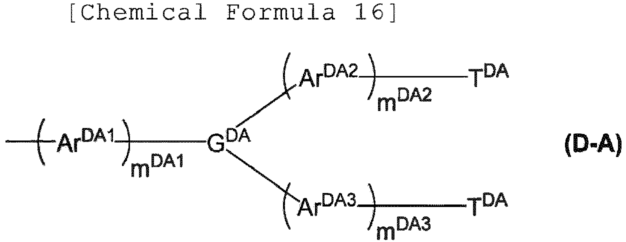
   [wherein,
   m^{DA1} represents an integer of 1 or more.
   m^{DA2} and m^{DA3} each independently represent an integer of 0 or more.
   G^{DA} represents a nitrogen atom, an aromatic hydrocarbon group or a hetero ring group, and these groups optionally have a substituent. When a plurality of the substituents are present, they may be the same or different and may be combined together to form a ring together with atoms to which they are attached.
   Ar^{DA1}, Ar^{DA2} and Ar^{DA3} each independently represent an arylene group or a divalent hetero ring group, and these groups optionally have a substituent. When a plurality of the substituents are present, they may be the same or different and may be combined together to form a ring together with atoms to which they are attached. When a plurality of Ar^{DA1}, Ar^{DA2} and Ar^{DA3} are present, they may be the same or different at each occurrence.
   T^{DA} represents an aryl group or a monovalent hetero ring group, and these groups optionally have a substituent. When a plurality of the substituents are present, they may be the same or different and may be combined together to form a ring together with atoms to which they are attached. A plurality of T^{DA} may be the same or different.] 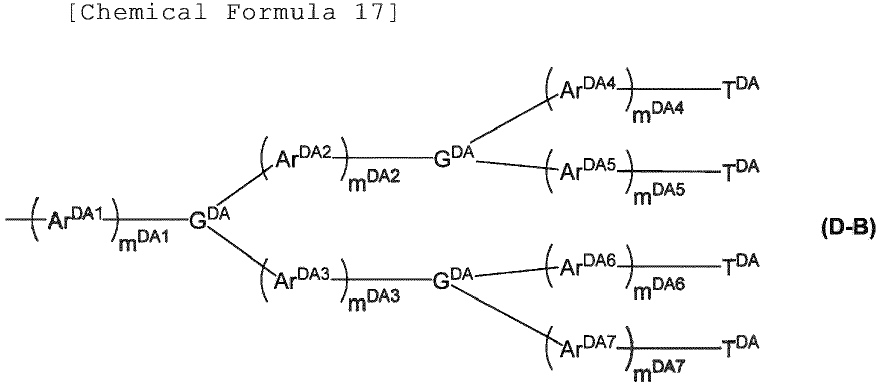
   [wherein,
   m^{DA1} represents an integer of 1 or more.
   m^{DA2}, m^{DA3}, m^{DA4}, m^{DA5}, m^{DA6} and m^{DA7} each independently represent an integer of 0 or more.
   G^{DA} represents a nitrogen atom, an aromatic hydrocarbon group or a hetero ring group, and these groups optionally have a substituent. When a plurality of the substituents are present, they may be the same or different and may be combined together to form a ring together with atoms to which they are attached. A plurality of G^{DA} may be the same or different.
   Ar^{DA1}, Ar^{DA2}, Ar^{DA3}, Ar^{DA4}, Ar^{DA5}, Ar^{DA6} and Ar^{DA7} each independently represent an arylene group or a divalent hetero ring group, and these groups optionally have a substituent. When a plurality of the substituents are present, they may be the same or different and may be combined together to form a ring together with atoms to which they are attached. When a plurality of Ar^{DA1}, Ar^{DA2}, Ar^{DA3}, Ar^{DA4}, Ar^{DA5}, Ar^{DA6} and Ar^{DA7} are present, they may be the same or different at each occurrence.
   T^{DA} represents an aryl group or a monovalent hetero ring group, and these groups optionally have a substituent. When a plurality of the substituents are present, they may be the same or different and may be combined together to form a ring together with atoms to which they are attached. A plurality of T^{DA} may be the same or different.].

### Effect of the Invention

According to the present invention, a light emitting device excellent in maximum light emission efficiency can be provided. In addition, according to the present invention, it is possible to provide a composition which is useful for producing such a light emitting device.

### Modes for Carrying Out the Invention

Suitable embodiments of the present invention will be described in detail below.

### <Explanation of common terms>

The terms commonly used in the present specification have the following meanings unless otherwise stated.

The "room temperature" indicates 25°C.

Me represents a methyl group, Et represents an ethyl group, Bu represents a butyl group, i-Pr represents an isopropyl group, and t-Bu represents a tert-butyl group.

The hydrogen atom may be a heavy hydrogen atom or a light hydrogen atom.

In formulae representing a metal complex, the solid line representing a bond with the central metal denotes a covalent bond or a coordination bond.

The "low molecular compound" means a compound not having molecular weight distribution and having a molecular weight of 1×10⁴ or less.

The "polymer compound" means a polymer having molecular weight distribution and having a polystyreneequivalent number-average molecular weight of 1×10³ or more (for example, 1×10³ to 1×10⁸).

The "constitutional unit" means a unit occurring once or more times in a polymer compound. The constitutional unit occurring twice or more times in a polymer compound is generally referred to also as "repeating unit".

The polymer compound may be any of a block copolymer, a random copolymer, an alternative copolymer, or a graft copolymer, and may also be in another form.

The end group of the polymer compound is preferably a stable group since if a polymerization active group remains intact there, there is a possibility of a decrease in a light emitting property or luminance life when the polymer compound is used for fabrication of a light emitting device. The end group of the polymer compound is preferably a group conjugatively bonded to the main chain and includes, for example, an aryl group or a monovalent hetero ring group bonding to the main chain of the polymer compound via a carbon-carbon bond.

The "alkyl group" may be any of linear or branched. The number of carbon atoms of the linear alkyl group is, not including the number of carbon atoms of the substituent, usually 1 to 50, preferably 1 to 20, and more preferably 1 to 10. The number of carbon atoms of the branched alkyl group is, not including the number of carbon atoms of the substituent, usually 3 to 50, preferably 3 to 20, and more preferably 4 to 10.

The alkyl group optionally has a substituent. The alkyl group includes, for example, a methyl group, an ethyl group, a propyl group, an isopropyl group, a butyl group, a 2-butyl group, an isobutyl group, a tert-butyl group, a pentyl group, an isoamyl group, a 2-ethylbutyl group, a hexyl group, a heptyl group, an octyl group, a 2-ethylhexyl group, a 3-propylheptyl group, a decyl group, a 3,7-dimethyloctyl group, a 2-ethyloctyl group, a 2-hexyldecyl group and a dodecyl group. Further, the alkyl group may be a group obtained by substituting a part or all of hydrogen atoms in these groups with a substituent (for example, a trifluoromethyl group, a pentafluoroethyl group, a perfluorobutyl group, a perfluorohexyl group, a perfluorooctyl group, a 3-phenylpropyl group, a 3-(4-methylphenyl)propyl group, a 3-(3,5-di-hexylphenyl)propyl group, or a 6-ethyloxyhexyl group).

The number of carbon atoms of the "cycloalkyl group" is, not including the number of carbon atoms of the substituent, usually 3 to 50, and preferably 4 to 10.

The cycloalkyl group optionally has a substituent. The cycloalkyl group includes, for example, a cyclohexyl group, and groups obtained by substituting a part or all of hydrogen atoms in the group with a substituent.

The number of carbon atoms of the "alkylene group" is, not including the number of carbon atoms of the substituent, usually 1 to 20, preferably 1 to 15, and more preferably 1 to 10.

The alkylene group optionally has a substituent. The alkylene group includes, for example, a methylene group, an ethylene group, a propylene group, a butylene group, a hexylene group, and an octylene group, and groups obtained by substituting a part or all of hydrogen atoms in these groups with a substituent.

The number of carbon atoms of the "cycloalkylene group" is, not including the number of carbon atoms of the substituent, usually 3 to 20, and preferably 4 to 10.

The cycloalkylene group optionally has a substituent. The cycloalkylene group includes, for example, a cyclohexylene group, and groups obtained by substituting a part or all of hydrogen atoms in the group with a substituent.

The "aromatic hydrocarbon group" means a group obtained by removing from an aromatic hydrocarbon one or more hydrogen atoms bonding directly to carbon atoms constituting the ring. A group obtained by removing from an aromatic hydrocarbon one hydrogen atom bonding directly to a carbon atom constituting the ring is referred to also as "aryl group". A group obtained by removing from an aromatic hydrocarbon two hydrogen atoms bonding directly to carbon atoms constituting the ring is referred to also as "arylene group".

The number of carbon atoms of the aromatic hydrocarbon group is, not including the number of carbon atoms of the substituent, usually 6 to 60, preferably 6 to 40, and more preferably 6 to 20.

The "aromatic hydrocarbon group" includes, for example, groups obtained by removing from a monocyclic aromatic hydrocarbon (including, for example, benzene) or a polycyclic aromatic hydrocarbon (including, for example, bicyclic aromatic hydrocarbons such as naphthalene, indene, naphthoquinone, indenone, tetralone, and the like; tricyclic aromatic hydrocarbons such as anthracene, phenanthrene, dihydrophenanthrene, fluorene, anthraquinone, phenanthoquinone, fluorenone, and the like; tetracyclic aromatic hydrocarbons such as benzoanthracene, benzophenanthrene, benzofluorene, pyrene, fluoranthene, and the like; pentacyclic aromatic hydrocarbons such as dibenzoanthracene, dibenzophenanthrene, dibenzofluorene, indenofluorene, perylene, benzofluoranthene, and the like; hexacyclic aromatic hydrocarbons such as spirobifluorene, and the like; and, heptacyclic aromatic hydrocarbons such as benzospirobifluorene, acenaphthofluoranthene, and the like) one or more hydrogen atoms bonding directly to carbon atoms constituting the ring. The aromatic hydrocarbon group includes groups obtained by bonding a plurality of these groups. The aromatic hydrocarbon group optionally has a substituent.

The "alkoxy group" may be any of linear or branched. The number of carbon atoms of the linear alkoxy group is, not including the number of carbon atoms of the substituent, usually 1 to 40, and preferably 1 to 10. The number of carbon atoms of the branched alkoxy group is, not including the number of carbon atoms of the substituent, usually 3 to 40, and preferably 4 to 10.

The alkoxy group optionally has a substituent. The alkoxy group includes, for example, a methoxy group, an ethoxy group, an isopropyloxy group, a butyloxy group, a hexyloxy group, a 2-ethylhexyloxy group, a 3,7-dimethyloctyloxy group, and a lauryloxy group, and groups obtained by substituting a part or all of hydrogen atoms in these groups with a substituent.

The number of carbon atoms of the "cycloalkoxy group" is, not including the number of carbon atoms of the substituent, usually 3 to 40, and preferably 4 to 10.

The cycloalkoxy group optionally has a substituent. The cycloalkoxy group includes, for example, a cyclohexyloxy group, and groups obtained by substituting a part or all of hydrogen atoms in the group with a substituent.

The number of carbon atoms of the "aryloxy group" is, not including the number of carbon atoms of the substituent, usually 6 to 60, preferably 6 to 40, and more preferably 6 to 20.

The aryloxy group optionally has a substituent. The aryloxy group includes, for example, a phenoxy group, a naphthyloxy group, an anthracenyloxy group, and a pyrenyloxy group, and groups obtained by substituting a part or all of hydrogen atoms in these groups with a substituent.

The "hetero ring group" means a group obtained by removing from a heterocyclic compound one or more hydrogen atoms bonding directly to atoms (carbon atoms or hetero atoms) constituting the ring. Among the hetero ring groups, an "aromatic hetero ring group" which is a group obtained by removing from an aromatic heterocyclic compound one or more hydrogen atoms bonding directly to atoms constituting the ring is preferred. A group obtained by removing from a heterocyclic compound p hydrogen atoms (p represents an integer of 1 or more) bonding directly to atoms constituting the ring is referred to also as "p-valent hetero ring group". A group obtained by removing from an aromatic heterocyclic compound p hydrogen atoms bonding directly to atoms constituting the ring is referred to also as "p-valent aromatic hetero ring group".

The "aromatic heterocyclic compound" includes, for example, a compound in which the hetero ring itself shows aromaticity, such as azole, thiophene, furan, pyridine, diazabenzene, triazine, azanaphthalene, diazanaphthalene, carbazole, and the like, and a compound in which an aromatic ring is condensed to the hetero ring even if the hetero ring itself shows no aromaticity, such as phenoxazine, phenothiazine, benzopyran, and the like.

The number of carbon atoms of the hetero ring group is, not including the number of carbon atoms of the substituent, usually 1 to 60, preferably 2 to 40, and more preferably 3 to 20. The number of hetero atoms of the hetero ring group is, not including the number of hetero atoms of the substituent, usually 1 to 30, preferably 1 to 10, more preferably 1 to 5, and further preferably 1 to 3.

The hetero ring group includes, for example, groups obtained by removing from a monocyclic heterocyclic compound (including, for example, furan, thiophene, oxadiazole, thiadiazole, pyrrole, diazole, triazole, tetrazole, pyridine, diazabenzene and triazine) or a polycyclic heterocyclic compound (including, for example, bicyclic heterocyclic compounds such as azanaphthalene, diazanaphthalene, benzofuran, benzothiophene, indole, azaindole, diazaindole, benzodiazole, benzothiadiazole, benzotriazole, benzothiophenedioxide, benzothiopheneoxide, benzopyranone, and the like; tricyclic heterocyclic compounds such as dibenzofuran, dibenzothiophene, dibenzothiophenedioxide, dibenzothiopheneoxide, dibenzopyranone, dibenzoborole, dibenzosilole, dibenzophosphole, dibenzoselenophene, carbazole, azacarbazole, diazacarbazole, phenoxazine, phenothiazine, 9,10-dihydroacridine, 5,10-dihydrophenazine, acridone, phenezaborine, phenophosphazine, phenoselenazine, phenazasiline, azaanthracene, diazaanthracene, azaphenanthrene, diazaphenanthrene, and the like; tetracyclic heterocyclic compounds such as hexaazatriphenylene, benzocarbazole, azabenzocarbazole, diazabenzocarbazole, benzonaphthofuran, benzonaphthothiophene, and the like; pentacyclic heterocyclic compounds such as dibenzocarbazole, indolocarbazole, indenocarbazole, azaindolocarbazole, diazaindolocarbazole, azaindenocarbazole, diazaindenocarbazole, and the like; hexacyclic heterocyclic compounds such as carbazolocarbazole, benzoindolocarbazole, benzoindenocarbazole, and the like; and, heptacyclic heterocyclic compounds such as dibenzoindolocarbazole, dibenzoindenocarbazole, and the like) one or more hydrogen atoms bonding directly to atoms constituting the ring. The hetero ring group includes groups obtained by bonding a plurality of these groups. The hetero ring group optionally has a substituent.

The "halogen atom" denotes a fluorine atom, a chlorine atom, a bromine atom or an iodine atom.

The "amino group" optionally has a substituent, and substituted amino groups (namely, secondary amino groups or tertiary amino groups, more preferably tertiary amino groups) are preferred. The substituent which an amino group has is preferably an alkyl group, a cycloalkyl group, an aryl group or a monovalent hetero ring group, and these groups optionally further have a substituent. When a plurality of the substituents which an amino group has are present, they may be the same or different and may be combined together to form a ring together with nitrogen atoms to which they are attached.

The substituted amino group includes, for example, a dialkylamino group, a dicycloalkylamino group, and a diarylamino group, and groups obtained by further substituting a part or all of hydrogen atoms in these groups with a substituent.

The substituted amino group includes, for example, a dimethylamino group, a diethylamino group, a diphenylamino group, a bis(methylphenyl)amino group, and a bis(3,5-di-tert-butylphenyl)amino group, and groups obtained by further substituting a part or all of hydrogen atoms in these groups with a substituent.

The "alkenyl group" may be any of linear or branched. The number of carbon atoms of the linear alkenyl group is, not including the number of carbon atoms of the substituent, usually 2 to 30, and preferably 3 to 20. The number of carbon atoms of the branched alkenyl group is, not including the number of carbon atoms of the substituent, usually 3 to 30, and preferably 4 to 20.

The number of carbon atoms of the "cycloalkenyl group" is, not including the number of carbon atoms of the substituent, usually 3 to 30, and preferably 4 to 20.

The alkenyl group and the cycloalkenyl group optionally have a substituent. The alkenyl group includes, for example, a vinyl group, a 1-propenyl group, a 2-butenyl group, a 3-butenyl group, a 3-pentenyl group, a 4-pentenyl group, a 1-hexenyl group, a 5-hexenyl group, and a 7-octenyl group, and groups obtained by substituting a part or all of hydrogen atoms in these groups with a substituent. The cycloalkenyl group includes, for example, a cyclohexenyl group, a cyclohexadienyl group, a cyclooctatrienyl group, and a norbornylenyl group, and groups obtained by substituting a part or all of hydrogen atoms in these groups with a substituent.

The "alkynyl group" may be any of linear or branched. The number of carbon atoms of the alkynyl group is, not including carbon atoms of the substituent, usually 2 to 20, and preferably 3 to 20. The number of carbon atoms of the branched alkynyl group is, not including carbon atoms of the substituent, usually 4 to 30, and preferably 4 to 20.

The number of carbon atoms of the "cycloalkynyl group" is, not including carbon atoms of the substituent, usually 4 to 30, and preferably 4 to 20.

The alkynyl group and the cycloalkynyl group optionally have a substituent. The alkynyl group includes, for example, an ethynyl group, a 1-propynyl group, a 2-propynyl group, a 2-butynyl group, a 3-butynyl group, a 3-pentynyl group, a 4-pentynyl group, a 1-hexynyl group, and a 5-hexynyl group, and groups obtained by substituting a part or all of hydrogen atoms in these groups with a substituent. The cycloalkynyl group includes, for example, a cyclooctynyl group, and groups obtained by substituting a part or all of hydrogen atoms in the group with a substituent.

The "cross-linkable group" is a group capable of generating a new bond by being subjected to a heating treatment, an ultraviolet irradiation treatment, a near ultraviolet irradiation treatment, a visible light irradiation treatment, an infrared irradiation treatment, a radical reaction, and the like. As the cross-linkable group, at least one cross-linkable group selected from Group A of cross-linkable group is preferred.

The "substituent" includes, for example, a halogen atom, a cyano group, an alkyl group, a cycloalkyl group, an aryl group, a monovalent hetero ring group, an alkoxy group, a cycloalkoxy group, an aryloxy group, an amino group, a substituted amino group, an alkenyl group, a cycloalkenyl group, an alkynyl group, and a cycloalkynyl group. The substituent may be a cross-linkable group or a group represented by the formula (1-T). When a plurality of substituents are present, they may be the same or different. In addition, when a plurality of substituents are present, they may be combined together to form a ring together with atoms to which they are attached, but it is preferable that they do not form a ring.

### <First layer>

In the light emitting device of the present embodiment, the first layer is a layer containing at least one compound (B-1) selected from compounds represented by the formula (B).

The first layer may contain the compound (B-1) only singly or in combination of two or more.

The content of the compound (B-1) in the first layer may be within a range in which the function as the first layer is exhibited. The content of the compound (B-1) in the first layer, for example, may be 1 to 100° by mass, and is preferably 5 to 100° by mass, more preferably 10 to 100% by mass, further preferably 20 to 100% by mass, and particularly preferably 30 to 100% by mass, based on the total amount of the first layer.

### [Compound represented by the formula (B)]

When Ar^{B1} is a group obtained by removing from an aromatic hydrocarbon n^{B1} hydrogen atoms bonding directly to carbon atoms constituting the ring or a group obtained by removing from a heterocyclic compound n^{B1} hydrogen atoms bonding directly to atoms constituting the ring (hereinafter, referred to also as "when Ar^{B1} is an aromatic hydrocarbon group or a hetero ring group"), the molecular weight of the compound represented by the formula (B) is preferably 2×10² to 1×10⁴, more preferably 3×10² to 7×10³, further preferably 4×10² to 5×10³, and particularly preferably 5×10² to 3×10³.

When n^{B1} is 1 or more and Ar^{B1} is an aromatic hydrocarbon group or a hetero ring group, the molecular weight of the compound represented by the formula (B) is preferably 3×10² to 1×10⁴, more preferably 4×10² to 7×10³, further preferably 5×10² to 5×10³, and particularly preferably 6×10² to 3×10³.

When Ar^{B1} is a group obtained by removing n^{B1} hydrogen atoms from a metal complex represented by the formula (1) (hereinafter, referred to also as "when Ar^{B1} is a metal complex group"), the molecular weight of the compound represented by the formula (B) is preferably 2×10² to 1×10⁴, more preferably 4×10² to 7×10³, further preferably 6×10² to 5×10³, and particularly preferably 8×10² to 3×10³.

When n^{B1} is 1 or more and Ar^{B1} is a metal complex group, the molecular weight of the compound represented by the formula (B) is preferably 6×10² to 1×10⁴, more preferably 8×10² to 7×10³, further preferably 1×10² to 5×10³, and particularly preferably 1.5×10³ to 3×10³.

It is preferable that the compound represented by the formula (B) is a low molecular compound.

When Ar^{B1} is an aromatic hydrocarbon group or a hetero ring group, it is preferable that the compound represented by the formula (B) is a compound containing no transition metal element (namely, a compound constituted of typical elements).

When Ar^{B1} is an aromatic hydrocarbon group or a hetero ring group, n^{B1} is usually 0 or more and 20 or less, and it is preferably an integer of 0 or more and 10 or less, more preferably an integer of 0 or more and 7 or less, further preferably an integer of 0 or more and 5 or less, and particularly preferably an integer of 0 or more and 3 or less, since the light emitting device of the present embodiment is excellent in maximum light emission efficiency.

When n^{B1} is 1 or more and Ar^{B1} is an aromatic hydrocarbon group or a hetero ring group, n^{B1} is usually 1 or more and 20 or less, and it is preferably an integer of 1 or more and 10 or less, more preferably an integer of 1 or more and 7 or less, further preferably an integer of 1 or more and 5 or less, and particularly preferably an integer of 1 or more and 3 or less, since the light emitting device of the present embodiment is excellent in maximum light emission efficiency.

When Ar^{B1} is a metal complex group, n^{B1} is usually 0 or more and 30 or less, and it is preferably an integer of 0 or more and 18 or less, more preferably an integer of 0 or more and 12 or less, and further preferably an integer of 0 or more and 6 or less, since the light emitting device of the present embodiment is excellent in maximum light emission efficiency.

When n^{B1} is 1 or more and Ar^{B1} is a metal complex group, n^{B1} is usually 1 or more and 30 or less, and it is preferably an integer of 1 or more and 18 or less, more preferably an integer of 1 or more and 12 or less, and further preferably an integer of 2 or more and 6 or less, since the light emitting device of the present embodiment is excellent in maximum light emission efficiency.

The aromatic hydrocarbon represented by Ar^{B1} includes, for example, aromatic hydrocarbons exemplified in the section of the aromatic hydrocarbon group described above, and it is preferably a monocyclic or bicyclic to heptacyclic aromatic hydrocarbon, more preferably a monocyclic or bicyclic to pentacyclic aromatic hydrocarbon, a monocyclic, bicyclic or a tricyclic aromatic hydrocarbon, and particularly preferably a monocyclic aromatic hydrocarbon, since the light emitting device of the present embodiment is more excellent in maximum light emission efficiency. In addition, the aromatic hydrocarbon represented by Ar^{B1} is preferably an aromatic hydrocarbon containing a 5-membered ring or a 6-membered ring, and more preferably an aromatic hydrocarbon containing a 6-membered ring, since the light emitting device of the present embodiment is more excellent in maximum light emission efficiency. The aromatic hydrocarbon containing a 5-membered ring or a 6-membered ring as the aromatic hydrocarbon represented by Ar^{B1} includes aromatic hydrocarbons containing a 5-membered ring or a 6-membered ring exemplified in the section of the aromatic hydrocarbon group described above.

The aromatic hydrocarbon represented by Ar^{B1} is preferably benzene, naphthalene, anthracene, phenanthrene, dihydrophenanthrene, fluorene, benzoanthracene, benzophenanthrene, benzofluorene, dibenzoanthracene, dibenzophenanthrene, dibenzofluorene, indenofluorene, spirobifluorene or benzospirobifluorene, more preferably benzene, naphthalene, anthracene, phenanthrene, dihydrophenanthrene, fluorene, benzoanthracene, benzophenanthrene, benzofluorene, dibenzoanthracene, dibenzophenanthrene, dibenzofluorene or indenofluorene, further preferably benzene, naphthalene, anthracene, phenanthrene, dihydrophenanthrene or fluorene, particularly preferably benzene, naphthalene or fluorene, and especially preferably benzene, since the light emitting device of the present embodiment is further excellent in maximum light emission efficiency.

The heterocyclic compound represented by Ar^{B1} includes, for example, heterocyclic compounds (preferably, aromatic heterocyclic compounds) exemplified in the section of the hetero ring group described above, and it is preferably a monocyclic or bicyclic to heptacyclic heterocyclic compound (preferably, an aromatic heterocyclic compound), and more preferably a monocyclic or bicyclic to pentacyclic heterocyclic compound (preferably, an aromatic heterocyclic compound), since the light emitting device of the present embodiment is more excellent in maximum light emission efficiency. In addition, the heterocyclic compound represented by Ar^{B1} is preferably a heterocyclic compound (preferably, an aromatic heterocyclic compound) containing a 5-membered ring or a 6-membered ring, since the light emitting device of the present embodiment is more excellent in maximum light emission efficiency. The heterocyclic compound containing a 5-membered ring or a 6-membered ring as the heterocyclic compound represented by Ar^{B1} includes heterocyclic compounds containing a 5-membered ring or a 6-membered ring exemplified in the section of the hetero ring group described above.

The heterocyclic compound represented by Ar^{B1} is preferably furan, thiophene, oxadiazole, thiadiazole, diazole, triazole, pyridine, diazabenzene, triazine, azanaphthalene, diazanaphthalene, benzofuran, benzothiophene, benzodiazole, benzotriazole, dibenzofuran, dibenzothiophene, carbazole, azacarbazole, diazacarbazole, phenoxazine, phenothiazine, 9,10-dihydroacridine, 5,10-dihydrophenazine, azaanthracene, diazaanthracene, azaphenanthrene, diazaphenanthrene, benzocarbazole, azabenzocarbazole, diazabenzocarbazole, benzonaphthofuran, benzonaphthothiophene, dibenzocarbazole, indolocarbazole, indenocarbazole, azaindolocarbazole, diazaindolocarbazole, azaindenocarbazole, diazaindenocarbazole, carbazolocarbazole, benzoindolocarbazole, benzoindenocarbazole, dibenzoindolocarbazole or dibenzoindenocarbazole, more preferably furan, thiophene, oxadiazole, thiadiazole, diazole, triazole, pyridine, diazabenzene, triazine, azanaphthalene, diazanaphthalene, benzofuran, benzothiophene, benzodiazole, benzotriazole, dibenzofuran, dibenzothiophene, carbazole, azacarbazole, diazacarbazole, phenoxazine, phenothiazine, 9,10-dihydroacridine, 5,10-dihydrophenazine, azaanthracene, diazaanthracene, azaphenanthrene, diazaphenanthrene, benzocarbazole, azabenzocarbazole, diazabenzocarbazole, benzonaphthofuran, benzonaphthothiophene, dibenzocarbazole, indolocarbazole, indenocarbazole, azaindolocarbazole, diazaindolocarbazole, azaindenocarbazole or a diazaindenocarbazole, further preferably oxadiazole, thiadiazole, diazole, triazole, pyridine, diazabenzene, triazine, azanaphthalene, diazanaphthalene, benzodiazole, benzotriazole, dibenzofuran, dibenzothiophene, carbazole, azacarbazole, diazacarbazole, phenoxazine, phenothiazine, 9,10-dihydroacridine, 5,10-dihydrophenazine, azaanthracene, diazaanthracene, azaphenanthrene, diazaphenanthrene, benzocarbazole, azabenzocarbazole, diazabenzocarbazole, dibenzocarbazole, indolocarbazole, indenocarbazole, azaindolocarbazole, diazaindolocarbazole, azaindenocarbazole or a diazaindenocarbazole, particularly preferably pyridine, diazabenzene, triazine, benzodiazole, benzotriazole, azanaphthalene, diazanaphthalene, dibenzofuran, dibenzothiophene, carbazole, azacarbazole, diazacarbazole, phenoxazine, phenothiazine, 9,10-dihydroacridine, 5,10-dihydrophenazine, benzocarbazole, dibenzocarbazole, indolocarbazole or indenocarbazole, especially preferably pyridine, diazabenzene, triazine, dibenzofuran, dibenzothiophene, carbazole, phenoxazine, phenothiazine, 9,10-dihydroacridine, 5,10-dihydrophenazine, benzocarbazole, dibenzocarbazole, indolocarbazole or indenocarbazole, and especially more preferably pyridine, diazabenzene, triazine, carbazole, benzocarbazole, dibenzocarbazole, indolocarbazole or indenocarbazole, since the light emitting device of the present embodiment is further excellent in maximum light emission efficiency.

Ar^{B1} is preferably a group obtained by removing from an aromatic hydrocarbon n^{B1} hydrogen atoms bonding directly to carbon atoms constituting the ring or a group obtained by removing from a heterocyclic compound n^{B1} hydrogen atoms bonding directly to atoms constituting the ring, and more preferably a group obtained by removing from a heterocyclic compound n^{B1} hydrogen atoms bonding directly to atoms constituting the ring, since the light emitting device of the present embodiment is more excellent in maximum light emission efficiency, and these groups optionally have a substituent.

When Ar^{B1} is an aromatic hydrocarbon group or a hetero ring group, Ar^{B1} is preferably a group obtained by removing from a monocyclic or bicyclic to pentacyclic aromatic hydrocarbon n^{B1} hydrogen atoms bonding directly to carbon atoms constituting the ring or a group represented by a monocyclic or bicyclic to pentacyclic heterocyclic compound (preferably, an aromatic heterocyclic compound) n^{B1} hydrogen atoms bonding directly to atoms constituting the ring, more preferably a group obtained by removing from a monocyclic, bicyclic or a tricyclic aromatic hydrocarbon n^{B1} hydrogen atoms bonding directly to carbon atoms constituting the ring or a group obtained by removing from a monocyclic or bicyclic to pentacyclic heterocyclic compound (preferably, an aromatic heterocyclic compound) n^{B1} hydrogen atoms bonding directly to atoms constituting the ring, further preferably a group obtained by removing from a monocyclic aromatic hydrocarbon n^{B1} hydrogen atoms bonding directly to carbon atoms constituting the ring or a group obtained by removing from a monocyclic or bicyclic to pentacyclic heterocyclic compound (preferably, an aromatic heterocyclic compound) n^{B1} hydrogen atoms bonding directly to atoms constituting the ring, and particularly preferably a group obtained by removing from a monocyclic or bicyclic to pentacyclic heterocyclic compound (preferably, an aromatic heterocyclic compound) n^{B1} hydrogen atoms bonding directly to atoms constituting the ring, since the light emitting device of the present embodiment is more excellent in maximum light emission efficiency, and these groups optionally have a substituent.

When Ar^{B1} is an aromatic hydrocarbon group or a hetero ring group, Ar^{B1} is preferably a group obtained by removing from benzene, naphthalene, fluorene, pyridine, diazabenzene, triazine, benzodiazole, benzotriazole, azanaphthalene, diazanaphthalene, dibenzofuran, dibenzothiophene, carbazole, azacarbazole, diazacarbazole, phenoxazine, phenothiazine, 9,10-dihydroacridine, 5,10-dihydrophenazine, benzocarbazole, dibenzocarbazole, indolocarbazole or indenocarbazole n^{B1} hydrogen atoms bonding directly to atoms constituting the ring, more preferably a group obtained by removing from benzene, pyridine, diazabenzene, triazine, benzodiazole, benzotriazole, azanaphthalene, diazanaphthalene, dibenzofuran, dibenzothiophene, carbazole, azacarbazole, diazacarbazole, phenoxazine, phenothiazine, 9,10-dihydroacridine, 5,10-dihydrophenazine, benzocarbazole, dibenzocarbazole, indolocarbazole or indenocarbazole n^{B1} hydrogen atoms bonding directly to atoms constituting the ring, further preferably a group obtained by removing from pyridine, diazabenzene, triazine, dibenzofuran, dibenzothiophene, carbazole, phenoxazine, phenothiazine, 9,10-dihydroacridine, 5,10-dihydrophenazine, benzocarbazole, dibenzocarbazole, indolocarbazole or indenocarbazole n^{B1} hydrogen atoms bonding directly to atoms constituting the ring, and particularly preferably a group obtained by removing from pyridine, diazabenzene, triazine, carbazole, benzocarbazole, dibenzocarbazole, indolocarbazole or indenocarbazole n^{B1} hydrogen atoms bonding directly to atoms constituting the ring, since the light emitting device of the present embodiment is further excellent in maximum light emission efficiency, and these groups optionally have a substituent.

It is preferable that Ar^{B1} is a group obtained by removing n^{B1} hydrogen atoms from a metal complex represented by the formula (1), since the light emitting device of the present embodiment is more excellent in maximum light emission efficiency, and the group optionally has a substituent.

### (Metal complex represented by the formula (1))

The metal complex represented by the formula (1) is preferably a metal complex showing phosphorescence at room temperature, and more preferably a metal complex showing light emission from triplet excited state at room temperature.

M is preferably an iridium atom or a platinum atom, and more preferably an iridium atom, since the light emitting device of the present embodiment is more excellent in maximum light emission efficiency.

When M is a rhodium atom or an iridium atom, n¹ is preferably 2 or 3, and more preferably 3.

When M is a palladium atom or a platinum atom, n¹ is preferably 2.

It is preferable that at least one of E¹ and E² is a carbon atom, and it is more preferable that E¹ and E² are carbon atoms.

E¹ and E² are preferably the same, since the metal complex represented by the formula (1) can be easily synthesized. When a plurality of E¹ are present, they are preferably the same, since the metal complex represented by the formula (1) can be easily synthesized. When a plurality of E² are present, they are preferably the same, since the metal complex represented by the formula (1) can be easily synthesized.

The number of carbon atoms of the aromatic hetero ring represented by Ring L¹ is, not including the number of carbon atoms of the substituent, preferably 1 to 30, and more preferably 2 to 15. The number of hetero atoms of the aromatic hetero ring represented by Ring L¹ is, not including the number of hetero atoms of the substituent, preferably 1 to 10, more preferably 1 to 5, and further preferably 1 to 3.

Ring L¹ includes, for example, aromatic hetero rings containing 1 or more nitrogen atoms in the ring among aromatic hetero rings exemplified in the section of the hetero ring group described above, and the aromatic hetero ring optionally has a substituent. Of them, Ring L¹ is preferably an aromatic hetero ring containing a 5-membered ring or an aromatic hetero ring containing a 6-membered ring, more preferably an aromatic hetero ring containing a 5-membered ring containing 2 or more and 4 or less nitrogen atoms in the ring or an aromatic hetero ring containing a 6-membered ring containing 1 or more and 4 or less nitrogen atoms in the ring, further preferably an aromatic hetero ring containing a 5-membered ring containing 2 or 3 nitrogen atoms in the ring or an aromatic hetero ring containing a 6-membered ring containing 1 or 2 nitrogen atoms in the ring, and particularly preferably an aromatic hetero ring containing a 6-membered ring containing 1 or 2 nitrogen atoms in the ring, since the light emitting device of the present embodiment is more excellent in maximum light emission efficiency, and these rings optionally have a substituent. When Ring L¹ is a 6-membered aromatic hetero ring, E¹ is preferably a carbon atom.

Ring L¹ is preferably a pyridine ring, a diazabenzene ring, an azanaphthalene ring, a diazanaphthalene ring, a triazole ring or a diazole ring, more preferably a pyridine ring, an azanaphthalene ring, a triazole ring or a diazole ring, further preferably a pyridine ring or an azanaphthalene ring, and particularly preferably a pyridine ring, since the light emitting device of the present embodiment is further excellent in maximum light emission efficiency, and these rings optionally have a substituent.

When a plurality of Ring L¹ are present, it is preferable that at least two of the plurality of Ring L¹ are the same, and it is more preferable that all of the plurality of Ring L¹ are the same, since the metal complex represented by the formula (1) can be easily synthesized.

The number of carbon atoms of the aromatic hydrocarbon ring represented by Ring L² is, not including the number of carbon atoms of the substituent, preferably 6 to 40, and more preferably 6 to 20.

The aromatic hydrocarbon ring represented by Ring L² includes, for example, aromatic hydrocarbon rings exemplified in the section of the aromatic hydrocarbon group described above, and the aromatic hydrocarbon ring optionally has a substituent. Of them, the aromatic hydrocarbon ring represented by Ring L² is preferably a monocyclic, bicyclic or tricyclic aromatic hydrocarbon ring exemplified in the section of the aromatic hydrocarbon group described above, more preferably a benzene ring, a naphthalene ring, a fluorene ring, a phenanthrene ring or a dihydrophenanthrene ring, further preferably a benzene ring, a fluorene ring or a dihydrophenanthrene ring, and particularly preferably a benzene ring, since the light emitting device of the present embodiment is more excellent in maximum light emission efficiency, and these rings optionally have a substituent.

The number of carbon atoms of the aromatic hetero ring represented by Ring L² is, not including the number of carbon atoms of the substituent, preferably 1 to 30, and more preferably 2 to 15. The number of hetero atoms of the aromatic hetero ring represented by Ring L² is, not including the number of hetero atoms of the substituent, preferably 1 to 10, more preferably 1 to 5, and further preferably 1 to 3.

The aromatic hetero ring represented by Ring L² includes, for example, aromatic hetero rings exemplified in the section of the hetero ring group described above, and the aromatic hetero ring optionally has a substituent. Of them, the aromatic hetero ring represented by Ring L² is preferably a monocyclic, bicyclic or tricyclic aromatic hetero ring exemplified in the section of the hetero ring group described above, more preferably a pyridine ring, a diazabenzene ring, an azanaphthalene ring, a diazanaphthalene ring, an indole ring, a benzofuran ring, a benzothiophene ring, a carbazole ring, an azacarbazole ring, a diazacarbazole ring, a dibenzofuran ring or a dibenzothiophene ring, further preferably a pyridine ring, a diazabenzene ring, a carbazole ring, a dibenzofuran ring or a dibenzothiophene ring, and particularly preferably a pyridine ring or a diazabenzene ring, since the light emitting device of the present embodiment is more excellent in maximum light emission efficiency, and these rings optionally have a substituent.

Ring L² is preferably a benzene ring, a pyridine ring or a diazabenzene ring, and more preferably a benzene ring, since the light emitting device of the present embodiment is more excellent in maximum light emission efficiency, and these rings optionally have a substituent.

When a plurality of Ring L² are present, it is preferable that at least two of the plurality of Ring L² is the same, and it is more preferable that all of the plurality of Ring L² are the same, since the metal complex represented by the formula (1) can be easily synthesized.

It is preferable that Ring L¹ is a pyridine ring, a diazabenzene ring, an azanaphthalene ring, a diazanaphthalene ring, a triazole ring or a diazole ring, and Ring L² is a benzene ring, a pyridine ring or a diazabenzene ring, it is more preferable that Ring L¹ is a pyridine ring, an azanaphthalene ring, a triazole ring or a diazole ring, and Ring L² is a benzene ring, it is further preferable that Ring L¹ is a pyridine ring or an azanaphthalene ring, and Ring L² is a benzene ring, and it is particularly preferable that Ring L¹ is a pyridine ring, and Ring L² is a benzene ring, since the light emitting device of the present embodiment is further excellent in maximum light emission efficiency, and these rings optionally have a substituent.

The substituent which Ring L¹ and Ring L² optionally have (hereinafter, referred to also as "primary substituent") is preferably an alkyl group, a cycloalkyl group, an alkoxy group, a cycloalkoxy group, an aryl group, an aryloxy group, a monovalent hetero ring group, a substituted amino group or a fluorine atom, more preferably an alkyl group, a cycloalkyl group, an aryl group, a monovalent hetero ring group or a substituted amino group, further preferably an alkyl group, a cycloalkyl group, an aryl group or a monovalent hetero ring group, and particularly preferably an alkyl group, a cycloalkyl group or an aryl group, and these groups optionally further have a substituent (hereinafter, referred to also as "secondary substituent").

It is preferable that at least one of Ring L¹ and Ring L² has a primary substituent, since the light emitting device of the present embodiment is more excellent in maximum light emission efficiency.

In the metal complex represented by the formula (1), when at least one of Ring L¹ and Ring L² has a primary substituent and when a plurality of Ring L¹ and Ring L² are present, at least one of a plurality of Ring L¹ and Ring L² may have a primary substituent, and it is preferable that at least two of the plurality of Ring L¹ and Ring L² have a primary substituent, and it is more preferable that at least three of the plurality of Ring L¹ and Ring L² have a primary substituent, since the light emitting device of the present embodiment is more excellent in maximum light emission efficiency. In addition, in the metal complex represented by the formula (1), when at least one of Ring L¹ and Ring L² has a primary substituent and when a plurality of Ring L¹ and Ring L² are present, it is preferable that at least two of the plurality of Ring L¹ have a primary substituent or at least two of the plurality of Ring L² have a primary substituent, and it is more preferable that all of the plurality of Ring L¹ have a primary substituent or all of the plurality of Ring L² have a primary substituent, since the light emitting device of the present embodiment is more excellent in maximum light emission efficiency.

In the metal complex represented by the formula (1), when at least one of Ring L¹ and Ring L² has a primary substituent, the number of primary substituents which at least one of Ring L¹ and Ring L² has is usually 1 to 10, preferably 1 to 5, more preferably 1 to 3, further preferably 1 or 2, and particularly preferably 1, since the metal complex represented by the formula (1) can be easily synthesized and the light emitting device of the present embodiment is more excellent in maximum light emission efficiency.

In the metal complex represented by the formula (1), when at least one of Ring L¹ and Ring L² has a primary substituent and when M is a rhodium atom or an iridium atom, the number of primary substituents which Ring L¹ and Ring L² have is usually 1 to 30, and it is preferably 1 to 18, more preferably 2 to 12, and further preferably 3 to 6, since the metal complex represented by the formula (1) can be easily synthesized and the light emitting device of the present embodiment is more excellent in maximum light emission efficiency.

In the metal complex represented by the formula (1), when at least one of Ring L¹ and Ring L² has a primary substituent and when M is a palladium atom or a platinum atom, the number of primary substituents which Ring L¹ and Ring L² have is usually 1 to 20, and it is preferably 1 to 12, more preferably 1 to 8, and further preferably 2 to 4, since the metal complex represented by the formula (1) can be easily synthesized and the light emitting device of the present embodiment is more excellent in maximum light emission efficiency.

The aryl group as the primary substituent is preferably a group obtained by removing from a monocyclic, bicyclic or tricyclic aromatic hydrocarbon one hydrogen atom bonding directly to a carbon atom constituting the ring, more preferably a phenyl group, a naphthyl group or a fluorenyl group, and further preferably a phenyl group, and these groups optionally have a substituent.

The monovalent hetero ring group as the primary substituent is preferably a group obtained by removing from a monocyclic, bicyclic or tricyclic heterocyclic compound one hydrogen atom bonding directly to a carbon atom or hetero atom constituting the ring, more preferably a group obtained by removing from a pyridine ring, a diazabenzene ring, a triazine ring, an azanaphthalene ring, a diazanaphthalene ring, a carbazole ring, a dibenzofuran ring or a dibenzothiophene ring one hydrogen atom bonding directly to a carbon atom or hetero atom constituting the ring, and further preferably a group obtained by removing from a pyridine ring, a diazabenzene ring or a triazine ring one hydrogen atom bonding directly to a carbon atom constituting the ring, and these groups optionally have a substituent.

In the substituted amino group as the primary substituent, the substituent which an amino group has is preferably an aryl group or a monovalent hetero ring group, and more preferably an aryl group, and these groups optionally further have a substituent. The examples and preferable ranges of the aryl group and the monovalent hetero ring group as the substituent which an amino group has are the same as the examples and preferable ranges of the aryl group and the monovalent hetero ring group as primary substituent, respectively.

The examples and preferable range of the secondary substituent (the substituent which the primary substituent optionally further has) are the same as the examples and preferable range of the primary substituent.

The secondary substituent optionally further has a substituent (hereinafter, referred to also as "tertiary substituent"). The examples and preferable range of the tertiary substituent (the substituent which the secondary substituent optionally further has) are the same as the examples and preferable range of the primary substituent.

The tertiary substituent optionally further has a substituent (hereinafter, referred to also as "quaternary substituent"). The quaternary substituent (the substituent which the tertiary substituent optionally further has) is preferably an alkyl group, a cycloalkyl group, an alkoxy group, a cycloalkoxy group, an aryl group, an aryloxy group, a monovalent hetero ring group, a substituted amino group or a fluorine atom, more preferably an alkyl group, a cycloalkyl group, an aryl group, a monovalent hetero ring group or a substituted amino group, further preferably an alkyl group, a cycloalkyl group or an aryl group, and particularly preferably an alkyl group or a cycloalkyl group, and these groups optionally further have a substituent, but it is preferable not to further have a substituent since the metal complex represented by the formula (1) can be easily synthesized.

The examples and preferable ranges of the aryl group, the monovalent hetero ring group and the substituted amino group as the secondary substituent, the tertiary substituent and the quaternary substituent are the same as the examples and preferable ranges of the aryl group, the monovalent hetero ring group and the substituted amino group as the primary substituent, respectively.

### • Anionic bidentate ligand

The anionic bidentate ligand represented by A¹-G¹-A² includes, for example, ligands represented by the following formulae. Note that the anionic bidentate ligand represented by A¹-G¹-A² is different from a ligand of which number is defined by an index n¹. 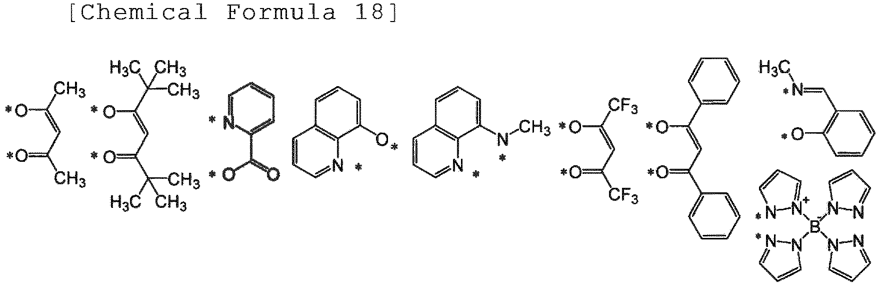 [wherein, * represents a site binding to M.]

When n^{B1} is 1 or more and Ar^{B1} is a metal complex group, it is preferable that at least one of Ring L¹ and Ring L² has a group represented by Ar^{B2} (namely, a group represented by the formula (D-A) or a group represented by the formula (D-B)), since the light emitting device of the present embodiment is more excellent in maximum light emission efficiency.

In the metal complex represented by the formula (1), when at least one of Ring L¹ and Ring L² has a group represented by Ar^{B2} and when a plurality of Ring L¹ and Ring L² are present, at least one of the plurality of Ring L¹ and Ring L² may have a group represented by Ar^{B2}, and it is preferable that at least two of the plurality of Ring L¹ and Ring L² have a group represented by Ar^{B2}, and it is more preferable that at least three of the plurality of Ring L¹ and Ring L² have a group represented by Ar^{B2}, since the light emitting device of the present embodiment is more excellent in maximum light emission efficiency. In addition, in the metal complex represented by the formula (1), when at least one of Ring L¹ and Ring L² has a group represented by Ar^{B2} and when a plurality of Ring L¹ and Ring L² are present, it is preferable that at least two of the plurality of Ring L¹ have a group represented by Ar^{B2} or at least two of the plurality of Ring L² have a group represented by Ar^{B2}, and it is more preferable that all of the plurality of Ring L¹ have a group represented by Ar^{B2} or all of the plurality of Ring L² have a group represented by Ar^{B2}, since the light emitting device of the present embodiment is more excellent in maximum light emission efficiency. In addition, in the metal complex represented by the formula (1), when at least one of Ring L¹ and Ring L² has a group represented by Ar^{B2} and when a plurality of Ring L¹ and Ring L² are present, it is preferable that at least two of the plurality of Ring L² have a group represented by Ar^{B2}, and it is more preferable that all of the plurality of Ring L² have a group represented by Ar^{B2}, since the light emitting device of the present embodiment is further excellent in maximum light emission efficiency.

In the metal complex represented by the formula (1), when at least one of Ring L¹ and Ring L² has a group represented by Ar^{B2}, the number of groups represented by Ar^{B2} which at least one of Ring L¹ and Ring L² has is usually 1 to 10, and it is preferably 1 to 5, more preferably 1 to 3, further preferably 1 or 2, and particularly preferably 1, since the metal complex represented by the formula (1) can be easily synthesized and the light emitting device of the present embodiment is more excellent in maximum light emission efficiency.

In the metal complex represented by the formula (1), when at least one of Ring L¹ and Ring L² has a group represented by Ar^{B2} and when M is a rhodium atom or an iridium atom, the total number of groups represented by Ar^{B2} which Ring L¹ and Ring L² have is usually 1 to 30, and it is preferably 1 to 18, more preferably 2 to 12, and further preferably 3 to 6, since the metal complex represented by the formula (1) can be easily synthesized and the light emitting device of the present embodiment is more excellent in maximum light emission efficiency.

In the metal complex represented by the formula (1), when at least one of Ring L¹ and Ring L² has a group represented by Ar^{B2} and when M is a palladium atom or a platinum atom, the total number of groups represented by Ar^{B2} which Ring L¹ and Ring L² have is usually 1 to 20, and it is preferably 1 to 12, more preferably 1 to 8, and further preferably 2 to 4, since the metal complex represented by the formula (1) can be easily synthesized and the light emitting device of the present embodiment is more excellent in maximum light emission efficiency.

The substituent which Ar^{B1} optionally has is preferably a halogen atom, a cyano group, an alkyl group, a cycloalkyl group, an alkoxy group, a cycloalkoxy group, an aryl group, a monovalent hetero ring group or a substituted amino group, more preferably an alkyl group, a cycloalkyl group, an aryl group, a monovalent hetero ring group or a substituted amino group, further preferably an alkyl group, a cycloalkyl group, an aryl group or a monovalent hetero ring group, and particularly preferably an alkyl group, a cycloalkyl group or an aryl group, and these groups optionally further have a substituent.

The aryl group as the substituent which Ar^{B1} optionally has is preferably a group obtained by removing from an aromatic hydrocarbon explained in the section of the aromatic hydrocarbon represented by Ar^{B1} one hydrogen atom bonding directly to a carbon atom constituting the ring, since the light emitting device of the present embodiment is more excellent in maximum light emission efficiency, and the group optionally has a substituent.

The examples and preferable range of the aromatic hydrocarbon for the aryl group as the substituent which Ar^{B1} optionally has are the same as the examples and preferable range of the aromatic hydrocarbon represented by Ar^{B1}.

The monovalent hetero ring group as the substituent which Ar^{B1} optionally has is preferably a group obtained by removing from a heterocyclic compound explained in the section of the heterocyclic compound represented by Ar^{B1} one hydrogen atom bonding directly to an atom constituting the ring, since the light emitting device of the present embodiment is more excellent in maximum light emission efficiency, and the group optionally has a substituent.

The examples and preferable range of the heterocyclic compound for the monovalent hetero ring group as the substituent which Ar^{B1} optionally has are the same as the examples and preferable range of the heterocyclic compound represented by Ar^{B1}.

In the substituted amino group as the substituent which Ar^{B1} optionally has, the substituent which an amino group has is preferably an aryl group or a monovalent hetero ring group, and more preferably an aryl group, and these groups optionally further have a substituent. The examples and preferable range of the aryl group as the substituent which an amino group has are the same as the examples and preferable range of the aryl group as the substituent which Ar^{B1} optionally has. The examples and preferable range of the monovalent hetero ring group as the substituent which an amino group has are the same as the examples and preferable range of the monovalent hetero ring group as the substituent which Ar^{B1} optionally has.

The substituent which the substituent which Ar^{B1} optionally has optionally further has is preferably a halogen atom, a cyano group, an alkyl group, a cycloalkyl group, an alkoxy group, a cycloalkoxy group, an aryl group, a monovalent hetero ring group or a substituted amino group, more preferably an alkyl group, a cycloalkyl group, an aryl group, a monovalent hetero ring group or a substituted amino group, further preferably an alkyl group, a cycloalkyl group or an aryl group, and particularly preferably an alkyl group or a cycloalkyl group, and these groups optionally further have a substituent, but it is preferable that they do not further have a substituent.

The examples and preferable range of the aryl group, the monovalent hetero ring group and the substituted amino group as the substituent which the substituent which Ar^{B1} optionally has optionally further has are the same as the examples and preferable range of the aryl group, the monovalent hetero ring group and the substituted amino group as the substituent which Ar^{B1} optionally has, respectively.

### (Group represented by the formula (D-A) or the formula (D-B))

m^{D A 1} is usually an integer of 1 or more and 10 or less, preferably an integer of 1 or more and 5 or less, more preferably an integer of 1 or more and 3 or less, further preferably 1 or 2, and particularly preferably 1.

m^{D A 2} to m^{D A 7} represent usually an integer of 0 or more and 10 or less, preferably an integer of 0 or more and 5 or less, more preferably an integer of 0 or more and 2 or less, and further preferably 0 or 1. It is preferable that m^{D A 2} to m^{D A 7} are the same integer.

The aromatic hydrocarbon group represented by G^{D A} is preferably a group obtained by removing from a monocyclic or bicyclic to hexacyclic aromatic hydrocarbon three hydrogen atoms bonding directly to carbon atoms constituting the ring, more preferably a group obtained by removing from a monocyclic, bicyclic or a tricyclic aromatic hydrocarbon three hydrogen atoms bonding directly to carbon atoms constituting the ring, further preferably a group obtained by removing from benzene, naphthalene, anthracene, phenanthrene, dihydrophenanthrene or fluorene three hydrogen atoms bonding directly to carbon atoms constituting the ring, particularly preferably a group obtained by removing from benzene, naphthalene or fluorene three hydrogen atoms bonding directly to carbon atoms constituting the ring, and especially preferably a group obtained by removing from benzene three hydrogen atoms bonding directly to carbon atoms constituting the ring, since the light emitting device of the present embodiment is more excellent in maximum light emission efficiency, and these groups optionally have a substituent.

The hetero ring group represented by G^{D A} is preferably a group obtained by removing from a monocyclic or bicyclic to hexacyclic heterocyclic compound three hydrogen atoms bonding directly to atoms constituting the ring, more preferably a group obtained by removing from a monocyclic, bicyclic or a tricyclic heterocyclic compound three hydrogen atoms bonding directly to atoms constituting the ring, further preferably a group obtained by removing from furan, thiophene, oxadiazole, thiadiazole, pyrrole, diazole, triazole, pyridine, diazabenzene, triazine, azanaphthalene, diazanaphthalene, benzofuran, benzothiophene, indole, azaindole, diazaindole, benzodiazole, benzotriazole, dibenzofuran, dibenzothiophene, carbazole, azacarbazole, diazacarbazole, phenoxazine, phenothiazine, 9,10-dihydroacridine, 5,10-dihydrophenazine, acridone, azaanthracene, diazaanthracene, azaphenanthrene or a diazaphenanthrene three hydrogen atoms bonding directly to atoms constituting the ring, particularly preferably a group obtained by removing from pyridine, diazabenzene, triazine, azanaphthalene, diazanaphthalene, dibenzofuran, dibenzothiophene, carbazole, azacarbazole, diazacarbazole, phenoxazine, phenothiazine, 9,10-dihydroacridine or 5,10-dihydrophenazine three hydrogen atoms bonding directly to atoms constituting the ring, especially preferably a group obtained by removing from pyridine, diazabenzene, triazine, carbazole, phenoxazine, phenothiazine, 9,10-dihydroacridine or 5,10-dihydrophenazine three hydrogen atoms bonding directly to atoms constituting the ring, especially more preferably a group obtained by removing from pyridine, diazabenzene, triazine or carbazole three hydrogen atoms bonding directly to atoms constituting the ring, and especially further preferably a group obtained by removing from pyridine, diazabenzene or a triazine three hydrogen atoms bonding directly to atoms constituting the ring, since the light emitting device of the present embodiment is more excellent in maximum light emission efficiency, and these groups optionally have a substituent.

G^{D A} is preferably a group obtained by removing from a monocyclic, bicyclic or a tricyclic aromatic hydrocarbon three hydrogen atoms bonding directly to carbon atoms constituting the ring or a group obtained by removing from a monocyclic, bicyclic or a tricyclic heterocyclic compound three hydrogen atoms bonding directly to atoms constituting the ring, more preferably a group obtained by removing from benzene, naphthalene, fluorene, pyridine, diazabenzene, triazine, azanaphthalene, diazanaphthalene, dibenzofuran, dibenzothiophene, carbazole, azacarbazole, diazacarbazole, phenoxazine, phenothiazine, 9,10-dihydroacridine or 5,10-dihydrophenazine three hydrogen atoms bonding directly to atoms constituting the ring, further preferably a group obtained by removing from benzene, fluorene, pyridine, diazabenzene, triazine, carbazole, phenoxazine, phenothiazine, 9,10-dihydroacridine or 5,10-dihydrophenazine three hydrogen atoms bonding directly to carbon atoms or nitrogen atoms constituting the ring, particularly preferably a group obtained by removing from benzene, pyridine, diazabenzene, triazine or carbazole three hydrogen atoms bonding directly to carbon atoms or nitrogen atoms constituting the ring, and especially preferably a group obtained by removing from benzene, pyridine, diazabenzene or a triazine three hydrogen atoms bonding directly to carbon atoms constituting the ring, since the light emitting device of the present embodiment is more excellent in maximum light emission efficiency, and these groups optionally have a substituent.

The examples and preferable range of the substituent which G^{D A} optionally has are the same as the examples and preferable range of the substituent which Ar^{B 1} optionally has.

G^{D A} is preferably a group represented by the formula (GDA-11) to the formula (GDA-15), more preferably a group represented by the formula (GDA-11) to the formula (GDA-14), and further preferably a group represented by the formula (GDA-11), since the light emitting device of the present embodiment is further excellent in maximum light emission efficiency. 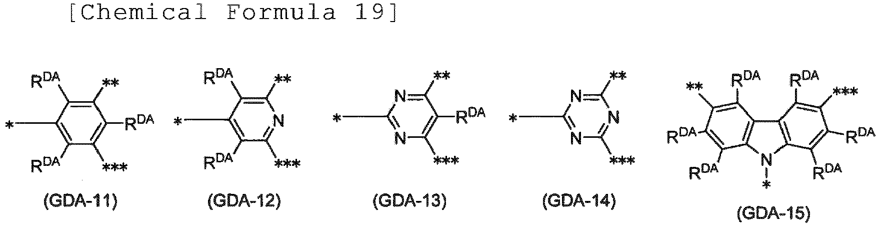 [wherein,
* represents a bond to Ar^{D A 1} in the formula (D-A), a bond to Ar^{D A 1} in the formula (D-B), a bond to Ar^{D A 2} in the formula (D-B), or a bond to Ar^{D A 3} in the formula (D-B).
** represents a bond to Ar^{D A 2} in the formula (D-A), a bond to Ar^{D A 2} in the formula (D-B), a bond to Ar^{D A 4} in the formula (D-B), or a bond to Ar^{D A 6} in the formula (D-B).
*** represents a bond to Ar^{D A 3} in the formula (D-A), a bond to Ar^{D A 3} in the formula (D-B), a bond to Ar^{D A 5} in the formula (D-B), or a bond to Ar^{D A 7} in the formula (D-B).

R^{D A} represents a hydrogen atom, a halogen atom, a cyano group, an alkyl group, a cycloalkyl group, an alkoxy group, a cycloalkoxy group, an aryl group, a monovalent hetero ring group or a substituted amino group, and these groups optionally have a substituent. When a plurality of the substituents are present, they may be the same or different and may be combined together to form a ring together with atoms to which they are attached. When a plurality of R^{D A} are present, they may be the same or different.]

R^{D A} is preferably a hydrogen atom, an alkyl group, a cycloalkyl group, an alkoxy group or a cycloalkoxy group, and more preferably a hydrogen atom, an alkyl group or a cycloalkyl group, and these groups optionally further have a substituent, but it is preferable that they do not further have a substituent.

The examples and preferable range of the aryl group, the monovalent hetero ring group and the substituted amino group represented by R^{D A} are the same as the examples and preferable range of the aryl group, the monovalent hetero ring group and the substituted amino group as the substituent which Ar^{B 1} optionally has, respectively.

The examples and preferable range of the substituent which R^{D A} optionally has are the same as the examples and preferable range of the substituent which the substituent which Ar^{B 1} optionally has optionally further has.

The arylene group represented by Ar^{D A 1} to Ar^{D A 7} is preferably a group obtained by removing from a monocyclic or bicyclic to hexacyclic aromatic hydrocarbon two hydrogen atoms bonding directly to carbon atoms constituting the ring, more preferably a group obtained by removing from a monocyclic, bicyclic or a tricyclic aromatic hydrocarbon two hydrogen atoms bonding directly to carbon atoms constituting the ring, further preferably a group obtained by removing from benzene, naphthalene, anthracene, phenanthrene, dihydrophenanthrene or fluorene two hydrogen atoms bonding directly to carbon atoms constituting the ring, particularly preferably a group obtained by removing from benzene, naphthalene or fluorene two hydrogen atoms bonding directly to carbon atoms constituting the ring, and especially preferably a phenylene group, since the light emitting device of the present embodiment is more excellent in maximum light emission efficiency, and these groups optionally have a substituent.

The divalent hetero ring group represented by Ar^{D A 1} to Ar^{D A 7} is preferably a group obtained by removing from a monocyclic or bicyclic to hexacyclic heterocyclic compound two hydrogen atoms bonding directly to atoms (preferably, carbon atoms) constituting the ring, more preferably a group obtained by removing from a monocyclic, bicyclic or a tricyclic heterocyclic compound two hydrogen atoms bonding directly to atoms (preferably, carbon atoms) constituting the ring, further preferably a group obtained by removing from pyridine, diazabenzene, triazine, azanaphthalene, diazanaphthalene, dibenzofuran, dibenzothiophene, carbazole, azacarbazole, diazacarbazole, phenoxazine, phenothiazine, 9,10-dihydroacridine or 5,10-dihydrophenazine two hydrogen atoms bonding directly to atoms (preferably, carbon atoms) constituting the ring, particularly preferably a group obtained by removing from pyridine, diazabenzene, triazine, azanaphthalene, diazanaphthalene, dibenzofuran, dibenzothiophene or carbazole two hydrogen atoms bonding directly to atoms (preferably, carbon atoms) constituting the ring, and especially preferably a group obtained by removing from pyridine, diazabenzene, triazine, carbazole, dibenzofuran or dibenzothiophene two hydrogen atoms bonding directly to atoms (preferably, carbon atoms) constituting the ring, since the light emitting device of the present embodiment is more excellent in maximum light emission efficiency, and these groups optionally have a substituent.

Ar^{D A 1} to Ar^{D A 7} represent preferably a group obtained by removing from a monocyclic, bicyclic or a tricyclic aromatic hydrocarbon two hydrogen atoms bonding directly to carbon atoms constituting the ring or a group obtained by removing from a monocyclic, bicyclic or a tricyclic heterocyclic compound two hydrogen atoms bonding directly to atoms constituting the ring, more preferably a group obtained by removing from benzene, naphthalene, fluorene, pyridine, diazabenzene, triazine, azanaphthalene, diazanaphthalene, dibenzofuran, dibenzothiophene or carbazole two hydrogen atoms bonding directly to carbon atoms or nitrogen atoms (preferably, carbon atoms) constituting the ring, further preferably a group obtained by removing from benzene, fluorene, pyridine, diazabenzene, triazine, dibenzofuran, dibenzothiophene or carbazole two hydrogen atoms bonding directly to carbon atoms or nitrogen atoms (preferably, carbon atoms) constituting the ring, particularly preferably a group obtained by removing from benzene, fluorene or carbazole two hydrogen atoms bonding directly to carbon atoms or nitrogen atoms (preferably, carbon atoms) constituting the ring, and especially preferably a phenylene group, since the light emitting device of the present embodiment is more excellent in maximum light emission efficiency, and these groups optionally have a substituent.

The examples and preferable range of the substituent which Ar^{D A 1} to Ar^{D A 7} optionally have are the same as the examples and preferable range of the substituent which Ar^{B 1} optionally has.

Ar^{D A 1} to Ar^{D A 7} represent preferably a group represented by the formula (ArDA-1) to the formula (ArDA-5), more preferably a group represented by the formula (ArDA-1) to the formula (ArDA-3), and further preferably a group represented by the formula (ArDA-1) or the formula (ArDA-2), since the light emitting device of the present embodiment is further excellent in maximum light emission efficiency. 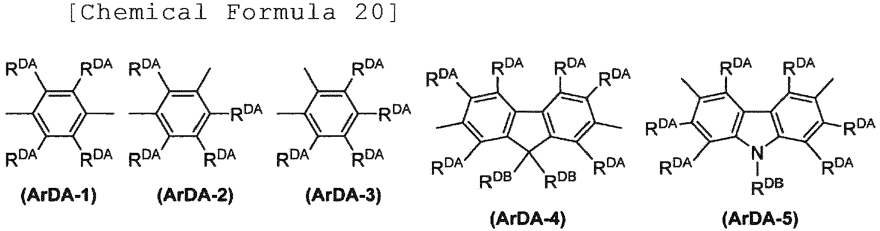 [wherein,
R^{D A} represents the same meaning as described above.

R^{D B} represents a hydrogen atom, a halogen atom, a cyano group, an alkyl group, a cycloalkyl group, an alkoxy group, a cycloalkoxy group, an aryl group, a monovalent hetero ring group or a substituted amino group, and these groups optionally have a substituent. When a plurality of the substituents are present, they may be the same or different and may be combined together to form a ring together with atoms to which they are attached. When a plurality of R^{D B} are present, they may be the same or different.]

R^{D B} represents preferably a hydrogen atom, an alkyl group, a cycloalkyl group, an aryl group or a monovalent hetero ring group, more preferably an alkyl group, a cycloalkyl group, an aryl group or a monovalent hetero ring group, further preferably an aryl group or a monovalent hetero ring group, and particularly preferably an aryl group, and these groups optionally have a substituent.

The examples and preferable range of the aryl group, the monovalent hetero ring group and the substituted amino group represented by R^{D B} are the same as the examples and preferable range of the aryl group, the monovalent hetero ring group and the substituted amino group as the substituent which Ar^{B 1} optionally has, respectively.

The examples and preferable range of the substituent which R^{D B} optionally has are the same as the examples and preferable range of the substituent which the substituent which Ar^{B 1} optionally has optionally further has.

The examples and preferable range of the aryl group and the monovalent hetero ring group represented by T^{D A} are the same as the examples and preferable range of the aryl group and the monovalent hetero ring group as the substituent which Ar^{B 1} optionally has, respectively.

T^{D A} is preferably a group obtained by removing from a monocyclic, bicyclic or a tricyclic aromatic hydrocarbon one hydrogen atom bonding directly to a carbon atom constituting the ring or a group obtained by removing from a monocyclic, bicyclic or a tricyclic heterocyclic compound one hydrogen atom bonding directly to an atom constituting the ring, more preferably a group obtained by removing from benzene, naphthalene, fluorene, pyridine, diazabenzene, triazine, carbazole, phenoxazine, phenothiazine, 9,10-dihydroacridine or 5,10-dihydrophenazine one hydrogen atom bonding directly to a carbon atom or a nitrogen atom constituting the ring, further preferably a group obtained by removing from benzene, fluorene, pyridine, diazabenzene, triazine or carbazole one hydrogen atom bonding directly to a carbon atom or a nitrogen atom constituting the ring, particularly preferably a group obtained by removing from benzene, fluorene or carbazole one hydrogen atom bonding directly to a carbon atom or a nitrogen atom constituting the ring, and particularly preferably a phenyl group, since the light emitting device of the present embodiment is more excellent in maximum light emission efficiency, and these groups optionally have a substituent.

The examples and preferable range of the substituent which T^{D A} optionally has are the same as the examples and preferable range of the substituent which the substituent which Ar^{B 1} optionally has optionally further has.

T^{D A} is preferably a group represented by the formula (TDA-1) to the formula (TDA-3), more preferably a group represented by the formula (TDA-1) or the formula (TDA-3), and further preferably a group represented by the formula (TDA-1), since the light emitting device of the present embodiment is further excellent in maximum light emission efficiency. 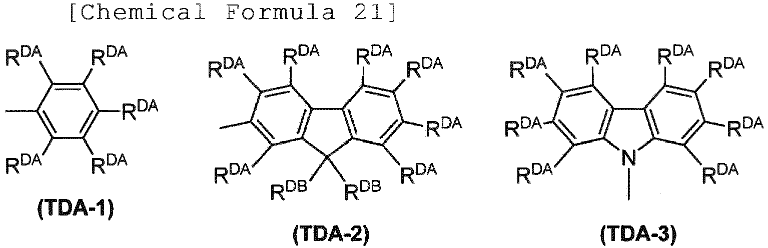 [wherein, R^{D A} and R^{D B} represent the same meaning as described above.]

The group represented by the formula (D-A) includes, for example, groups represented by the formulae (D-A1) to (D-A8). 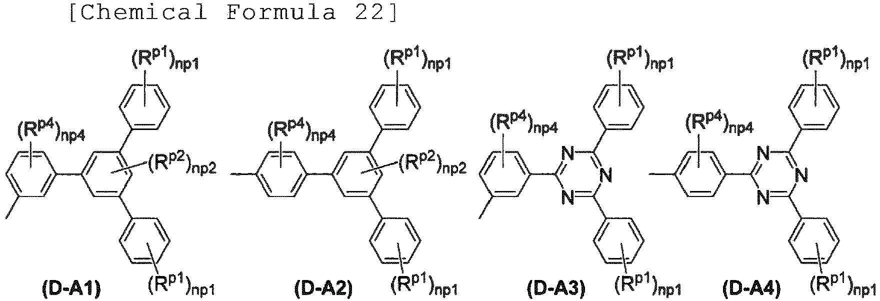 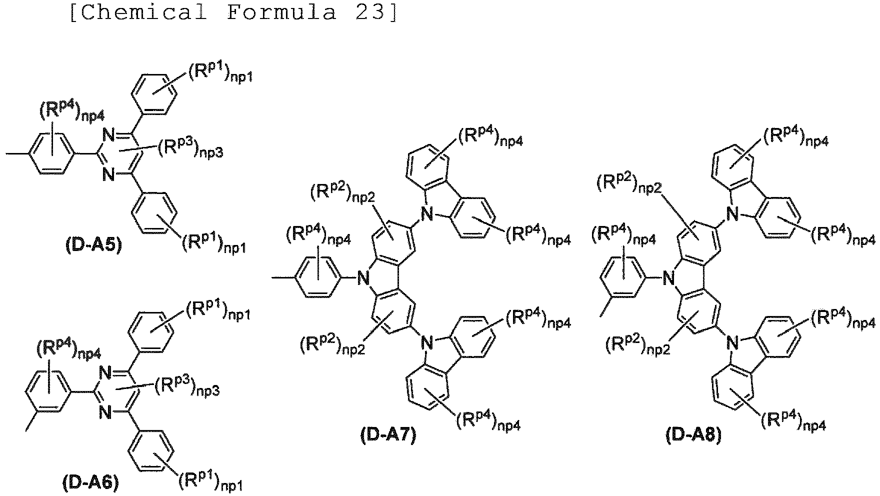 [wherein,
RP1, R^{p 2}, R^{p 3} and R^{p 4} each independently represent an alkyl group, a cycloalkyl group, an alkoxy group, a cycloalkoxy group or a halogen atom, and these groups optionally have a substituent. When a plurality of the substituents are present, they may be the same or different and may be combined together to form a ring together with atoms to which they are attached. When a plurality of R^{p 1}, R^{p 2} and R^{p 4} are present, they may be the same or different at each occurrence.

np1 represents an integer of 0 to 5, np2 represents an integer of 0 to 3, np3 represents 0 or 1, and np4 represents an integer of 0 to 4. A plurality of np1 may be the same or different. When a plurality of np2 are present, they may be the same or different. When a plurality of np4 are present, they may be the same or different.]

The group represented by the formula (D-B) includes, for example, groups represented by the formulae (D-B1) to (D-B6). 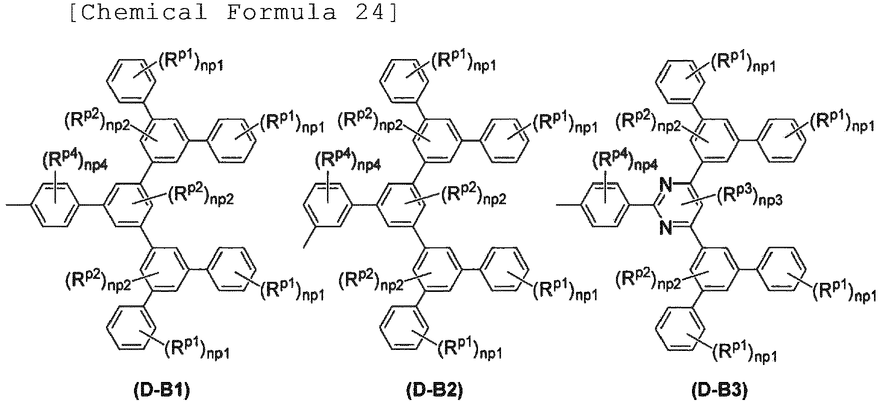 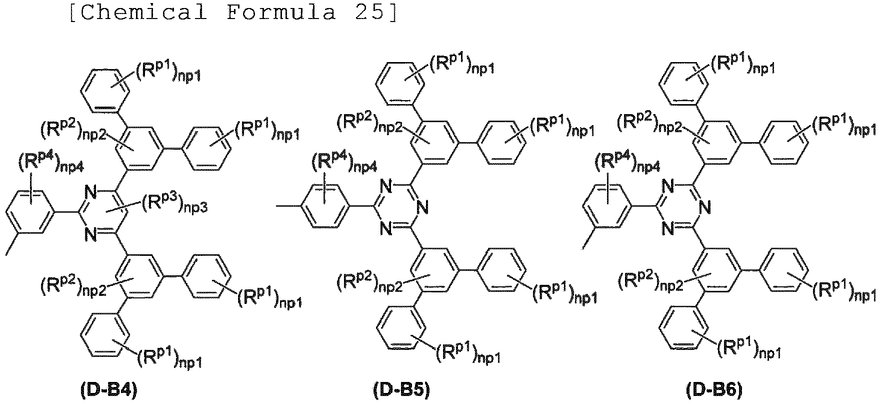 [wherein,
R^{p 1}, RP2, R^{p 3} and R^{p 4} each independently represent an alkyl group, a cycloalkyl group, an alkoxy group, a cycloalkoxy group or a halogen atom, and these groups optionally have a substituent. When a plurality of the substituents are present, they may be the same or different and may be combined together to form a ring together with atoms to which they are attached. When a plurality of R^{p 1}, R^{p 2} and R^{p 4} are present, they may be the same or different at each occurrence.

np1 represents an integer of 0 to 5, np2 represents an integer of 0 to 3, np3 represents 0 or 1, and np4 represents an integer of 0 to 4. A plurality of np1 may be the same or different. A plurality of np2 may be the same or different.]

np1 is preferably an integer of 0 to 2, and more preferably 0 or 1. np2 is preferably 0 or 1, and more preferably 0. np3 is preferably 0. np4 is preferably an integer of 0 to 2, and more preferably 0 or 1.

R^{p 1}, R^{p 2}, R^{p 3} and R^{p 4} represent preferably an alkyl group optionally having a substituent or a cycloalkyl group optionally having a substituent, more preferably an alkyl group optionally having a substituent, and further preferably an alkyl group having no substituent.

The examples and preferable range of the substituent which R^{p 1}, R^{p 2}, R^{p 3} and R^{p 4} optionally have are the same as the examples and preferable range of the substituent which the substituent which Ar^{B 1} optionally has optionally further has.

The compound represented by the formula (B) includes, for example, compounds represented by the following formulae. In the formulae, Z¹ represents a group represented by -N= or a group represented by - CH=. Z² represents an oxygen atom or a sulfur atom. A plurality of Z¹ and Z² each may be the same or different.

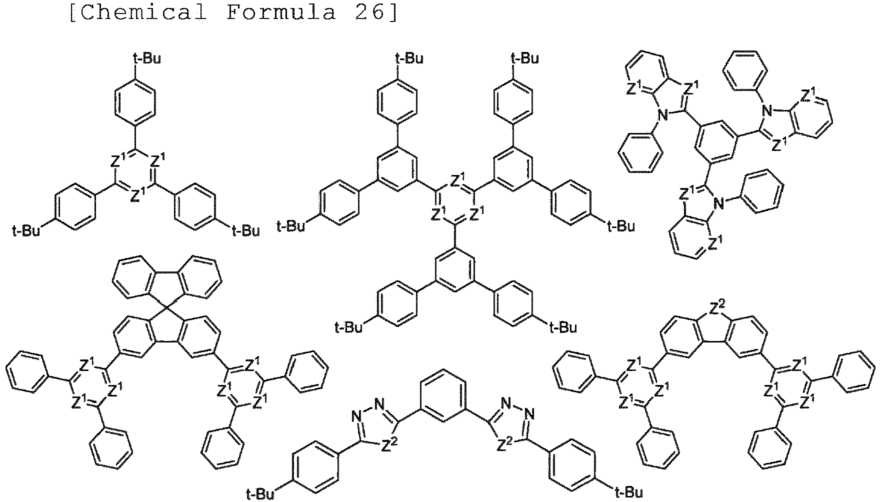

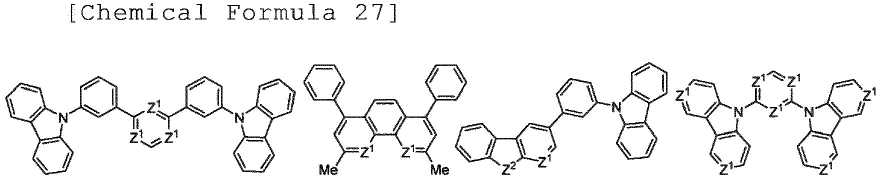

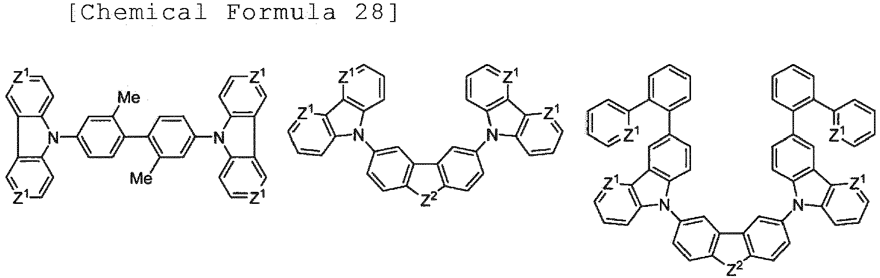

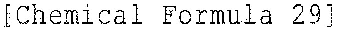

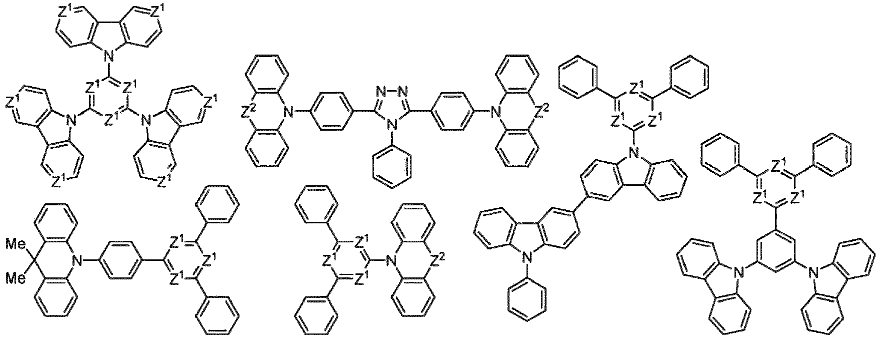

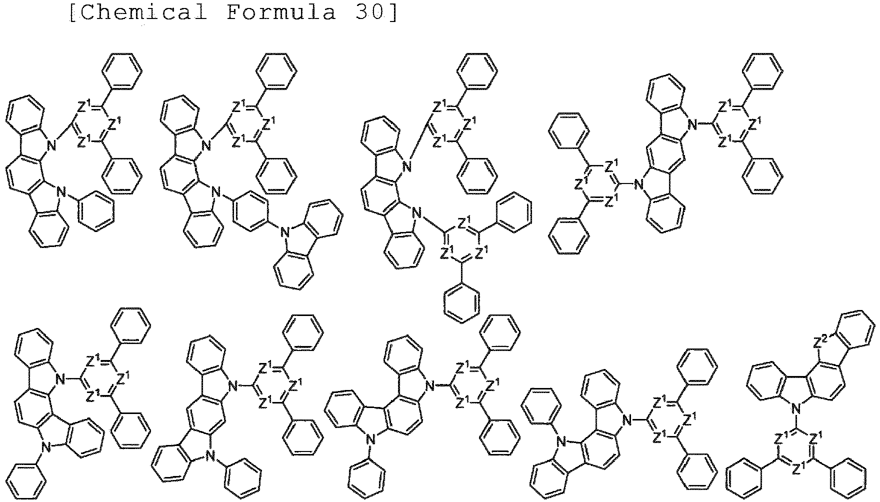

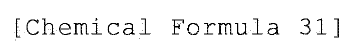

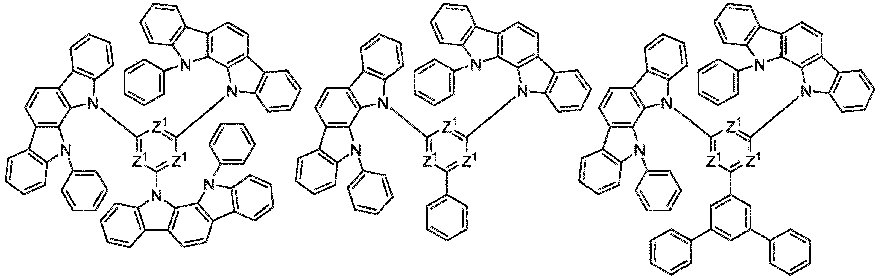

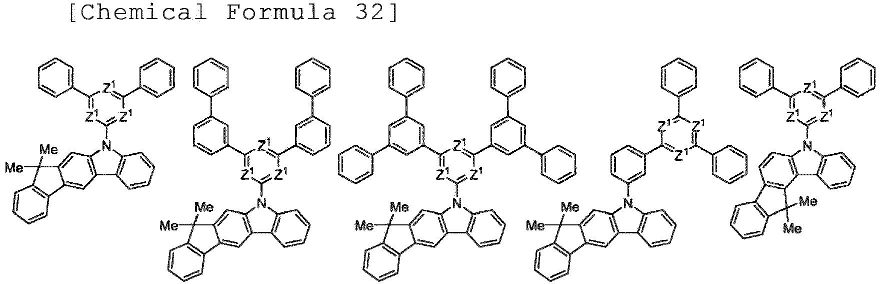

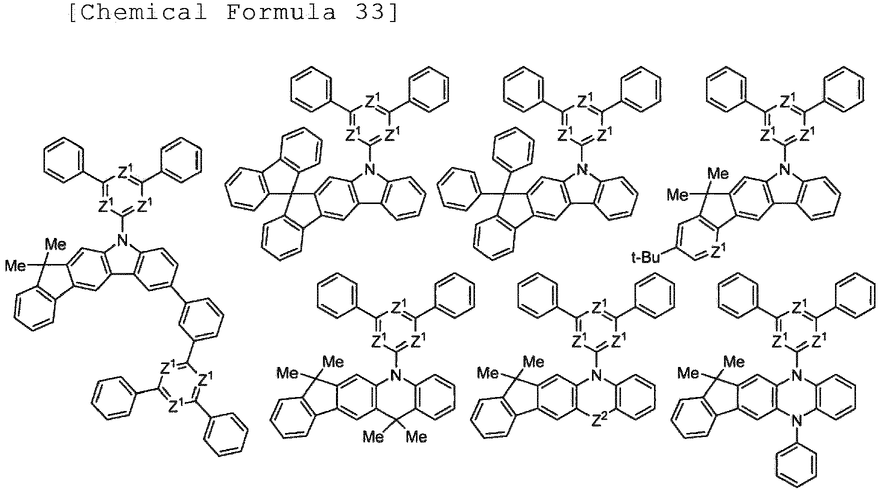

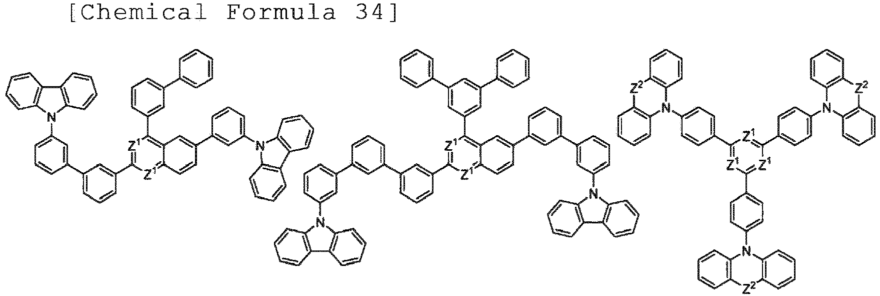

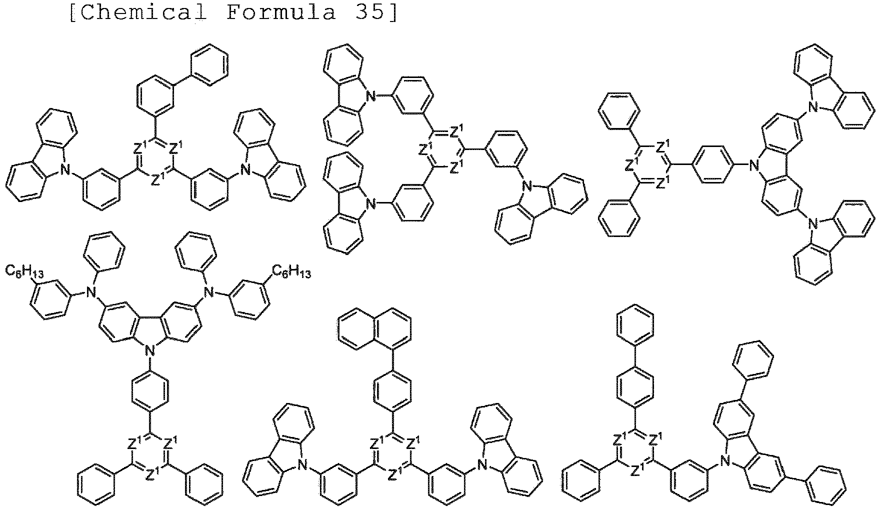

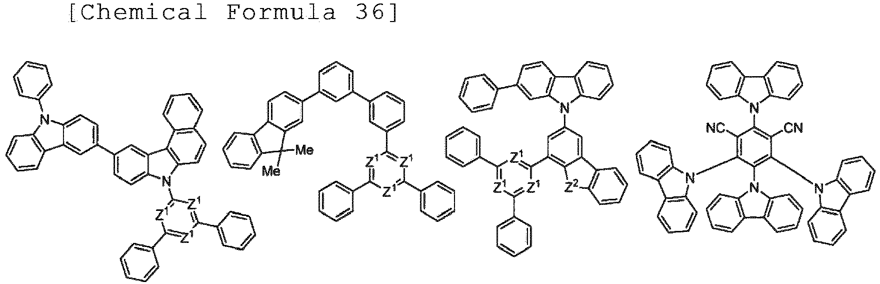

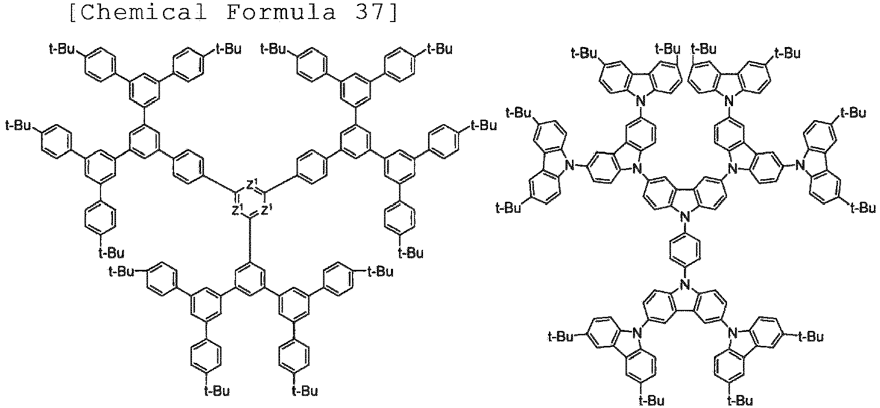

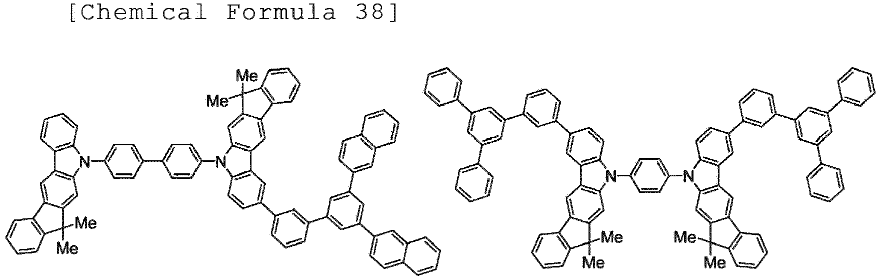

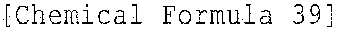

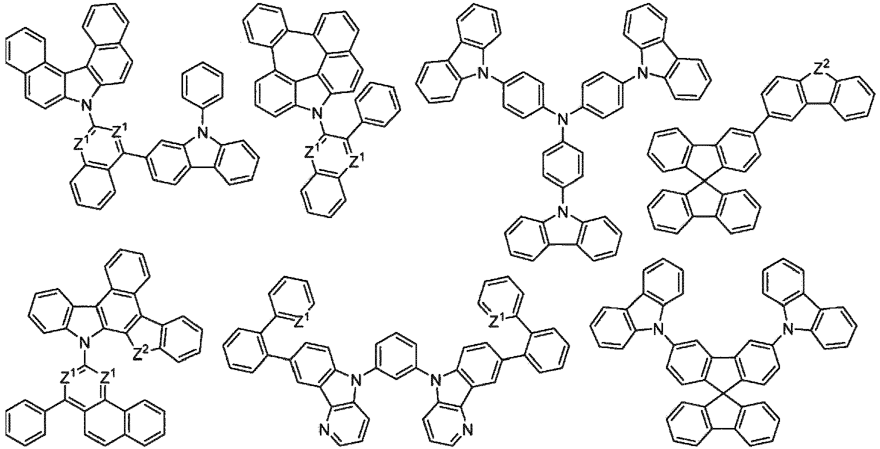

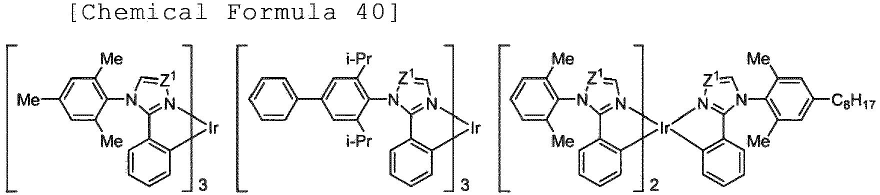

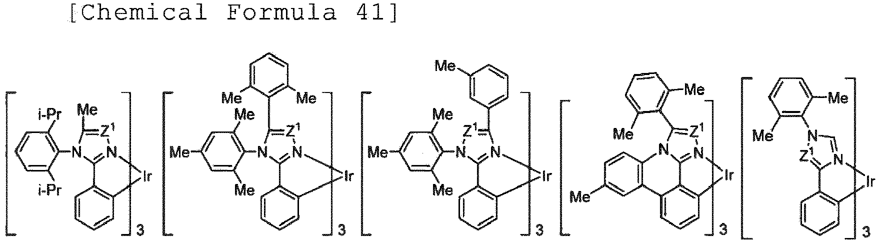

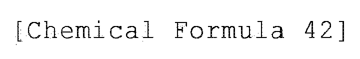

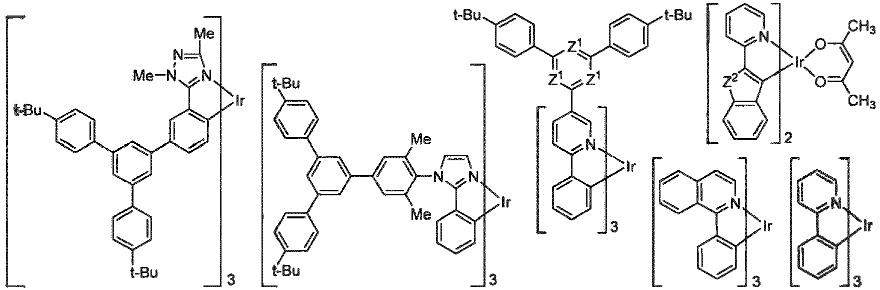

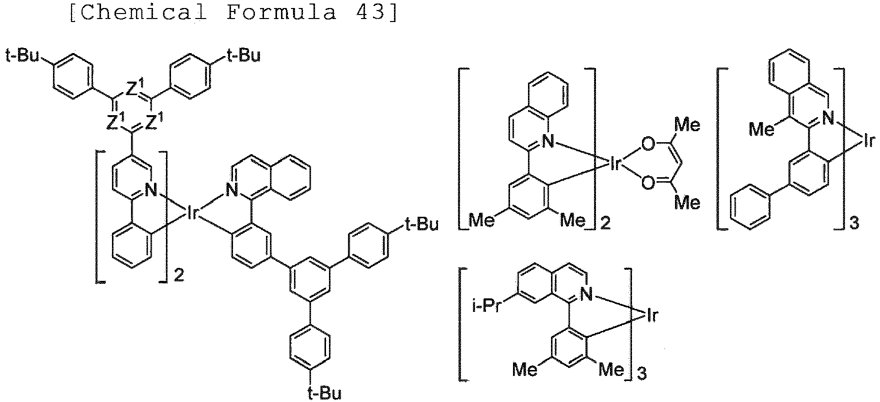

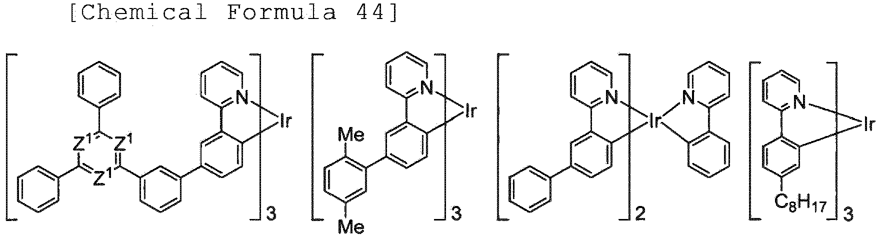

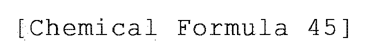

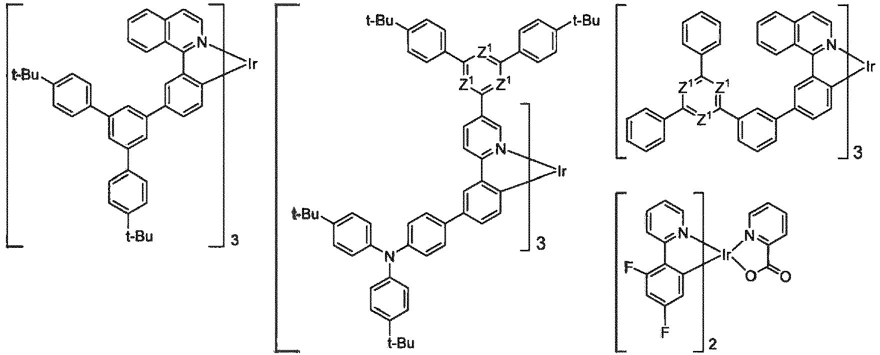

In the first layer, the compound (B-1) is preferably a host material or a guest material.

In the light emitting device of the present embodiment, a host material means a material that physically, chemically or electrically interacts with a guest material. This interaction makes it possible, for example, to improve or adjust a light emitting property, a charge transporting property or a charge injection property of the light emitting device of the present embodiment. In the light emitting device of the present embodiment, taking the light emitting material as an example, the host material and the guest material electrically interact with each other, and electric energy is efficiently transferred from the host material to the guest material, so that the guest material can emit light more efficiently, and the light emitting device of the present embodiment is more excellent in maximum light emission efficiency.

In the light emitting device of the present embodiment, it is preferable that the host material has at least one function selected from hole injectability, hole transportability, electron injectability and electron transportability.

In the light emitting device of the present embodiment, when the first layer is a layer containing a host material and a guest material, the content of a host material is usually 1 to 99.9 parts by mass, and it is preferably 10 to 99 parts by mass, more preferably 30 to 97 parts by mass, further preferably 50 to 95 parts by mass, and especially preferably 70 to 90 parts by mass, when the total content of a host material and a guest material is taken as 100 parts by mass.

In the light emitting device of the present embodiment, when the first layer is a layer containing a host material and a guest material, the total content of a host material and a guest material in the first layer may be within a range in which a function as the first layer is performed. The total content of a host material and a guest material in the first layer may be, for example, 1 to 100% by mass, and it is preferably 10 to 100% by mass, more preferably 30 to 100% by mass, further preferably 50 to 100% by mass, particularly preferably 70 to 100% by mass, and especially preferably 90 to 100% by mass, based on the total amount of the first layer.

In the light emitting device of the present embodiment, it is preferable that the lowest excited triplet state (T₁) of a host material is at higher energy level than the lowest excited triplet state (T₁) of a guest material, since the light emitting device of the present embodiment is more excellent in maximum light emission efficiency.

In the light emitting device of the present embodiment, it is preferable that a host material exhibits solubility in a solvent capable of dissolving a guest material, since the light emitting device of the present embodiment can be fabricated by a wet method.

In the light emitting device of the present embodiment, the number of types of the compound (B-1) contained in the first layer is usually 1 to 20, and it is preferably 1 to 10, more preferably 1 to 5, further preferably 1 to 3, and particularly preferably 1 or 2, since production of the light emitting device of the present embodiment is easy.

In the light emitting device of the present embodiment, it is preferable that the first layer is a layer containing two or more compounds (B-1), since the light emitting device of the present embodiment is more excellent in maximum light emission efficiency.

When the first layer is a layer containing two or more compounds (B-1), the content of at least one of two or more compounds (B-1) is usually 1 to 99 parts by mass, preferably 5 to 95 parts by mass, more preferably 10 to 90 parts by mass, further preferably 20 to 80 parts by mass, and especially preferably 30 to 70 parts by mass, when the total content of two or more compounds (B-1) is taken as 100 parts by mass.

When the first layer is a layer containing two or more compounds (B-1), it is preferable that at least one compound (B-1) contained in the first layer is a host material or a guest material, since the light emitting device of the present embodiment is more excellent in maximum light emission efficiency.

When the first layer is a layer containing two or more compounds (B-1), it is preferable that at least one of two or more compounds (B-1) is a host material and at least one of two or more compounds (B-1) is a guest material, since the light emitting device of the present embodiment is more excellent in maximum light emission efficiency.

In the first layer, when the compound (B-1) is a host material, Ar^{B 1} in the compound (B-1) is preferably a group obtained by removing from an aromatic hydrocarbon n^{B 1} hydrogen atoms bonding directly to carbon atoms constituting the ring or a group obtained by removing from a heterocyclic compound n^{B 1} hydrogen atoms bonding directly to atoms constituting the ring, and more preferably a group obtained by removing from a heterocyclic compound n^{B 1} hydrogen atoms bonding directly to atoms constituting the ring, since the light emitting device of the present embodiment is more excellent in maximum light emission efficiency, and these groups optionally have a substituent.

In the first layer, when the compound (B-1) is a guest material, Ar^{B 1} in the compound (B-1) is preferably a group obtained by removing n^{B 1} hydrogen atoms from a metal complex represented by the formula (1), since the light emitting device of the present embodiment is more excellent in maximum light emission efficiency, and the group optionally has a substituent.

In the light emitting device of the present embodiment, the first layer is preferably a layer containing a polymer compound containing at least one constitutional unit selected from the group consisting of a constitutional unit represented by the formula (X) described later and a constitutional unit represented by the formula (Y) described later (hereinafter, referred to also as "the polymer compound of the first layer") and, a compound (B-1), since the light emitting device of the present embodiment is more excellent in maximum light emission efficiency.

In the light emitting device of the present embodiment, when the first layer is a layer containing the polymer compound of the first layer and a compound (B-1), the polymer compound of the first layer may be contained only singly or in combination of two or more in the first layer.

In the light emitting device of the present embodiment, when the first layer is a layer containing the polymer compound of the first layer and a compound (B-1), the content of the polymer compound of the first layer is usually 1 to 99.9 parts by mass, preferably 10 to 99 parts by mass, more preferably 30 to 97 parts by mass, further preferably 50 to 95 parts by mass, and especially preferably 70 to 90 parts by mass, when the total content of the polymer compound of the first layer and a compound (B-1) is taken as 100 parts by mass.

In the light emitting device of the present embodiment, when the first layer is a layer containing the polymer compound of the first layer and a compound (B-1), the total content of the polymer compound of the first layer and a compound (B-1) in the first layer may be within a range in which a function as the first layer is performed. The total content of the polymer compound of the first layer and a compound (B-1) in the first layer may be, for example, 1 to 100% by mass, and it is preferably 10 to 100% by mass, more preferably 30 to 100% by mass, further preferably 50 to 100% by mass, and particularly preferably 70 to 100% by mass, based on the total amount of the first layer.

In the light emitting device of the present embodiment, when the first layer is a layer containing the polymer compound of the first layer and a compound (B-1), it is preferable that the polymer compound of the first layer is a host material or a guest material and the compound (B-1) is a host material or a guest material, it is more preferable that the polymer compound of the first layer is a host material and the compound (B-1) is a guest material, or the polymer compound of the first layer is a guest material and the compound (B-1) is a host material, and it is further preferable that the polymer compound of the first layer is a host material and the compound (B-1) is a guest material, since the light emitting device of the present embodiment is more excellent in maximum light emission efficiency.

The polymer compound of the first layer preferably a polymer compound containing a constitutional unit represented by the formula (Y) described later, since the light emitting device of the present embodiment is more excellent in maximum light emission efficiency.

The examples and preferable range of the constitutional unit represented by the formula (Y) in the polymer compound of the first layer are the same as the examples and preferable range of the constitutional unit represented by the formula (Y) in the polymer compound having a cross-linkable group described later.

When the polymer compound of the first layer contains a constitutional unit represented by the formula (Y), the content of the constitutional unit represented by the formula (Y) contained in the polymer compound of the first layer is preferably 1 to 100% by mol, more preferably 30 to 100% by mol, further preferably 50 to 100% by mol, and particularly preferably 70 to 100% by mol, with respect to the total content of constitutional units contained in the polymer compound of the first layer, since the light emitting device of the present embodiment is more excellent in maximum light emission efficiency.

In the polymer compound of the first layer, the constitutional unit represented by the formula (Y) may be contained only singly or in combination of two or more in the polymer compound of the first layer.

The polymer compound of the first layer preferably contains a constitutional unit represented by the formula (X) described later, since hole transportability is excellent.

The examples and preferable range of the constitutional unit represented by the formula (X) in the polymer compound of the first layer are the same as the examples and preferable range of the constitutional unit represented by the formula (X) in the polymer compound having a cross-linkable group described later.

When the polymer compound of the first layer contains a constitutional unit represented by the formula (X), the content of the constitutional unit represented by the formula (X) contained in the polymer compound of the first layer is preferably 0.1 to 100% by mol, more preferably 0.5 to 70% by mol, further preferably 1 to 50% by mol, and particularly preferably 5 to 30% by mol, with respect to the total content of constitutional units contained in the polymer compound of the first layer, since the light emitting device of the present embodiment is more excellent in maximum light emission efficiency.

In the polymer compound of the first layer, the constitutional unit represented by the formula (X) may be contained only singly or in combination of two or more in the polymer compound of the first layer.

The polymer compound of the first layer preferably contains a constitutional unit represented by the formula (Y) and a constitutional unit represented by the formula (X), since hole transportability is excellent and since the light emitting device of the present embodiment is more excellent in maximum light emission efficiency.

When the polymer compound of the first layer contains a constitutional unit represented by the formula (Y) and a constitutional unit represented by the formula (X), the total content of a constitutional unit represented by the formula (Y) and a constitutional unit represented by the formula (X) contained in the polymer compound of the first layer is preferably 1 to 100% by mol, more preferably 30 to 100% by mol, further preferably 50 to 100% by mol, and particularly preferably 70 to 100% by mol, with respect to the total content of constitutional units contained in the polymer compound of the first layer, since the light emitting device of the present embodiment is more excellent in maximum light emission efficiency.

The polymer compound of the first layer includes, for example, polymer compounds HP-1 to HP-3.

**[Table 1]**

| Polymer compound | Constitutional unit and molar ratio thereof | | |
|---|---|---|---|
| | Formula (Y) | Formula (X) | other |
| | p | q | r |
| HP-1 | 100 | 0 | 0 to 30 |
| HP-2 | 0 | 100 | 0 to 30 |
| HP-3 | 0.1 to 99.9 | 0.1 to 99.9 | 0 to 30 |

[in the table, p, q and r represent the molar ratio of each constitutional unit. p+q+r = 100, and 100 ≥ p+q ≥ 70. Other constitutional units denote constitutional units other than the constitutional unit represented by the formula (Y) and the constitutional unit represented by the formula (X).]

The polymer compound of the first layer may be any of a block copolymer, a random copolymer, an alternative copolymer or a graft copolymer, and may also be in another form, and is preferably a copolymer in which plural kinds of raw material monomers are copolymerized.

The examples and preferable range of the polystyrene-equivalent number-average molecular weight of the polymer compound of the first layer are the same as the examples and preferable range of the polystyrene-equivalent number-average molecular weight of the polymer compound having a cross-linkable group described later. The examples and preferable range of the polystyrene-equivalent weight-average molecular weight of the polymer compound of the first layer are the same as the examples and preferable range of the polystyrene-equivalent weight-average molecular weight of the polymer compound having a cross-linkable group described later.

### [Production method of polymer compound of first organic layer]

The polymer compound of the first organic layer can be produced by the same method as the production method for the polymer compound having a cross-linkable group described later.

### [First composition]

The first layer may be a layer containing a composition containing a compound (B-1), and at least one selected from the group consisting of the polymer compound of the first layer described above, a hole transporting material, a hole injection material, an electron transporting material, an electron injection material, a light emitting material and an antioxidant (hereinafter, referred to also as "the first composition"). Note that in the first composition, the hole transporting material, the hole injection material, the electron transporting material, the electron injection material and the light emitting material are different from the compound (B-1).

In the first composition, the compound (B-1), the hole transporting material, the hole injection material, the electron transporting material, the electron injection material, the light emitting material and the antioxidant each may be contained singly or in combination of two or more.

In the first composition, the total content of the compound (B-1), the polymer compound of the first layer described above, the hole transporting material, the hole injection material, the electron transporting material, the electron injection material, the light emitting material and the antioxidant may be within a range in which the function as the first composition is exhibited. In the first composition, the total content of the compound (B-1), the polymer compound of the first layer described above, the hole transporting material, the hole injection material, the electron transporting material, the electron injection material, the light emitting material and the antioxidant may be, for example, 1 to 100% by mass, may be 10 to 100% by mass, may be 30 to 100% by mass, may be further preferably 50 to 100% by mass, may be 70 to 100% by mass, or may be 90 to 100% by mass, based on the total amount of the first composition.

### (Hole transporting material)

The hole transporting material is classified into a low molecular compound and a polymer compound. The hole transporting material may have a cross-linkable group.

The low molecular compound includes, for example, aromatic amine compounds such as triphenylamine and derivatives thereof, N,N'-di-1-naphthyl-N,N'-diphenylbenzidine (α-NPD), N,N'-diphenyl-N,N'-di(m-tolyl)benzidine (TPD), and the like.

The polymer compound includes, for example, polyvinylcarbazole and derivatives thereof; polyarylenes having an aromatic amine structure in the side chain or main chain, and derivatives thereof. The polymer compound may also be a compound to which an electron-accepting site is bonded such as fullerene, tetrafluorotetracyanoquinodimethane, tetracyanoethylene, trinitrofluorenone, and the like.

In the first composition, when a hole transporting material is contained, the content of the hole transporting material is usually 1 to 1000 parts by mass when the content of the compound (B-1) is taken as 100 parts by mass. The hole transporting material may be used singly or in combination of two or more.

### (Electron transporting material)

The electron transporting material is classified into a low molecular compound and a polymer compound. The electron transporting material may have a cross-linkable group.

The low molecular compound includes, for example, metal complexes having 8-hydroxyquinoline as a ligand; oxadiazole, anthraquinodimethane, benzoquinone, naphthoquinone, anthraquinone, tetracyanoanthraquinodimethane, fluorenone, diphenyldicyanoethylene and diphenoquinone, and derivatives thereof.

The polymer compound includes, for example, polyphenylene, polyfluorene, and derivatives thereof. The polymer compound may be doped with a metal.

In the first composition, when an electron transporting material is contained, the content of the electron transporting material is usually 1 to 1000 parts by mass when the content of the compound (B-1) is taken as 100 parts by mass. The electron transporting material may be used singly or in combination of two or more.

### (Hole injection material and electron injection material)

The hole injection material and the electron injection material are each classified into a low molecular compound and a polymer compound. The hole injection material and the electron injection material may have a cross-linkable group.

The low molecular compound includes, for example, metal phthalocyanines such as copper phthalocyanine, and the like; carbon; oxides of metals such as molybdenum, tungsten, and the like; and metal fluorides such as lithium fluoride, sodium fluoride, cesium fluoride, potassium fluoride, and the like.

The polymer compound includes, for example, polyaniline, polythiophene, polypyrrole, polyphenylenevinylene, polythienylenevinylene, polyquinoline and polyquinoxaline, and derivatives thereof; electrically conductive polymers such as polymers containing an aromatic amine structure in the main chain or side chain, and the like.

In the first composition, when a hole injection material and/or an electron injection material is contained, the contents of the hole injection material and the electron injection material are each usually 1 to 1000 parts by mass when the content of the compound (B-1) is taken as 100 parts by mass. The hole injection material and the electron injection material each may be used singly or in combination of two or more.

### •Ion doping

A hole injection material or an electron injection material may be doped with ions. For example, when the hole injection material or the electron injection material contains an electrically conductive polymer, the electric conductivity of the electrically conductive polymer is preferably 1×10⁻⁵ S/cm to 1×10³ S/cm. For adjusting the electric conductivity of the electrically conductive polymer within such a range, the electrically conductive polymer can be doped with an appropriate amount of ions.

The type of ions to be doped into a hole injection material or an electron injection material includes, for example, anions for the hole injection material, and cations for the electron injection material. The anion includes, for example, a polystyrenesulfonate ion, an alkylbenzenesulfonate ion, and a camphor sulfonate ion. The cation includes, for example, a lithium ion, a sodium ion, a potassium ion, and a tetrabutylammonium ion.

The ion to be doped may be used singly or in combination of two or more.

### (Light emitting material)

The light emitting material is classified into a low molecular compound and a polymer compound. The light emitting material may have a cross-linkable group.

The low molecular compound includes, for example, naphthalene and derivatives thereof, anthracene and derivatives thereof, perylene and derivatives thereof, and phosphorescent compounds containing iridium, platinum or europium as the central metal.

The phosphorescent compound as the light emitting material includes, for example, compounds represented by the following formulae.

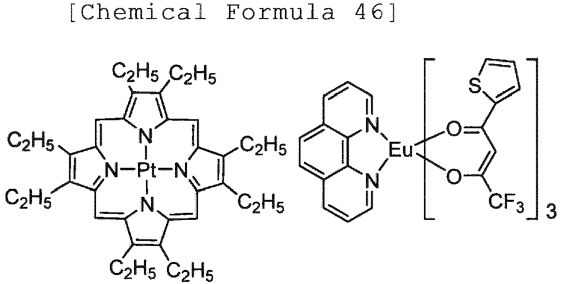

The polymer compound includes, for example, polymer compounds containing a constitutional unit represented by the formula (Y) and/or a constitutional unit represented by the formula (X) described later.

In the first composition, when a light emitting material is contained, the content of the light emitting material is usually 1 to 1000 parts by mass when the content of the compound (B-1) is taken as 100 parts by mass. The light emitting material may be used singly or in combination of two or more.

### (Antioxidant)

The antioxidant may be a compound which is soluble in a solvent which is the same as the solvent for the compound (B-1) and does not inhibit light emission and charge transportation, and includes, for example, phenol type antioxidants and phosphorus-based antioxidants.

In the first composition, when an antioxidant is contained, the content of the antioxidant is usually 0.001 to 10 parts by mass when the content of the compound (B-1) is taken as 100 parts by mass. The antioxidant may be used singly or in combination of two or more.

### [First ink]

The first layer can be formed, for example, by using a composition containing the compound (B-1) and a solvent (hereinafter, referred to also as "first ink").

The first ink can be suitably used for fabrication of a light emitting device using a wet method such as, for example, a spin coat method, a casting method, a micro gravure coat method, a gravure coat method, a bar coat method, a roll coat method, a wire bar coat method, a dip coat method, a spray coat method, a screen printing method, a flexo printing method, an offset printing method, an inkjet printing method, a capillary coat method, a nozzle coat method, and the like.

The viscosity of the first ink may advantageously be adjusted depending on the type of the wet method. The viscosity of the first ink is preferably 1 to 20 mPa●s at 25°C, when applied to printing methods in which a solution passes through a discharge device such as an inkjet printing method, and the like, since clogging and flight curving in discharging less likely to occur.

The solvent contained in the first ink is preferably a solvent which can dissolve or uniformly disperse solid components in the ink. The solvent contained in the first ink includes, for example, chlorine-based solvents such as 1,2-dichloroethane, 1,1,2-trichloroethane, chlorobenzene, o-dichlorobenzene, and the like; ether solvents such as tetrahydrofuran, dioxane, anisole, 4-methylanisole, and the like; aromatic hydrocarbon solvents such as toluene, xylene, mesitylene, ethylbenzene, n-hexylbenzene, cyclohexylbenzene, and the like; aliphatic hydrocarbon solvents such as cyclohexane, methylcyclohexane, n-pentane, n-hexane, n-heptane, n-octane, n-nonane, n-decane, n-dodecane, bicyclohexyl, and the like; ketone solvents such as acetone, methyl ethyl ketone, cyclohexanone, acetophenone, and the like; ester solvents such as ethyl acetate, butyl acetate, ethyl cellosolve acetate, methyl benzoate, phenyl acetate, and the like; alcohol solvents such as ethylene glycol, glycerin, 1,2-hexane diol, isopropyl alcohol, cyclohexanol, and the like; sulfoxide solvents such as dimethyl sulfoxide, and the like; amide solvents such as N-methyl-2-pyrrolidone, N,N-dimethylformamide, and the like; and water.

In the first ink, the content of the solvent is usually 1000 to 100000 parts by mass when the content of the compound (B-1) is taken as 100 parts by mass. The solvent may be used singly or in combination of two or more.

The first ink may further contain at least one selected from the group consisting of the polymer compound of the first layer described above, a hole transporting material, a hole injection material, an electron transporting material, an electron injection material, a light emitting material and an antioxidant.

The examples and preferable ranges of the hole transporting material, the electron transporting material, the hole injection material, the electron injection material, the light emitting material and the antioxidant which the first ink may further contain are the same as the examples and preferable ranges of the hole transporting material, the electron transporting material, the hole injection material, the electron injection material, the light emitting material and the antioxidant contained in the first composition.

The contents of the hole transporting material, the electron transporting material, the hole injection material, the electron injection material and the light emitting material which the first ink may further contain are each usually 1 to 1000 parts by mass when the content of the compound (B-1) is taken as 100 parts by mass. The content of the antioxidant which the fist ink may further contain is usually 0.001 to 10 parts by mass when the content of the compound (B-1) is taken as 100 parts by mass.

### <Second layer>

In the light emitting device of the present embodiment, the second layer is a layer containing a compound (B-2) and a crosslinked body of a compound having a cross-linkable group. Namely, in the second layer, the compound (B-2) is contained as a separate compound from the crosslinked body of a compound having a cross-linkable group. Here, it is preferable that the compound (B-2) is a compound containing no cross-linkable group.

The examples and preferable range of the compound (B-2) in the second layer are the same as the examples and preferable range of the compound (B-1) in the first layer.

In the second layer, the compound (B-2) may be contained only singly or in combination of two or more. In the second layer, the crosslinked body of a compound having a cross-linkable group may be contained only singly or in combination of two or more.

In the light emitting device of the present embodiment, the number of types of the compound (B-2) contained in the second layer is usually 1 to 20, and it is preferably 1 to 10, more preferably 1 to 5, further preferably 1 to 3, particularly preferably 1 or 2, and especially preferably 1, since production of the light emitting device of the present embodiment is easy.

In the light emitting device of the present embodiment, the number of types of the crosslinked body of a compound having a cross-linkable group contained in the second layer is usually 1 to 20, and it is preferably 1 to 10, more preferably 1 to 5, further preferably 1 to 3, particularly preferably 1 or 2, and especially preferably 1, since production of the light emitting device of the present embodiment is easy.

The total content of the compound (B-2) and the crosslinked body of a compound having a cross-linkable group in the second layer may be within a range in which the function as the second layer is exhibited. The total content of the compound (B-2) and the crosslinked body of a compound having a cross-linkable group in the second layer may be, for example, 1 to 100° by mass based on the total amount of the second layer, and it is preferably 10 to 100% by mass, more preferably 30 to 100% by mass, further preferably 50 to 100% by mass, particularly preferably 70 to 100% by mass, and especially preferably 90 to 100% by mass, since the light emitting device of the present embodiment is more excellent in maximum light emission efficiency.

In the second layer, the content of the compound (B-2) is usually 0.01 to 99 parts by mass, preferably 0.1 to 90 parts by mass, more preferably 0.5 to 70 parts by mass, further preferably 1 to 50 parts by mass, and particularly preferably 5 to 30 parts by mass, when the total content of the compound (B-2) and the crosslinked body of a compound having a cross-linkable group is taken as 100 parts by mass.

### [Cross-linked body of compound having cross-linkable group]

The crosslinked body of a compound having a cross-linkable group is obtained by cross-linking a compound having a cross-linkable group by methods and conditions described later, and the like.

The compound having a cross-linkable group may be a low molecular compound having a cross-linkable group or may be a polymer compound having a cross-linkable group, and is preferably a polymer compound having a cross-linkable group.

In the compound having a cross-linkable group, the cross-linkable group is preferably at least one cross-linkable group selected from Group A of cross-linkable group, more preferably a cross-linkable group represented by the formula (XL-1), the formula (XL-3), the formula (XL-9), the formula (XL-10) or the formula (XL-16) to the formula (XL-19), further preferably a cross-linkable group represented by the formula (XL-1) or the formula (XL-16) to the formula (XL-19), and particularly preferably a cross-linkable group represented by the formula (XL-1) or the formula (XL-17), since the light emitting device of the present embodiment is more excellent in maximum light emission efficiency.

The examples and preferable range of the substituent which a cross-linkable group optionally has in at least one cross-linkable group selected from Group A of cross-linkable group are the same as the examples and preferable range of the substituent which a group represented by Ar^{Y1} optionally has described later.

In the compound having a cross-linkable group, the cross-linkable group may be contained only singly or in combination of two or more.

### (Polymer compound having cross-linkable group)

The polymer compound having a cross-linkable group preferably contain a cross-linkable group in the form of a constitutional unit having a cross-linkable group, since the polymer compound having a cross-linkable group is more excellent in crosslinkability and the light emitting device of the present embodiment is more excellent in maximum light emission efficiency. That is, the polymer compound having a cross-linkable group is preferably a polymer compound containing a constitutional unit having a cross-linkable group.

The examples and preferable range of the cross-linkable group in the polymer compound having a cross-linkable group and the constitutional unit having a cross-linkable group are the same as the examples and preferable range of the cross-linkable group in a compound having the cross-linkable group.

When the polymer compound having a cross-linkable group contains a constitutional unit having a cross-linkable group, the content of the constitutional unit having a cross-linkable group is usually 0.1 to 100% by mol, with respect to the total content of constitutional units contained in the polymer compound having a cross-linkable group, and it is preferably 1 to 90% by mol, more preferably 2 to 70% by mol, further preferably 3 to 50% by mol, and particularly preferably 5 to 20% by mol, since the polymer compound having a cross-linkable group is excellent in stability and crosslinkability.

The constitutional unit having a cross-linkable group may be contained singly or in combination of two or more in the polymer compound having a cross-linkable group.

The constitutional unit having a cross-linkable group is preferably a constitutional unit represented by the formula (Z) or a constitutional unit represented by the formula (Z'), since the light emitting device of the present embodiment is more excellent in maximum light emission efficiency.

### • Constitutional unit represented by the formula (Z)

n is usually an integer of 1 to 10, and it is preferably an integer of 1 to 7, more preferably an integer of 1 to 4, further preferably 1 or 2, and particularly preferably 2, since the light emitting device of the present embodiment is more excellent in maximum light emission efficiency.

nA is usually an integer of 0 to 10, and it is preferably an integer of 0 to 7, more preferably an integer of 0 to 4, and further preferably an integer of 0 to 2, since the light emitting device of the present embodiment is more excellent in maximum light emission efficiency.

The hydrocarbon group represented by Ar³ includes aromatic hydrocarbon groups optionally having a substituent and aliphatic hydrocarbon groups optionally having a substituent. The hydrocarbon group represented by Ar³ includes groups obtained by bonding a plurality of these groups.

The aliphatic hydrocarbon group represented by Ar³ includes groups obtained by removing from an alkylene group or a cycloalkylene group n hydrogen atoms, preferably groups obtained by removing from an alkylene group n hydrogen atoms, and these groups optionally have a substituent.

The aromatic hydrocarbon group represented by Ar³ includes groups obtained by removing from an arylene group n hydrogen atoms, and this group optionally has a substituent. The examples and preferable range of this arylene group include the examples and preferable range of the arylene group represented by Ar^{Y1} described later.

The hetero ring group represented by Ar³ includes groups obtained by removing from a divalent hetero ring group n hydrogen atoms, and this group optionally has a substituent. The examples and preferable range of this divalent hetero ring group include the examples and preferable range of the divalent hetero ring group represented by Ar^{Y1} described later.

The examples and preferable ranges of a hydrocarbon group and a hetero ring group in the group in which at least one hydrocarbon group and at least one hetero ring group are bonded directly represented by Ar³ are the same as the examples and preferable ranges of the hydrocarbon group and the hetero ring group represented by Ar³, respectively.

The group in which at least one hydrocarbon group and at least one hetero ring group are bonded directly represented by Ar³ includes, for example, groups obtained by removing from a group in which at least one arylene group and at least one divalent hetero ring group are bonded directly represented by Ar^{Y1} described later n hydrogen atoms.

Ar³ is preferably a hydrocarbon group or a hetero ring group, more preferably a hydrocarbon group, and further preferably an aromatic hydrocarbon group, since the light emitting device of the present embodiment is more excellent in maximum light emission efficiency, and these groups optionally have a substituent.

The examples and preferable range of the arylene group represented by L^{A} include the examples and preferable range of the arylene group represented by Ar^{Y1} described later, and the arylene group represented by L^{A} is preferably a phenylene group or a fluorenediyl group, since the light emitting device of the present embodiment is more excellent in maximum light emission efficiency, and these groups optionally have a substituent.

The examples and preferable range of the divalent hetero ring group represented by L^{A} are the same as the examples and preferable range of the divalent hetero ring group represented by Ar^{Y1} described later.

L^{A} is preferably an arylene group or an alkylene group, and more preferably a phenylene group, a fluorenediyl group or an alkylene group, since synthesis of the polymer compound having a cross-linkable group is easy, and these groups optionally have a substituent.

R' represents preferably an aryl group or a monovalent hetero ring group, and more preferably an aryl group, and these groups optionally have a substituent.

The examples and preferable ranges of the aryl group and the monovalent hetero ring group represented by R' are the same as the examples and preferable ranges of the aryl group and the monovalent hetero ring group as the substituent which Ar^{Y1} optionally has described later, respectively.

The examples and preferable range of the substituent which a group represented by Ar³, L^{A} and R' optionally has are the same as the examples and preferable range of the substituent which a group represented by Ar^{Y1} optionally has described later.

The examples and preferable range of the cross-linkable group represented by X are the same as the examples and preferable range of the cross-linkable group in a compound having the cross-linkable group.

### • Constitutional unit represented by the formula (Z')

mA is usually an integer of 0 to 10, and it is preferably an integer of 0 to 7, more preferably an integer of 0 to 4, further preferably an integer of 0 to 2, particularly preferably 0 or 1, and especially preferably 0, since the light emitting device of the present embodiment is more excellent in maximum light emission efficiency.

m is usually an integer of 0 to 10, and it is preferably an integer of 0 to 7, more preferably an integer of 0 to 4, further preferably an integer of 0 to 2, and particularly preferably 0, since the light emitting device of the present embodiment is more excellent in maximum light emission efficiency.

c is usually an integer of 0 to 10, and it is preferably an integer of 0 to 5, more preferably an integer of 0 to 2, further preferably 0 or 1, and particularly preferably 0, since production of the polymer compound having a cross-linkable group is easy and the light emitting device of the present embodiment is more excellent in maximum light emission efficiency.

The examples and preferable ranges of the hydrocarbon group and the hetero ring group represented by Ar⁵ are the same as the examples and preferable ranges of the hydrocarbon group and the hetero ring group represented by Ar³, respectively.

The examples and preferable range of the group in which at least one hydrocarbon group and at least one hetero ring group are bonded directly represented by Ar⁵ are the same as the examples and preferable range of the group in which at least one hydrocarbon group and at least one hetero ring group are bonded directly represented by Ar³.
Ar⁵ is preferably a hydrocarbon group or a hetero ring group, more preferably a hydrocarbon group, and further preferably an aromatic hydrocarbon group, since the light emitting device of the present embodiment is more excellent in maximum light emission efficiency, and these groups optionally have a substituent.

Ar⁴ and Ar⁶ represent preferably an arylene group optionally having a substituent, since the light emitting device of the present embodiment is more excellent in maximum light emission efficiency.

The examples and preferable range of the arylene group represented by Ar⁴ and Ar⁶ are the same as the examples and preferable range of the arylene group represented by Ar^{X1}, Ar^{X2}, Ar^{X3} and Ar^{X4} described later.

The examples and preferable range of the divalent hetero ring group represented by Ar⁴ and Ar⁶ are the same as the examples and preferable range of the divalent hetero ring group represented by Ar^{X1}, Ar^{X2}, Ar^{X3} and Ar^{X4} described later.

The examples and preferable range of the substituent which a group represented by Ar⁴ to Ar⁶ optionally has are the same as the examples and preferable range of the substituent which a group represented by Ar^{Y1} optionally has described later.

The examples and preferable range of K^{A} are the same as the examples and preferable range of L^{A}.

The examples and preferable range of R" are the same as the examples and preferable range of R'.

The examples and preferable range of the cross-linkable group represented by X' are the same as the examples and preferable range of the cross-linkable group represented by X.

The examples and preferable ranges of the aryl group and the monovalent hetero ring group represented by X' are the same as the examples and preferable ranges of the aryl group and the monovalent hetero ring group represented by R^{X1}, R^{X2} and R^{X3} described later, respectively.

X' is preferably a cross-linkable group, an alkyl group, a cycloalkyl group, an aryl group or a monovalent hetero ring group, more preferably a cross-linkable group, an aryl group or a monovalent hetero ring group, further preferably a cross-linkable group or an aryl group, and these groups optionally have a substituent.

The examples and preferable range of the substituent which a group represented by X' optionally has are the same as the examples and preferable range of the substituent which a group represented by Ar^{Y1} optionally has described later.

The constitutional unit having a cross-linkable group includes, for example, constitutional units represented by the following formulae. In the formulae, Z² represents the same meaning as described above. X^{A} represents at least one cross-linkable group selected from Group A of cross-linkable group. When a plurality of X^{A} are present, they may be the same or different. The preferable range of X^{A} is the same as the preferable range of the cross-linkable group in the compound having a cross-linkable group.

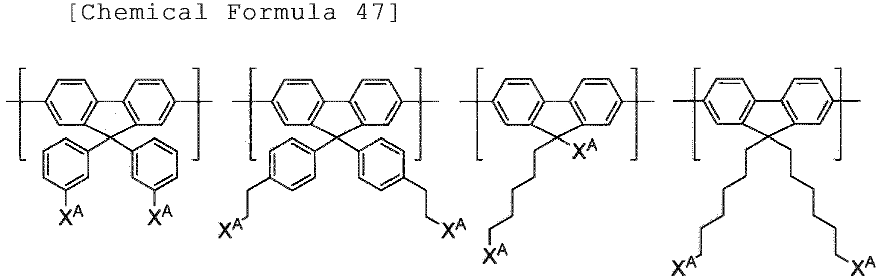

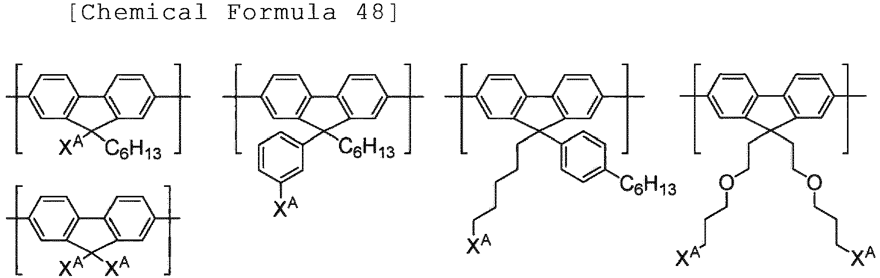

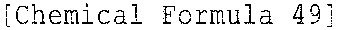

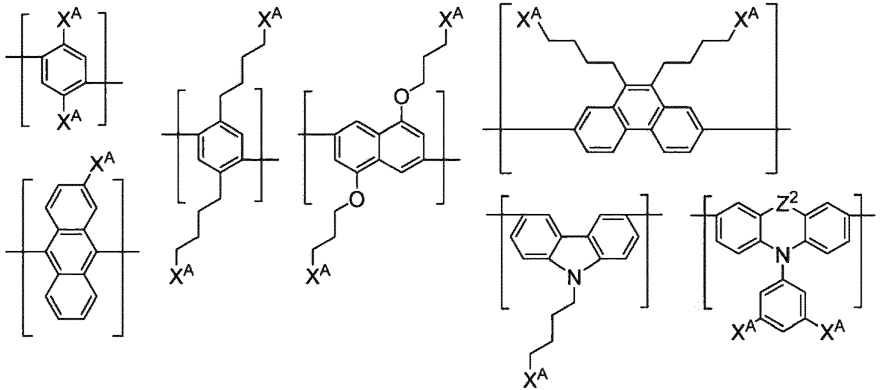

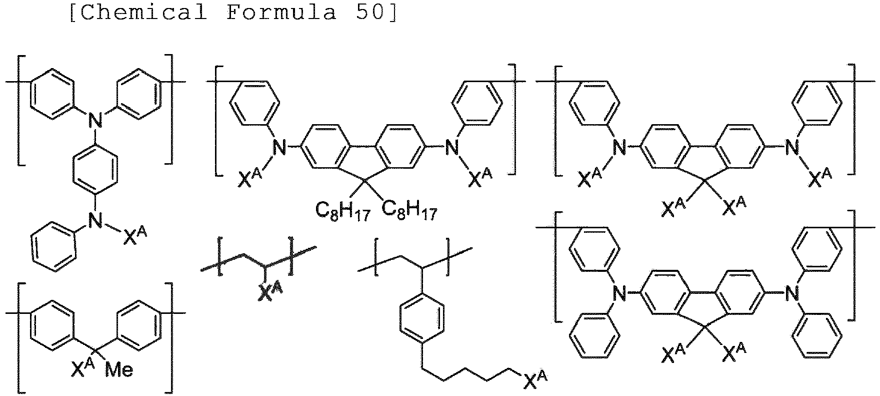

### • Other constitutional units

The polymer compound having a cross-linkable group preferably contains at least one constitutional unit selected from the group consisting of a constitutional unit represented by the formula (X) and a constitutional unit represented by the formula (Y), and preferably contains at least one constitutional unit selected from the group consisting of a constitutional unit represented by the formula (X) and a constitutional unit represented by the formula (Y), and a constitutional unit having a cross-linkable group, since the light emitting device of the present embodiment is more excellent in maximum light emission efficiency.

When the polymer compound having a cross-linkable group contains at least one constitutional unit selected from the group consisting of a constitutional unit represented by the formula (X) and a constitutional unit represented by the formula (Y), and a constitutional unit having a cross-linkable group, it is preferable that the constitutional unit having a cross-linkable group is different from the constitutional unit represented by the formula (X) and the constitutional unit represented by the formula (Y).

The polymer compound having a cross-linkable group preferably contains a constitutional unit represented by the formula (Y), and more preferably contains a constitutional unit represented by the formula (Y) and a constitutional unit having a cross-linkable group, since the light emitting device of the present embodiment is more excellent in maximum light emission efficiency.

The polymer compound having a cross-linkable group preferably contains a constitutional unit represented by the formula (X), and more preferably contains a constitutional unit represented by the formula (X) and a constitutional unit having a cross-linkable group, since hole transportability is excellent.

The polymer compound having a cross-linkable group preferably contains a constitutional unit represented by the formula (X) and a constitutional unit represented by the formula (Y), and more preferably contains a constitutional unit represented by the formula (X), a constitutional unit represented by the formula (Y) and a constitutional unit having a cross-linkable group, since hole transportability is excellent and the light emitting device of the present embodiment is more excellent in maximum light emission efficiency.

When the polymer compound having a cross-linkable group contains a constitutional unit represented by the formula (X), the content of the constitutional unit represented by the formula (X) is usually 0.1 to 99% by mol, with respect to the total content of constitutional units contained in the polymer compound having a cross-linkable group, and it is preferably 1 to 90° by mol, more preferably 10 to 80% by mol, further preferably 20 to 70% by mol, and particularly preferably 30 to 50° by mol, since the polymer compound having a cross-linkable group is excellent in hole transportability and the light emitting device of the present embodiment is more excellent in maximum light emission efficiency.

The constitutional unit represented by the formula (X) may be contained only singly or in combination of two or more in the polymer compound having a cross-linkable group.

When the polymer compound having a cross-linkable group contains a constitutional unit represented by the formula (Y), the content of the constitutional unit represented by the formula (Y) is usually 0.1 to 99% by mol, with respect to the total content of constitutional units contained in the polymer compound having a cross-linkable group, and it is preferably 1 to 95% by mol, more preferably 10 to 90% by mol, further preferably 20 to 80% by mol, and particularly preferably 30 to 70% by mol, since the light emitting device of the present embodiment is more excellent in maximum light emission efficiency.

When the polymer compound having a cross-linkable group contains a constitutional unit represented by the formula (Y) and Ar^{Y1} is a divalent hetero ring group or a divalent group in which at least one arylene group and at least one divalent hetero ring group are bonded directly, the content of the constitutional unit represented by the formula (Y) is usually 0.1 to 99% by mol, with respect to the total content of constitutional units contained in the polymer compound having a cross-linkable group, and it is preferably 1 to 90% by mol, more preferably 5 to 80% by mol, further preferably 10 to 70% by mol, and particularly preferably 20 to 50% by mol, since the polymer compound having a cross-linkable group is excellent in charge transportability.

The constitutional unit represented by the formula (Y) may be contained singly or in combination of two or more in the polymer compound having a cross-linkable group.

When the polymer compound having a cross-linkable group contains a constitutional unit represented by the formula (X), a constitutional unit represented by the formula (Y) and a constitutional unit having a cross-linkable group, the total content of the constitutional unit represented by the formula (X), the constitutional unit represented by the formula (Y) and the constitutional unit having a cross-linkable group is usually 1 to 100% by mol, with respect to the total content of constitutional units contained in the polymer compound having a cross-linkable group, and it is preferably 10 to 100% by mol, more preferably 30 to 100% by mol, further preferably 50 to 100% by mol, particularly preferably 70 to 100% by mol, and especially preferably 90 to 100% by mol, since the light emitting device of the present embodiment is more excellent in maximum light emission efficiency.

### • Constitutional unit represented by the formula (Y)

The arylene group represented by Ar^{Y1} is preferably a group obtained by removing from a monocyclic or bicyclic to hexacyclic aromatic hydrocarbon two hydrogen atoms bonding directly to atoms constituting the ring, more preferably a group obtained by removing from a monocyclic, bicyclic or tricyclic aromatic hydrocarbon two hydrogen atoms bonding directly to atoms constituting the ring, further preferably a group obtained by removing from benzene, naphthalene, anthracene, phenanthrene, dihydrophenanthrene or fluorene two hydrogen atoms bonding directly to atoms constituting the ring, and particularly preferably a group obtained by removing from benzene, phenanthrene, dihydrophenanthrene or fluorene two hydrogen atoms bonding directly to atoms constituting the ring, since the light emitting device of the present embodiment is more excellent in maximum light emission efficiency, and these optionally have a substituent.

The divalent hetero ring group represented by Ar^{Y1} is preferably a group obtained by removing from a monocyclic or bicyclic to hexacyclic heterocyclic compound two hydrogen atoms bonding directly to atoms constituting the ring, more preferably a group obtained by removing from a monocyclic, bicyclic or tricyclic heterocyclic compound two hydrogen atoms bonding directly to atoms constituting the ring, further preferably a group obtained by removing from pyridine, diazabenzene, triazine, azanaphthalene, diazanaphthalene, carbazole, dibenzofuran, dibenzothiophene, phenoxazine, phenothiazine, 9,10-dihydroacridine or 5,10-dihydrophenazine two hydrogen atoms bonding directly to atoms constituting the ring (preferably carbon atoms or nitrogen atoms, more preferably carbon atoms), and particularly preferably a group obtained by removing from pyridine, diazabenzene, triazine, carbazole, dibenzofuran, dibenzothiophene, phenoxazine or phenothiazine two hydrogen atoms bonding directly to atoms constituting the ring (preferably carbon atoms or nitrogen atoms, more preferably carbon atoms), since the light emitting device of the present embodiment is more excellent in maximum light emission efficiency, and these optionally have a substituent.

The preferable ranges of the arylene group and the divalent hetero ring group in the divalent group in which at least one arylene group and at least one divalent hetero ring group are bonded directly represented by Ar^{Y1} are the same as the preferable ranges of the arylene group and the divalent hetero ring group represented by Ar^{Y1}, respectively.

The "divalent group in which at least one arylene group and at least one divalent hetero ring group are bonded directly" represented by Ar^{Y1} includes, for example, groups represented by the following formulae, and these optionally have a substituent.

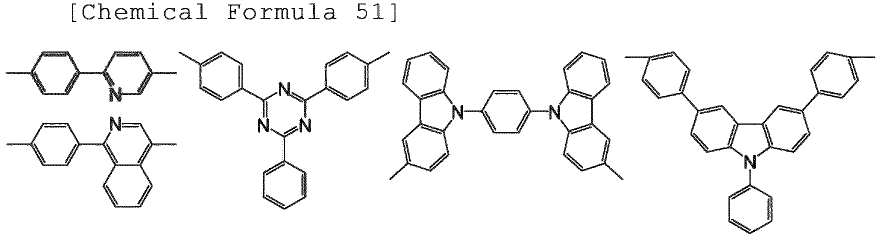

Ar^{Y1} is preferably an arylene group optionally having a substituent, since the light emitting device of the present embodiment is more excellent in maximum light emission efficiency.

The substituent which a group represented by Ar^{Y1} optionally has is preferably an alkyl group, a cycloalkyl group, an alkoxy group, a cycloalkoxy group, an aryl group, an aryloxy group, a monovalent hetero ring group, a substituted amino group or a fluorine atom, more preferably an alkyl group, a cycloalkyl group, an alkoxy group, a cycloalkoxy group, an aryl group, a monovalent hetero ring group or a substituted amino group, further preferably an alkyl group, a cycloalkyl group, an aryl group, a monovalent hetero ring group or a substituted amino group, and particularly preferably an alkyl group, a cycloalkyl group or an aryl group, and these groups optionally further have a substituent.

The aryl group as the substituent which a group represented by Ar^{Y1} optionally has is preferably a group obtained by removing from a monocyclic or bicyclic to hexacyclic aromatic hydrocarbon one hydrogen atom bonding directly to an atom constituting the ring, more preferably a group obtained by removing from a monocyclic, bicyclic or tricyclic aromatic hydrocarbon one hydrogen atom bonding directly to an atom constituting the ring, further preferably a group obtained by removing from benzene, naphthalene, anthracene, phenanthrene, dihydrophenanthrene or fluorene one hydrogen atom bonding directly to an atom constituting the ring, and particularly preferably a group obtained by removing from benzene, phenanthrene, dihydrophenanthrene or fluorene one hydrogen atom bonding directly to an atom constituting the ring, since the light emitting device of the present embodiment is more excellent in maximum light emission efficiency, and these optionally further have a substituent.

The monovalent hetero ring group as the substituent which a group represented by Ar^{Y1} optionally has is preferably a group obtained by removing from a monocyclic or bicyclic to hexacyclic heterocyclic compound one hydrogen atom bonding directly to an atom constituting the ring, more preferably a group obtained by removing from a monocyclic, bicyclic or tricyclic heterocyclic compound one hydrogen atom bonding directly to an atom constituting the ring, further preferably a group obtained by removing from pyridine, diazabenzene, triazine, azanaphthalene, diazanaphthalene, carbazole, dibenzofuran, dibenzothiophene, phenoxazine, phenothiazine, 9,10-dihydroacridine or 5,10-dihydrophenazine one hydrogen atom bonding directly to an atom constituting the ring, and particularly preferably a group obtained by removing from pyridine, diazabenzene, triazine, carbazole, dibenzofuran, dibenzothiophene, phenoxazine or phenothiazine one hydrogen atom bonding directly to an atom constituting the ring, since the light emitting device of the present embodiment is more excellent in maximum light emission efficiency, and these optionally further have a substituent.

In the substituted amino group as the substituent which a group represented by Ar^{Y1} optionally has, the substituent which an amino group has is preferably an aryl group or a monovalent hetero ring group, and more preferably an aryl group, and these groups optionally further have a substituent. The examples and preferable ranges of the aryl group and the monovalent hetero ring group as the substituent which an amino group has are the same as the examples and preferable ranges of the aryl group and the monovalent hetero ring group as the substituent which a group represented by Ar^{Y1} optionally has, respectively.

The substituent which the substituent which a group represented by Ar^{Y1} optionally has optionally further has is preferably an alkyl group, a cycloalkyl group, an alkoxy group, a cycloalkoxy group, an aryl group, an aryloxy group, a monovalent hetero ring group, a substituted amino group or a fluorine atom, more preferably an alkyl group, a cycloalkyl group, an aryl group, a monovalent hetero ring group or a substituted amino group, further preferably an alkyl group, a cycloalkyl group or an aryl group, and particularly preferably an alkyl group or a cycloalkyl group, and these groups optionally further have a substituent, but it is preferable that they do not further have a substituent.

The examples and preferable ranges of the aryl group, the monovalent hetero ring group and the substituted amino group as the substituent which the substituent which a group represented by Ar^{Y1} optionally has optionally further has are the same as the examples and preferable ranges of the aryl group, the monovalent hetero ring group and the substituted amino group as the substituent which a group represented by Ar^{Y1} optionally has, respectively.

The constitutional unit represented by the formula (Y) is preferably a constitutional unit represented by the formula (Y-1) or the formula (Y-2), since the light emitting device of the present embodiment is more excellent in maximum light emission efficiency. 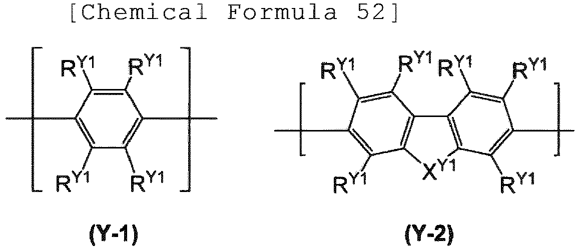 [wherein,
R^{Y1} represents a hydrogen atom, an alkyl group, a cycloalkyl group, an alkoxy group, a cycloalkoxy group, an aryl group, an aryloxy group, a monovalent hetero ring group, a substituted amino group or a fluorine atom, and these groups optionally have a substituent. When a plurality of the substituents are present, they may be the same or different and may be combined together to form a ring together with atoms to which they are attached. A plurality of R^{Y1} may be the same or different and may be combined together to form a ring together with carbon atoms to which they are attached.

X^{Y1} represents a group represented by -C(R^{Y2})₂-, - C(R^{Y2})=C(R^{Y2})- or -C(R^{Y2})₂-C(R^{Y2})₂-. R^{Y2} represents a hydrogen atom, an alkyl group, a cycloalkyl group, an alkoxy group, a cycloalkoxy group, an aryl group, an aryloxy group, a monovalent hetero ring group, a substituted amino group or a fluorine atom, and these groups optionally have a substituent. When a plurality of the substituents are present, they may be the same or different and may be combined together to form a ring together with atoms to which they are attached. A plurality of R^{Y2} may be the same or different and may be combined together to form a ring together with carbon atoms to which they are attached.]

R^{Y1} is preferably a hydrogen atom, an alkyl group, a cycloalkyl group, an aryl group, a monovalent hetero ring group or a substituted amino group, more preferably a hydrogen atom, an alkyl group, a cycloalkyl group or an aryl group, and further preferably a hydrogen atom or an alkyl group, and these groups optionally have a substituent.

In the formula (Y-1), at least one of R^{Y1} (preferably at least two of R^{Y1}) is preferably an alkyl group, a cycloalkyl group, an alkoxy group, a cycloalkoxy group, an aryl group, an aryloxy group, a monovalent hetero ring group, a substituted amino group or a fluorine atom, more preferably an alkyl group, a cycloalkyl group, an aryl group, a monovalent hetero ring group or a substituted amino group, further preferably an alkyl group, a cycloalkyl group or an aryl group, and particularly preferably an alkyl group, since the light emitting device of the present embodiment is more excellent in maximum light emission efficiency, and these groups optionally have a substituent.

R^{Y2} is preferably an alkyl group, a cycloalkyl group, an alkoxy group, a cycloalkoxy group, an aryl group, a monovalent hetero ring group or a substituted amino group, more preferably an alkyl group, a cycloalkyl group, an aryl group or a monovalent hetero ring group, and further preferably an alkyl group, a cycloalkyl group or an aryl group, since the light emitting device of the present embodiment is more excellent in maximum light emission efficiency, and these groups optionally have a substituent.

The examples and preferable ranges of the aryl group, the monovalent hetero ring group and the substituted amino group represented by R^{Y1} and R^{Y2} are the same as the examples and preferable ranges of the aryl group, the monovalent hetero ring group and the substituted amino group as the substituent which a group represented by Ar^{Y1} optionally has, respectively.

The examples and preferable range of the substituent which R^{Y1} and R^{Y2} optionally have are the same as the examples and preferable range of the substituent which a group represented by Ar^{Y1} optionally has.

X^{Y1} is preferably a group represented by -C(R^{Y2})₂- or -C(R^{Y2})₂-C(R^{Y2})₂-, and more preferably a group represented by -C(R^{Y2})₂-, since the light emitting device of the present embodiment is more excellent in maximum light emission efficiency.

The constitutional unit represented by the formula (Y) includes, for example, constitutional units represented by the following formulae. In the formulae, Z¹ and Z² represent the same meaning as described above.

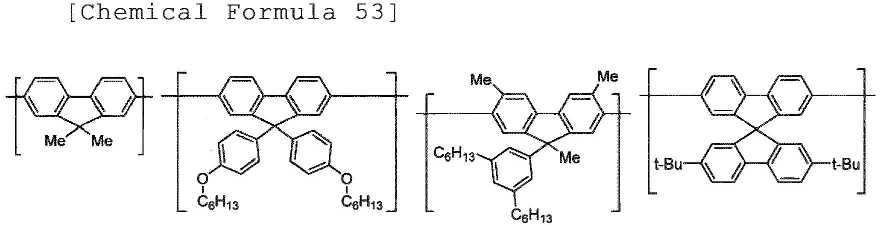

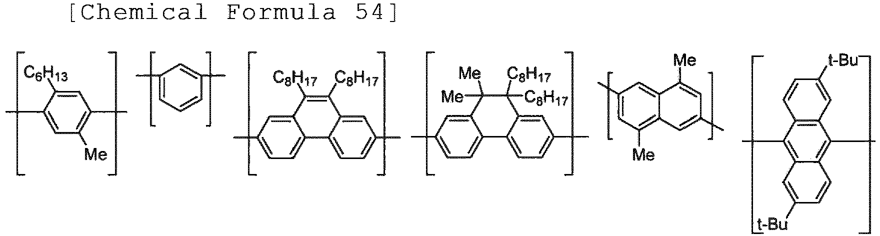

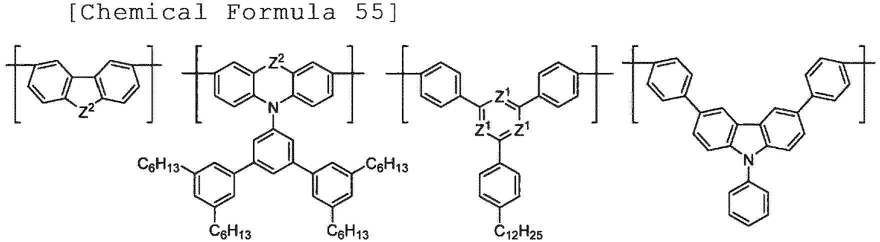

### • Constitutional unit represented by the formula (X)

a^{X1} and a^{X2} are usually integers of 0 to 10, and they are preferably integers of 0 to 5, more preferably integers of 0 to 3, further preferably integers of 0 to 2, and particularly preferably 0 or 1, since the light emitting device of the present embodiment is more excellent in maximum light emission efficiency.

R^{X1}, R^{X2} and R^{X3} represent preferably an alkyl group, a cycloalkyl group, an aryl group or a monovalent hetero ring group, more preferably an aryl group or a monovalent hetero ring group, and further preferably an aryl group, and these groups optionally have a substituent.

The examples and preferable ranges of the aryl group and the monovalent hetero ring group represented by R^{X1}, R^{X2} and R^{X3} are the same as the examples and preferable ranges of the aryl group and the monovalent hetero ring group as the substituent which a group represented by Ar^{Y1} optionally has, respectively.

The examples and preferable ranges of the arylene group and the divalent hetero ring group represented by Ar^{X1}, Ar^{X2}, Ar^{X3} and Ar^{X4} are the same as the examples and preferable ranges of the arylene group and the divalent hetero ring group represented by Ar^{Y1}, respectively.

The examples and preferable ranges of the arylene group and the divalent hetero ring group in the divalent group in which at least one arylene group and at least one divalent hetero ring group are bonded directly represented by Ar^{X2} and Ar^{X4} are the same as the examples and preferable ranges of the arylene group and the divalent hetero ring group represented by Ar^{Y1}, respectively.

The divalent group in which at least one arylene group and at least one divalent hetero ring group are bonded directly represented by Ar^{X2} and Ar^{X4} includes those that are the same as the divalent group in which at least one arylene group and at least one divalent hetero ring group are bonded directly represented by Ar^{Y1}.

Ar^{X1}, Ar^{X2}, Ar^{X3} and Ar^{X4} represent preferably an arylene group optionally having a substituent.

The examples and preferable range of the substituent which a group represented by Ar^{X1} to Ar^{X4} and R^{X1} to R^{X3} optionally has are the same as the examples and preferable range of the substituent which a group represented by Ar^{Y1} optionally has.

The constitutional unit represented by the formula (X) includes constitutional units represented by the following formulae. In the formulae, Z² represents the same meaning as described above.

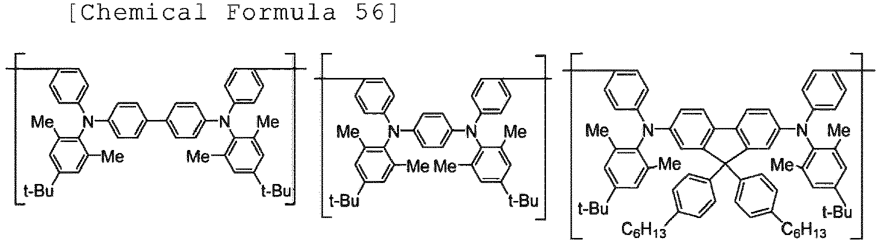

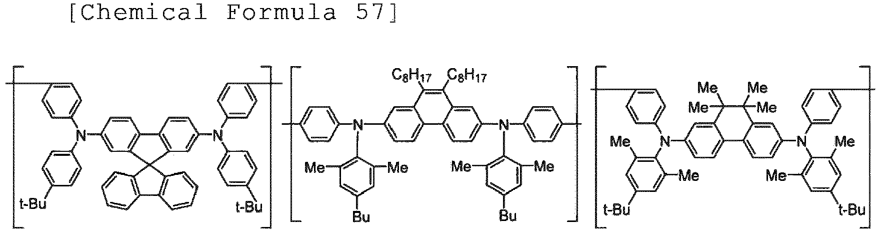

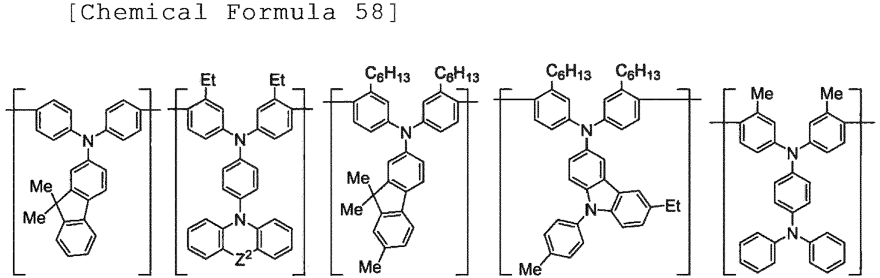

The polymer compound having a cross-linkable group includes, for example, polymer compounds P-1 to P-10 listed in Table 2. Here, "other" denotes a constitutional unit other than the constitutional unit represented by the formula (Z), the constitutional unit represented by the formula (Z'), the constitutional unit represented by the formula (X) and the constitutional unit represented by the formula (Y).

**[Table 2]**

| Polymer compound | Constitutional unit and molar ratio thereof | | | | |
|---|---|---|---|---|---|
| | Formula (Z) | Formula (Z') | Formula (X) | Formula (Y) | other |
| | p' | q' | r' | s' | t' |
| P-1 | 0.1 to 99.9 | 0 | 0.1 to 99.9 | 0 | 0 to 30 |
| P-2 | 0.1 to 99.9 | 0 | 0 | 0.1 to 99.9 | 0 to 30 |
| P-3 | 0 | 0.1 to 99.9 | 0.1 to 99.9 | 0 | 0 to 30 |
| P-4 | 0 | 0.1 to 99.9 | 0 | 0.1 to 99.9 | 0 to 30 |
| P-5 | 0.1 to 99.8 | 0.1 to 99.8 | 0.1 to 99.8 | 0 | 0 to 30 |
| P-6 | 0.1 to 99.8 | 0.1 to 99.8 | 0 | 0.1 to 99.8 | 0 to 30 |
| P-7 | 0.1 to 99.7 | 0.1 to 99.7 | 0.1 to 99.7 | 0.1 to 99.7 | 0 to 30 |
| P-8 | 0.1 to 100 | 0 | 0 | 0 | 0 to 30 |
| P-9 | 0 | 0.1 to 100 | 0 | 0 | 0 to 30 |
| P-10 | 0.1 to 99.9 | 0.1 to 99.9 | 0 | 0 | 0 to 30 |

In Table 2, p', q', r', s' and t' represent the molar ratio (% by mol) of each constitutional unit. p'+q'+r'+s'+t' = 100, and 70 ≤ p'+q'+r'+s' ≤ 100.

The polymer compound having a cross-linkable group may be any of a block copolymer, a random copolymer, an alternative copolymer, or a graft copolymer, and may also be in another form, and is preferably a copolymer obtained by copolymerizing multiple types of raw material monomers.

The polystyrene-equivalent number-average molecular weight of the polymer compound having a cross-linkable group is preferably 5×10³ to 1×10⁶, more preferably 1×10⁴ to 5×10⁵, further preferably 1.5×10⁴ to 2×10⁵, and particularly preferably 2×10⁴ to 1×10⁵. The polystyrene-equivalent weight-average molecular weight of the polymer compound having a cross-linkable group is preferably 1×10⁴ to 2×10⁶, more preferably 2×10⁴ to 1×10⁶, further preferably 5×10⁴ to 5×10⁵, and particularly preferably 1×10⁵ to 3×10⁵.

### • Method for producing polymer compound having cross-linkable group

The polymer compound having a cross-linkable group be produced by using known polymerization methods described in Chemical Review (Chem. Rev.), vol. 109, pp. 897-1091 (2009) and the like, and exemplified are methods of polymerizing by a coupling reaction using a transition metal catalyst such as the Suzuki reaction, the Yamamoto reaction, the Buchwald reaction, the Stille reaction, the Negishi reaction, the Kumada reaction, and the like.

The method of charging monomers in the above-described production method includes a method in which the whole monomer is charged in the reaction system at once, a method in which partial monomers are charged and reacted, then, the remaining monomers are charged at once, continuously or in parts, a method in which monomers are charged continuously or in parts, and the like.

The transition metal catalyst includes palladium catalysts, nickel catalysts and the like.

The post treatment of the polymerization reaction is conducted by using known methods, for example, a method of removing water-soluble impurities by liquid separation, a method of adding the reaction liquid after the polymerization reaction to a lower alcohol such as methanol and the like, filtrating the deposited precipitate, then, drying it, and other methods, singly or in combination. When the purity of the polymer compound having a cross-linkable group is low, it can be purified by a usual method such as, for example, recrystallization, re-precipitation, continuous extraction with a Soxhlet extractor, column chromatography and the like.

### • Low molecular compound having cross-linkable group

The low molecular compound having a cross-linkable group is preferably a low molecular compound represented by the formula (Z"), since the light emitting device of the present embodiment is more excellent in maximum light emission efficiency.

The examples and preferable range of m^{B1} are the same as the examples and preferable range of mA.

The examples and preferable range of m^{B2} are the same as the examples and preferable range of c.

The examples and preferable range of m^{B3} are the same as the examples and preferable range of m.

The examples and preferable range of Ar⁷ are the same as the examples and preferable range of Ar⁵.

The examples and preferable range of L^{B1} are the same as the examples and preferable range of L^{A}.

The examples and preferable range of R‴ are the same as the examples and preferable range of R".

The examples and preferable range of X" are the same as the examples and preferable range of X'.

The low molecular compound having a cross-linkable group includes, for example, compounds shown below.

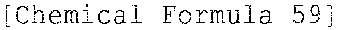

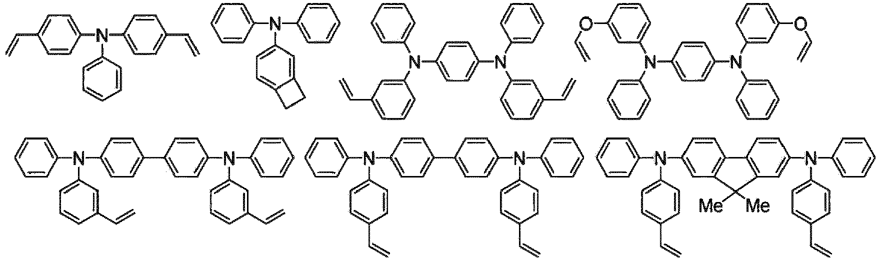

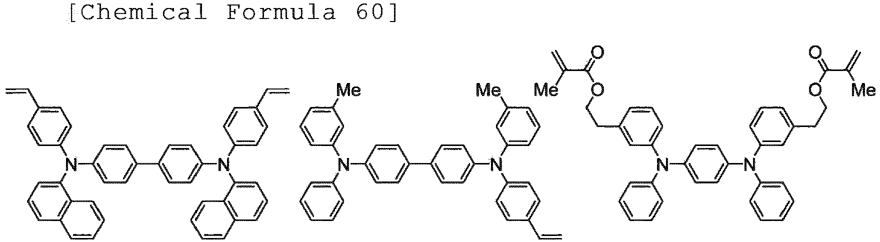

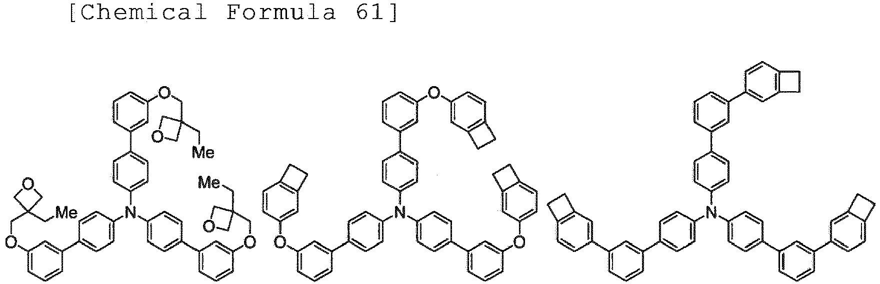

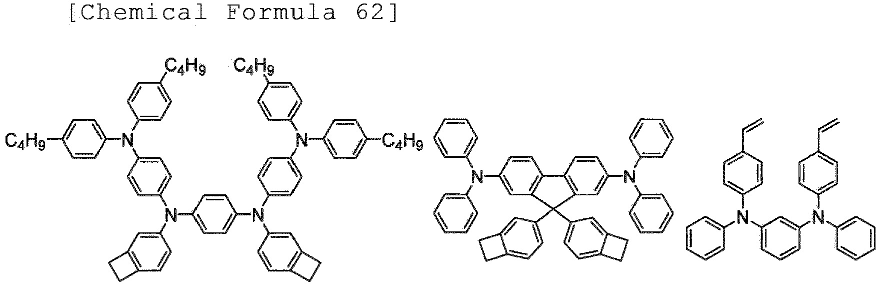

### [Second composition]

The second layer may be a layer containing a composition containing a compound (B-2), a crosslinked body of a compound having a cross-linkable group, and at least one material selected from the group consisting of a hole transporting material, a hole injection material, an electron transporting material, an electron injection material, a light emitting material and an antioxidant (hereinafter, referred to also as "second composition"). In the second layer, the hole transporting material, the hole injection material, the electron transporting material, the electron injection material and the light emitting material are different from the compound (B-2) and the crosslinked body of a compound having a cross-linkable group.

In the second composition, the compound (B-2), the crosslinked body of a compound having a cross-linkable group, the hole transporting material, the hole injection material, the electron transporting material, the electron injection material, the light emitting material and the antioxidant each may be contained singly or in combination of two or more.

The examples and preferable ranges of the hole transporting material, the hole injection material, the electron transporting material, the electron injection material, the light emitting material and the antioxidant contained in the second composition are the same as the examples and preferable range of the hole transporting material, the hole injection material, the electron transporting material, the electron injection material, the light emitting material and the antioxidant contained in the first composition.

In the second composition, the total content of the compound (B-2), the crosslinked body of a compound having a cross-linkable group, the hole transporting material, the hole injection material, the electron transporting material, the electron injection material, the light emitting material and the antioxidant may be within a range in which the function as the second composition is exhibited. In the second composition, the total content of the compound (B-2), the crosslinked body of a compound having a cross-linkable group, the hole transporting material, the hole injection material, the electron transporting material, the electron injection material, the light emitting material and the antioxidant, for example, may be 1 to 100% by mass, may be 10 to 100% by mass, may be 30 to 100% by mass, may be further preferably 50 to 100% by mass, may be 70 to 100% by mass, or may be 90 to 100% by mass, based on the total amount of the second composition.

In the second composition, the contents of the hole transporting material, the hole injection material, the electron transporting material, the electron injection material and the light emitting material are each usually 1 to 1000 parts by mass, when the total content of the compound (B-2) and the crosslinked body of a compound having a cross-linkable group is taken as 100 parts by mass.

The examples and preferable range of the antioxidant contained in the second composition are the same as the examples and preferable range of the antioxidant contained in the first composition. In the second composition, the content of the antioxidant is usually 0.001 to 10 parts by mass, when the total content of the compound (B-2) and the crosslinked body of a compound having a cross-linkable group is taken as 100 parts by mass.

### • Second ink

The second layer can be formed, for example, using a composition containing a compound (B-2), a compound having a cross-linkable group, and a solvent (hereinafter, referred to also as "second ink"). The second ink can be suitably used for wet methods explained in the section of the first ink. The preferable range of the viscosity of the second ink is the same as the preferable range of the viscosity of the first ink. The examples and preferable range of the solvent contained in second ink are the same as the examples and preferable range of the solvent contained in the first ink.

In the second ink, the content of the solvent is usually 1000 to 100000 parts by mass, when the total content of the compound (B-2) and the compound having a cross-linkable group is taken as 100 parts by mass.

The second ink may further contain at least one selected from the group consisting of a hole transporting material, a hole injection material, an electron transporting material, an electron injection material, a light emitting material and an antioxidant.

The examples and preferable ranges of the hole transporting material, the electron transporting material, the hole injection material, the electron injection material, the light emitting material and the antioxidant which the second ink may further contain are the same as the examples and preferable ranges of the hole transporting material, the electron transporting material, the hole injection material, the electron injection material, the light emitting material and the antioxidant contained in the second composition.

The contents of the hole transporting material, the electron transporting material, the hole injection material, the electron injection material and the light emitting material which the second ink may further contain are each usually 1 to 1000 parts by mass, when the total content of the compound (B-2) and the compound having a cross-linkable group is taken as 100 parts by mass. The content of the antioxidant which the second ink may further contain is usually 0.001 to 10 parts by mass, when the total content of the compound (B-2) and the compound having a cross-linkable group is taken as 100 parts by mass.

### <Light emitting device>

The light emitting device of the present embodiment is a light emitting device having an anode and a cathode, and a first layer and a second layer disposed between the anode and the cathode.

The light emitting device of the present embodiment may further have other layers than the anode, the cathode, the first layer and the second layer.

The light emitting device of the present embodiment is excellent in maximum light emission efficiency. Although the reason for such an effect is not necessarily clear, it is believed that compounds corresponding to compounds represented by the formula (B) (compound (B-1) and compound (B-2)) contained in two layers (first layer and second layer) disposed between an anode and a cathode cause, for example, effects such as a reduction of charge injection barrier at the layer interface, an improvement of charge injectability between layers, and the like, thereby improving the maximum light emission efficiency of the light emitting device of the present embodiment.

From the above-described standpoint, in the light emitting device of the present embodiment, the compound (B-1) and the compound (B-2) may be the same or different, but it is preferable that at least one of the compound (B-1) and at least one of the compound (B-2) are the same.

From the above-described standpoint, it is preferable that the first layer and the second layer are adjacent, since the light emitting device of the present embodiment is more excellent in maximum light emission efficiency.

In the light emitting device of the present embodiment, at least one of the compound (B-1) contained in the first layer and the compound (B-2) contained in the second layer is a compound represented by the formula (B) in which n^{B1} is 1 or more (hereinafter, referred to also as "compound (B')").

In the light emitting device of the present embodiment, it is preferable that at least one of the compound (B-1) contained in the first layer is a compound (B'), since the light emitting device of the present embodiment is more excellent in maximum light emission efficiency. That is, the first layer is preferably a layer containing a compound (B'), since the light emitting device of the present embodiment is more excellent in maximum light emission efficiency. The first layer may contain a compound (B') only singly or in combination of two or more.

In the light emitting device of the present embodiment, when the first layer is a layer containing a compound (B'), the number of types of the compound (B') contained in the first layer is usually 1 to 20, and it is preferably 1 to 10, more preferably 1 to 5, further preferably 1 to 3, and particularly preferably 1 or 2, since production of the light emitting device of the present embodiment is easy.

In the light emitting device of the present embodiment, when the first layer is a layer containing a compound (B'), the content of the compound (B') in the first layer (namely, the proportion of the compound (B') occupying the compound (B-1)) may be 1 to 100% by mass, may be 5 to 100% by mass, may be 10 to 100% by mass, may be 30 to 100% by mass, may be 50 to 100% by mass, and may be 70 to 100% by mass, when the total content of the compound (B-1) contained in the first layer is taken as 100 parts by mass.

In the light emitting device of the present embodiment, at least one of the compound (B-2) contained in the second layer may be a compound (B'). That is, in the light emitting device of the present embodiment, the second layer may be a layer containing a compound (B') and a crosslinked body of a compound having a cross-linkable group. The second layer may contain the compound (B') only singly or in combination of two or more.

In the light emitting device of the present embodiment, when the second layer is a layer containing a compound (B'), the number of types of the compound (B') contained in the second layer is usually 1 to 20, and it is preferably 1 to 10, more preferably 1 to 5, further preferably 1 to 3, particularly preferably 1 or 2, and especially preferably 1, since production of the light emitting device of the present embodiment is easy.

In the light emitting device of the present embodiment, when the second layer is a layer containing a compound (B'), the content of the compound (B') in the second layer (namely, the proportion of the compound (B') occupying the compound (B-2)) may be 1 to 100% by mass, may be 5 to 100% by mass, may be 10 to 100% by mass, may be 30 to 100% by mass, may be 50 to 100% by mass, may be 70 to 100% by mass, and may be 90 to 100% by mass, when the total content of the compound (B-2) contained in the second layer is taken as 100 parts by mass.

The first layer is usually a light emitting layer (hereinafter, referred to as "first light emitting layer").

The second layer is usually a hole injection layer, a hole transporting layer, a light emitting layer (namely, separate light emitting layer from the first light emitting layer, hereinafter, referred to as "second light emitting layer") or an electron transporting layer, preferably a hole injection layer, a hole transporting layer or a second light emitting layer, further preferably a hole injection layer or a hole transporting layer, and particularly preferably a hole transporting layer.

The second layer is preferably a layer disposed between an anode and a first layer, more preferably a hole injection layer, a hole transporting layer or a second light emitting layer disposed between an anode and a first layer, further preferably a hole injection layer or a hole transporting layer disposed between an anode and a first layer, and particularly preferably a hole transporting layer disposed between an anode and a first layer, since the light emitting device of the present embodiment is more excellent in maximum light emission efficiency.

When the second layer is a second light emitting layer disposed between an anode and a first layer, it is preferable to further has at least one of a hole injection layer and a hole transporting layer between an anode and a second layer, since the light emitting device of the present embodiment is more excellent in maximum light emission efficiency. In addition, when the second layer is a second light emitting layer disposed between an anode and a first layer, it is preferable to further has at least one of an electron injection layer and an electron transporting layer between a cathode and a first layer, since the light emitting device of the present embodiment is more excellent in maximum light emission efficiency.

When the second layer is a second light emitting layer disposed between a cathode and a first layer, it is preferable to further has at least one of a hole injection layer and a hole transporting layer between an anode and a first layer, since the light emitting device of the present embodiment is more excellent in maximum light emission efficiency. In addition, when the second layer is a second light emitting layer disposed between a cathode and a first layer, it is preferable to further has at least one of an electron injection layer and an electron transporting layer between a cathode and a second layer, since the light emitting device of the present embodiment is more excellent in maximum light emission efficiency.

When the second layer is a hole transporting layer disposed between an anode and a first layer, it is preferable to further has a hole injection layer between an anode and a second layer, since the light emitting device of the present embodiment is more excellent in maximum light emission efficiency. In addition, when the second layer is a hole transporting layer disposed between an anode and a first layer, it is preferable to further has at least one of an electron injection layer and an electron transporting layer between a cathode and a first layer, since the light emitting device of the present embodiment is more excellent in maximum light emission efficiency.

When the second layer is a hole injection layer disposed between an anode and a first layer, it is preferable to further has a hole transporting layer between a first layer and a second layer, since the light emitting device of the present embodiment is more excellent in maximum light emission efficiency. In addition, when the second layer is a hole injection layer disposed between an anode and a first layer, it is preferable to further has at least one of an electron injection layer and an electron transporting layer between a cathode and a first layer, since the light emitting device of the present embodiment is more excellent in maximum light emission efficiency.

When second layer is an electron transporting layer disposed between a cathode and a first layer, it is preferable to further has at least one of a hole injection layer and a hole transporting layer between an anode and a first layer, since the light emitting device of the present embodiment is more excellent in maximum light emission efficiency. In addition, when the second layer is an electron transporting layer disposed between a cathode and a first layer, it is preferable to further has an electron injection layer between a cathode and a second layer, since the light emitting device of the present embodiment is more excellent in maximum light emission efficiency.

The specific layer constitution of the light emitting device of the present embodiment includes, for example, layer constitutions represented by the following (D1) to (D17). The light emitting device of the present embodiment usually has a substrate, and lamination may be carried out from an anode on the substrate, or lamination may be carried out from a cathode on the substrate.
(D1) anode/hole transporting layer (second layer)/first light emitting layer (first layer)/cathode
(D2) anode/hole injection layer/hole transporting layer (second layer)/first light emitting layer (first layer)/cathode
(D3) anode/hole injection layer/hole transporting layer (second layer)/first light emitting layer (first layer)/electron transporting layer/cathode
(D4) anode/hole injection layer/hole transporting layer (second layer)/first light emitting layer (first layer)/electron injection layer/cathode
(D5) anode/hole injection layer/hole transporting layer (second layer)/first light emitting layer (first layer)/electron transporting layer/electron injection layer/cathode
(D6) anode/hole injection layer/hole transporting layer (second layer)/first light emitting layer (first layer)/second light emitting layer/electron transporting layer/electron injection layer/cathode
(D7) anode/hole injection layer/hole transporting layer (second layer)/second light emitting layer/first light emitting layer (first layer)/electron transporting layer/electron injection layer/cathode
(D8) anode/second light emitting layer (second layer)/first light emitting layer (first layer)/cathode
(D9) anode/hole injection layer/second light emitting layer (second layer)/first light emitting layer (first layer)/electron transporting layer/cathode
(D10) anode/hole injection layer/second light emitting layer (second layer)/first light emitting layer (first layer)/electron injection layer/cathode
(D11) anode/hole injection layer/second light emitting layer (second layer)/first light emitting layer (first layer)/electron transporting layer/electron injection layer/cathode
(D12) anode/hole injection layer/hole transporting layer/second light emitting layer (second layer)/first light emitting layer (first layer)/electron transporting layer/electron injection layer/cathode
(D13) anode/hole injection layer/hole transporting layer/first light emitting layer (first layer)/second light emitting layer (second layer)/electron transporting layer/electron injection layer/cathode
(D14) anode/hole injection layer/hole transporting layer/first light emitting layer (first layer)/electron transporting layer (second layer)/electron injection layer/cathode
(D15) anode/hole injection layer (second layer)/first light emitting layer (first layer)/cathode
(D16) anode/hole injection layer (second layer)/first light emitting layer (first layer)/electron transporting layer/electron injection layer/cathode
(D17) anode/hole injection layer (second layer)/hole transporting layer/first light emitting layer (first layer)/electron transporting layer/electron injection layer/cathode

In the above-described (D1) to (D17), "/" means that layers before and after it are laminated adjacently. For example, "hole transporting layer (second layer)/first light emitting layer (first layer)" means that a hole transporting layer (second layer) and a first light emitting layer (first layer) are laminated adjacently.

In the light emitting device of the present embodiment, two or more layers of an anode, a hole injection layer, a hole transporting layer, a light emitting layer, an electron transporting layer, an electron injection layer and a cathode may be provided as necessary, respectively. When a plurality of anodes, hole injection layers, hole transporting layers, light emitting layers, electron transporting layers, electron injection layers and cathodes are present, materials constituting them may be the same or different, respectively.

The thicknesses of the anode, the hole injection layer, the hole transporting layer, the first layer, the second layer, the light emitting layer, the electron transporting layer, the electron injection layer and the cathode are usually 1 nm to 1 µm, preferably 2 nm to 500 nm, and further preferably 5 nm to 150 nm.

In the light emitting device of the present embodiment, the order, number and thickness of layers to be laminated may be adjusted in consideration of the maximum light emission efficiency and luminance life of the light emitting device, and the like.

### [First light emitting layer]

The first light emitting layer is usually a first layer.

### [Second light emitting layer]

The second light emitting layer is usually a second layer or a layer containing a light emitting material, and preferably a layer containing a light emitting material. When the second light emitting layer is a layer containing a light emitting material, the light emitting material contained in the second light emitting layer includes, for example, light emitting materials which the second composition may contain described above. The light emitting material contained in the second light emitting layer may be contained singly or in combination of two or more.

When the light emitting device of the present embodiment has a second light emitting layer and when a hole injection layer described later, a hole transporting layer described later and an electron transporting layer described later are not a second layer, it is preferable that the second light emitting layer is a second layer.

### [Hole transporting layer]

The hole transporting layer is a second layer or a layer containing a hole transporting material, and is preferably a second layer. When the hole transporting layer is a layer containing a hole transporting material, the hole transporting material includes, for example, hole transporting materials which the second composition may contain described above. The hole transporting material contained in the hole transporting layer may be contained singly or in combination of two or more.

When the light emitting device of the present embodiment has a hole transporting layer and when a hole injection layer described later, a second light emitting layer described above and an electron transporting layer described later are not a second layer, it is preferable that the hole transporting layer is a second layer.

### [Electron transporting layer]

The electron transporting layer is a second layer or a layer containing an electron transporting material, and is preferably a layer containing an electron transporting material. When the electron transporting layer is a layer containing an electron transporting material, the electron transporting material contained in the electron transporting layer includes, for example, electron transporting materials which the second composition may contain described above. The electron transporting material contained in the electron transporting layer may be contained singly or in combination of two or more.

When the light emitting device of the present embodiment has an electron transporting layer and when a hole injection layer described later, a second light emitting layer described above and a hole transporting layer described above are not a second layer, it is preferable that the electron transporting layer is a second layer.

### [Hole injection layer]

The hole injection layer is a second layer or a layer containing a hole injection material, and is preferably a layer containing a hole injection material. When the hole injection layer is a layer containing a hole injection material, the hole injection material contained in the hole injection layer includes, for example, hole injection materials which the second composition may contain described above. The hole injection material contained in the hole injection layer may be contained singly or in combination of two or more.

When the light emitting device of the present embodiment has a hole injection layer and when a second light emitting layer described above, a hole transporting layer described above and an electron transporting layer described above are not a second layer, it is preferable that the hole injection layer is a second layer.

### [Electron injection layer]

The electron injection layer is a layer containing an electron injection material. The electron injection material contained in the electron injection layer includes, for example, electron injection materials which the second composition may contain described above. The electron injection material contained in the electron injection layer may be contained singly or in combination of two or more.

### [Substrate/electrode]

The substrate in a light emitting device is preferably a substrate that does not chemically change in forming an electrode and forming an organic layer. The substrate may be a substrate made of a material such as, for example, glass, plastic, silicon, and the like. When a non-transparent substrate is used, it is preferable that an electrode farthest from the substrate is transparent or semi-transparent.

The material of the anode includes, for example, electrically conductive metal oxides and semi-transparent metals, and includes preferably indium oxide, zinc oxide, tin oxide; electrically conductive compounds such as indium•tin•oxide (ITO), indium•zinc•oxide, and the like; argentine-palladium-copper (APC) composite; NESA, gold, platinum, silver, and copper.

The material of the cathode includes, for example, metals such as lithium, sodium, potassium, rubidium, cesium, beryllium, magnesium, calcium, strontium, barium, aluminum, zinc, indium, and the like; alloys composed of two or more of them; alloys composed of one or more of them and one or more of silver, copper, manganese, titanium, cobalt, nickel, tungsten, and tin; and, graphite and graphite intercalation compounds. The alloy includes, for example, a magnesium-silver alloy, a magnesium-indium alloy, a magnesium-aluminum alloy, an indium-silver alloy, a lithium-aluminum alloy, a lithium-magnesium alloy, a lithium-indium alloy, and a calcium-aluminum alloy.

In the light emitting device of the present embodiment, at least one of an anode and a cathode is usually transparent or semi-transparent, and it is preferable that an anode is transparent or semi-transparent.

The method for forming an anode and a cathode includes, for example, a vacuum vapor deposition method, a sputtering method, an ion plating method, a plating method and a lamination method.

### [Production method of light emitting device]

In the method for producing a light emitting device of the present embodiment, the method for forming a first layer, a second layer, and other layers than the first layer and the second layer includes, for example, dry methods such as a vacuum vapor deposition method and the like and wet methods explained in the section of the first ink in the case where a low molecular compound is used, and includes, for example, wet methods explained in the section of the first ink in the case where a polymer compound is used. In the method for producing a light emitting device of the present embodiment, a first layer, a second layer, and other layers than the first layer and the second layer may be formed by wet methods explained in the section of the first ink using various inks described above or inks containing various materials described above, or may be formed by dry methods such as a vacuum vapor deposition method and the like.

The method for producing a first layer and a second layer includes, for example, dry methods and wet methods, and wet methos are preferable, since production of the light emitting device of the present embodiment is easy. In the method for producing a first layer and a second layer, the dry method includes, for example, a vacuum vapor deposition method. In the method for producing a first layer and a second layer, the wet method includes, for example, wet methods explained in the section of the first ink.

When a first layer is formed by a wet method, it is preferable to use a first ink, since production of the light emitting device of the present embodiment is easy. That is, it is preferable to form a first layer by a wet method using a first ink.

When a second layer is formed by a wet method, it is preferable to use a second ink, since production of the light emitting device of the present embodiment is easy. That is, it is preferable to form a second layer by a wet method using a second ink.

In the method for producing a light emitting device of the present embodiment, a layer containing a crosslinked body of a compound having a cross-linkable (for example, second layer) can be formed, for example, by forming a layer containing a compound having a cross-linkable group, then, performing heating or irradiation (preferably heating), thereby crosslinking the compound having a cross-linkable group contained in the layer. When the compound having a cross-linkable group is contained in the layer in the crosslinked state (a crosslinked body of a compound having a cross-linkable group), the layer is substantially insolubilized in a solvent. Therefore, the layer containing a crosslinked body of a compound having a cross-linkable group can be suitably used for lamination of layers, in production of a light emitting device of the present embodiment.

From the above-described standpoint, in the method for producing a light emitting device of the present embodiment, the step of forming a second layer preferably includes a step in which a layer containing a compound having a cross-linkable group is formed, then, the compound having a cross-linkable group contained in the layer is crosslinked, to form a second layer containing a crosslinked body of the compound having a cross-linkable group. In the step of forming a second layer, the method for crosslinking a compound having a cross-linkable group is preferably a method for crosslinking by heating or irradiation, and more preferably a method for crosslinking by heating, since production of the light emitting device of the present embodiment is easy.

The temperature of heating for crosslinking is usually 25°C to 300°C, preferably 50°C to 260°C, more preferably 130°C to 230°C, and further preferably 180°C to 210°C.

The time of heating is usually 0.1 minutes to 1000 minutes, preferably 0.5 minutes to 500 minutes, more preferably 1 minute to 120 minutes, and further preferably 10 minutes to 60 minutes.

The kind of the light used for irradiation includes, for example, ultraviolet, near-ultraviolet and visible light.

The step of forming a second layer includes, for example, a method in which a layer is formed by a wet method using a second ink, then, a compound having a cross-linkable group contained in the layer is crosslinked, to form a second layer; a method in which a layer is formed by a dry method or a wet method using a compound having a cross-linkable group and a compound (B-2), then, the compound having a cross-linkable group contained in the layer is crosslinked, to form a second layer; and a method in which a layer containing a compound having a cross-linkable group is formed by a dry method or a wet method (preferably wet method) using the compound having a cross-linkable group, the compound having a cross-linkable group contained in the layer is crosslinked, to form a layer containing a crosslinked body of the compound having a cross-linkable group, then, the layer containing the crosslinked body is allowed to contain a compound (B-2), to form a second layer.

The method for analyzing components contained in a first layer, a second layer, or layers other than the first layer and the second layer includes, for example, chemical separation analysis methods such as extraction and the like, instrumental analysis methods such as infrared spectroscopy (IR), nuclear magnetic resonance spectroscopy (NMR), mass spectrometry (MS), and the like, and analysis methods combining chemical separation analysis methods and instrumental analysis methods.

By performing solid-liquid extraction using an organic solvent such as toluene, xylene, chloroform, tetrahydrofuran, and the like on a first layer, a second layer, or layers other than the first layer and the second layer, it is possible to separate into components substantially insoluble in organic solvents (insoluble components) and components soluble in organic solvents (soluble components). The insoluble components can be analyzed by infrared spectroscopy or nuclear magnetic resonance spectroscopy, while the soluble components can be analyzed by nuclear magnetic resonance spectroscopy or mass spectrometry.

The light emitting device of the present embodiment can be produced, for example, by laminating layers in series on a substrate. Specifically, an anode is provided on a substrate, layers such as a hole injection layer, a hole transporting layer, and the like are provided thereon, a light emitting layer is provided thereon, layers such as an electron transporting layer, an electron injection layer, and the like are provided thereon, and further, a cathode is laminated thereon, thus, a light emitting device can be produced. In another production method, a cathode is provided on a substrate, layers such as an electron injection layer, an electron transporting layer, a light emitting layer, a hole transporting layer, a hole injection layer, and the like are provided thereon, and further, an anode is laminated thereon, thus, a light emitting device can be produced. In still another production method, a light emitting device can be produced by bonding an anode or an anode-side base material in which each layer is laminated on the anode and a cathode or a cathode-side base material in which each layer is laminated on the cathode, facing each other.

In production of the light emitting device of the present embodiment, when a material used for forming a hole injection layer, a material for forming a light emitting layer, a material for forming a hole transporting layer, a material for forming an electron transporting layer, and a material for forming an electron injection layer are soluble in solvents used in forming layers adjacent to a hole injection layer, a light emitting layer, a hole transporting layer, an electron transporting layer and an electron injection layer, respectively, it is preferable to avoid dissolution of the material in the solvent. As the method for avoiding dissolution of the material, i) a method of using a material having a cross-linkable group, or ii) a method of making a difference in solubility in solvents for adjacent layers, is preferred. In the above method i), a layer can be insolubilized by forming a layer using a material having a cross-linkable group, then, crosslinking the cross-linkable group. In the above method ii), for example, when an electron transporting layer is laminated on a light emitting layer utilizing a difference in solubility, the electron transporting layer can be laminated on the light emitting layer by using an ink having low solubility in the light emitting layer.

### [Application]

The light emitting device of the present embodiment can be suitably used as light sources for backlight of liquid displays, light sources for illumination, organic EL illumination, displays for computers, televisions, portable terminals, and the like (for example, organic EL displays and organic EL televisions).

### EXAMPLES

The present invention will be explained further in detail by examples below, but the present invention is not limited to these examples.

In examples, the polystyrene-equivalent number-average molecular weight (Mn) and the polystyrene-equivalent weight-average molecular weight (Mw) of a polymer compound were determined by size exclusion chromatography (SEC) using tetrahydrofuran as the mobile phase. Each measurement conditions for SEC are as described below.

A polymer compound to be measured was dissolved at a concentration of about 0.05% by mass in tetrahydrofuran, and 10 µL of the solution was injected into SEC. The mobile phase was flowed at a flow rate of 2.0 mL/min. As the column, PLgel MIXED-B (manufactured by Polymer Laboratories Inc.) was used. As the detector, UV-VIS detector (trade name: SPD-10Avp, manufactured by Shimadzu Corp.) was used.

### <Synthesis of compounds M1 to M17>

A compound M1 and a compound M17 were synthesized according to a method described in JP-A No. 2010-189630.

A compound M2 was synthesized according to a method described in JP-A No. 2008-106241.

A compound M3 was synthesized according to a method described in JP-A No. 2010-215886.

A compound M4, a compound M9 and a compound M15 were synthesized with reference to a method described in International Publication WO2002/045184.

A compound M5 was synthesized according to a method described in International Publication WO2005/049546.

A compound M6 was synthesized according to a method described in International Publication WO2011/049241.

A compound M7 was synthesized according to a method described in International Publication WO2015/145871.

A compound M8 was synthesized according to a method described in International Publication WO2013/146806.

A compound M10 was synthesized with reference to a method described in JP-A No. 2014-1328.

A compound M11 was synthesized according to a method described in International Publication WO2016/031639.

A compound M12 and a compound M14 were synthesized according to a method described in JP-A No. 2011-174062.

A compound M13 was synthesized with reference to a method described in JP-A No. 2010-215886.

A compound M16 was synthesized according to a method described in International Publication WO2012/086671.

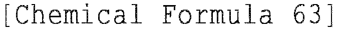

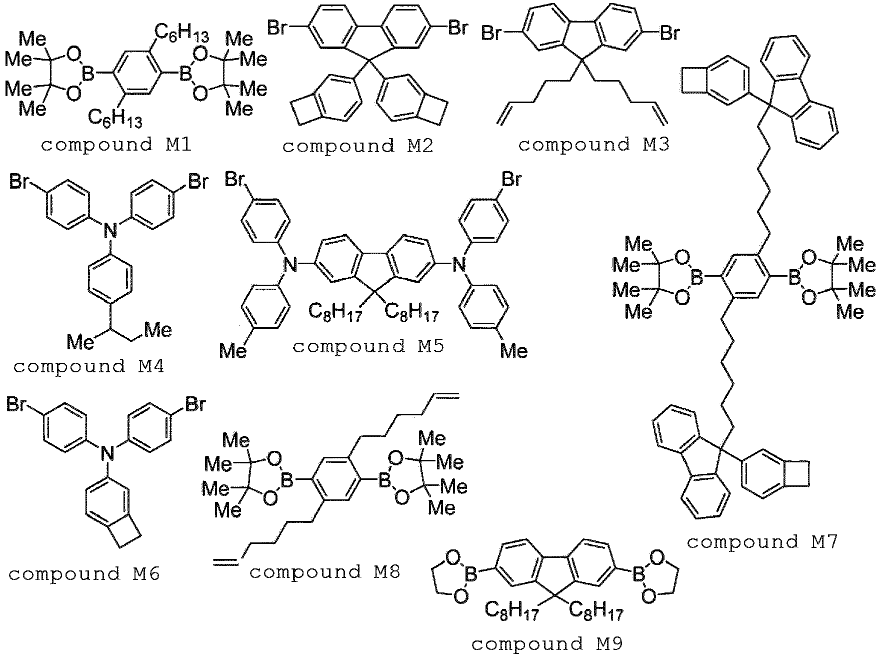

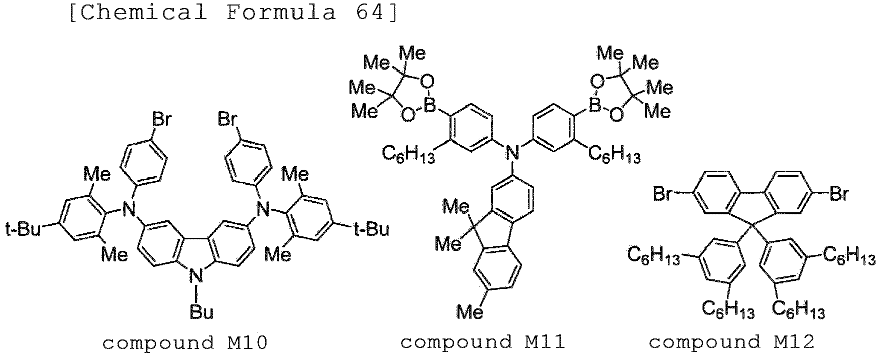

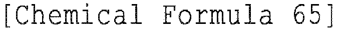

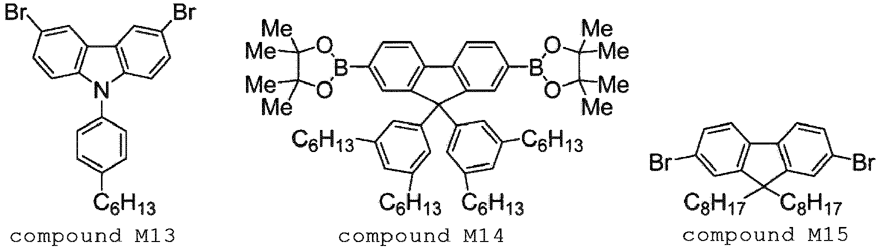

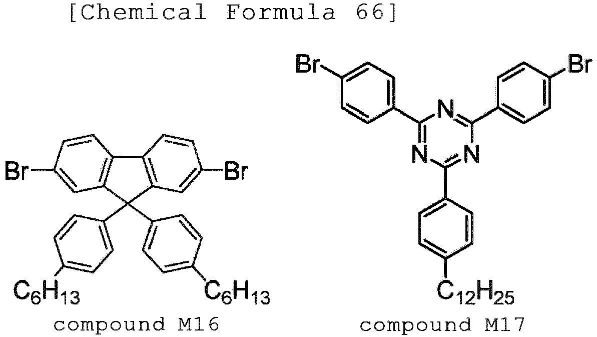

### <Synthesis of polymer compounds HTL-1 to HTL-8>

Polymer compounds HTL-1 to HTL-8 were synthesized using compounds of the types and molar ratios shown in Table 3 by synthesis methods shown in the same table. Mn and Mw of the resultant polymer compounds are as described in Table 3.

The synthesis of the polymer compound HTL-1 is explained below as an example.

The polymer compound HTL-1 was synthesized according to a method described in JP-A No. 2015-110751 using the compound M1, the compound M2, the compound M3 and the compound M4. The polymer compound HTL-1 had an Mn of 5.9×10⁴ and an Mw of 2.5×10⁵.

The polymer compound HTL-1 is a copolymer constituted of a constitutional unit derived from the compound M1, a constitutional unit derived from the compound M2, a constitutional unit derived from the compound M3, and a constitutional unit derived from the compound M4, at a molar ratio of 50:5:5:40, according to theoretical values calculated from the amounts of the charged raw materials.

**[Table 3]**

| Polymer compound | Constitutional unit derived from compound (molar ratio) | | | | Synthesis method | Mn | Mw |
|---|---|---|---|---|---|---|---|
| HTL-1 | M1 (50) | M2 (5) | M3 (5) | M4 (40) | Method described in JP-A No. 2015-110751 | 5.9×10⁴ | 2.5×10⁵ |
| HTL-2 | M1 (50) | M5 (42.5) | M6 (7.5) | - | Method described in WO2013/146806 | 1.9×10⁴ | 9.9×10⁴ |
| HTL-3 | M7 (45) | M8 (5) | M5 (50) | - | Method described in WO2015/145871 | 2.3×10⁴ | 1.2×10⁵ |
| HTL-4 | M9 (50) | M4 (42.5) | M6 (7.5) | - | Method described in WO2011/049241 | 8.9×10⁴ | 4.2×10⁵ |
| HTL-5 | M11 (50) | M12 (40) | M2 (5) | M3 (5) | Method described in WO2016/031639 | 5.3×10⁴ | 2.0×10⁵ |
| HTL-6 | M1 (50) | M10 (40) | M2 (5) | M3 (5) | Method with reference to JP-A No. 2104-001328 | 1.8×10⁴ | 1.5×10⁵ |
| HTL-7 | M1 (50) | M13 (40) | M2 (5) | M3 (5) | Method with reference to JP-A No. 2010-215886 | 2.4×10⁴ | 1.7×10⁵ |
| HTL-8 | M14 (50) | M5 (30) | M15 (12.5) | M2 (7.5) | Method described in JP-A No. 2012-144722 | 7.8×10⁴ | 2.6×10⁵ |

### <Acquisition of compound HTM-1>

A compound HTM-1 was purchased from Luminescence Technology Corp.

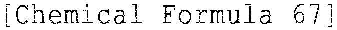

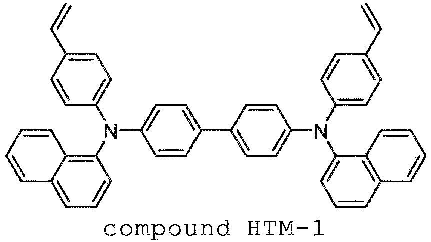

### <Synthesis of polymer compound HP-1>

A polymer compound HP-1 was synthesized according to a method described in JP-A No. 2012-036388, using the compound M1, the compound M16 and the compound M17. The polymer compound HP-1 had an Mn of 9.6×10⁴ and an Mw of 2.2×10⁵.

The polymer compound HP-1 is a copolymer constituted of a constitutional unit derived from the compound M1, a constitutional unit derived from the compound M16, and a constitutional unit derived from the compound M17 at a molar ratio of 50:40:10, according to the theoretical values calculated from the amounts of the charged raw materials.

### <Synthesis and acquisition of compounds B-H1 to B-H7>

A compound B-H1 was synthesized according to a method described in JP-A No. 2015-110751.

A compound B-H2 was synthesized with reference to a method described in International Publication WO2010/136109.

A compound B-H3 was synthesized with reference to a method described in JP-A No. 2010-254676.

A compound B-H4, a compound B-H6 and a compound B-H8 were purchased from Luminescence Technology Corp.

A compound B-H5 was synthesized according to a method described in JP-A No. 2010-189630.

A compound B-H7 was synthesized with reference to a method described in International Publication WO2010/015306.

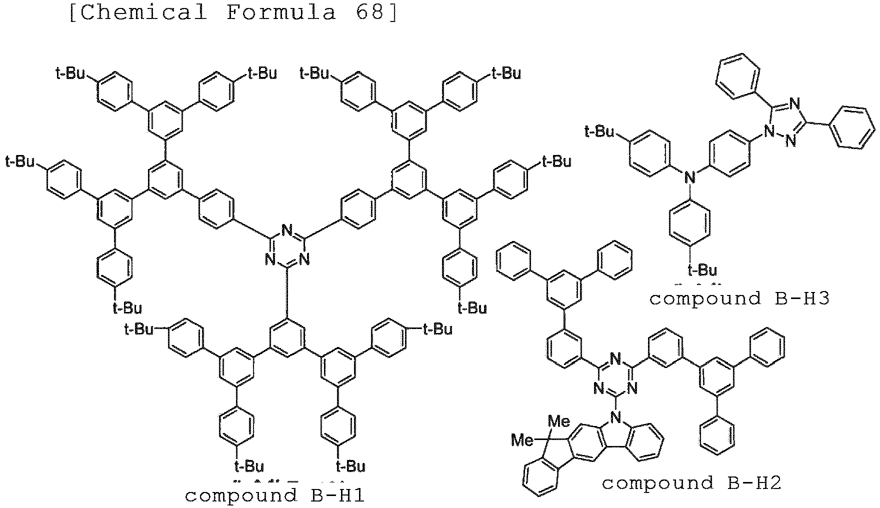

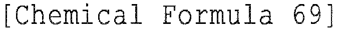

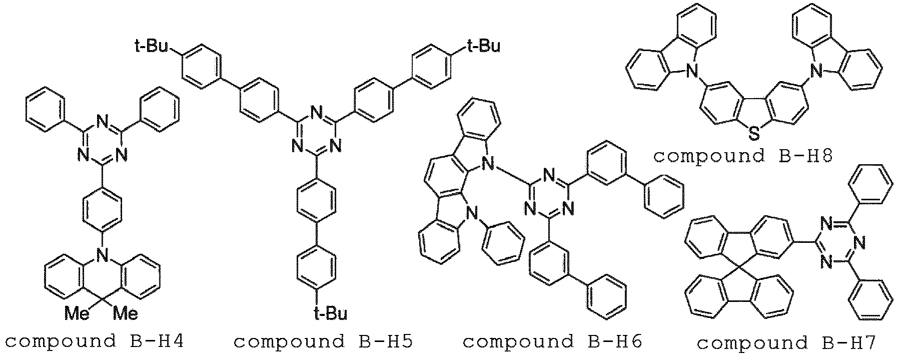

### <Synthesis of metal complexes B-G1, B-G2 and B-R1>

A metal complex B-G1 was synthesized according to a method described in International Publication WO2009/131255.

A metal complex B-G2 was synthesized with reference to a method described in International Publication WO2011/032626.

A metal complex B-R1 was synthesized with reference to a method described in JP-A No. 2006-188673.

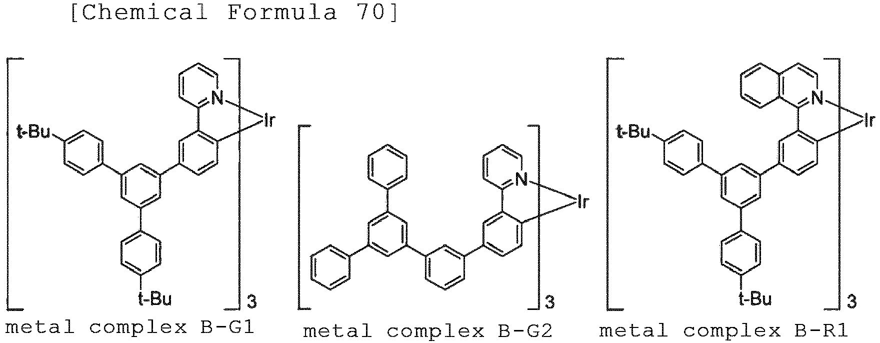

### <Example D1> Fabrication and evaluation of light emitting device D1

### (Formation of anode and hole injection layer)

An ITO film with a thickness of 45 nm was attached to a glass substrate by a sputtering method, to form an anode. On the anode, a polythiophene•sulfonic acid type hole injection agent AQ-1200 (manufactured by Plextronics) was spin-coated to form a film with a thickness of 35 nm, and the film was heated on a hot plate at 170°C for 15 minutes under an air atmosphere, to form a hole injection layer.

### (Formation of second layer)

The polymer compound HTL-1 and the metal complex B-G2 (polymer compound HTL-1/ metal complex B-G2 = 70% by mass/30% by mass) were dissolved at a concentration of 0.5% by mass in chlorobenzene. The resultant chlorobenzene solution was spin-coated on the hole injection layer to form a film with a thickness of 20 nm, and the film was heated on a hot plate at 180°C for 60 minutes under a nitrogen gas atmosphere, to form a second layer (hole transporting layer). By this heating, the polymer compound HTL-1 became a crosslinked state (crosslinked body).

### (Formation of first layer)

The polymer compound HP-1 and the metal complex B-G2 (polymer compound HP-1/metal complex B-G2 = 70% by mass/30% by mass) were dissolved at a concentration of 2% by mass in chlorobenzene. The resultant chlorobenzene solution was spin-coated on the second layer to form a film with a thickness of 80 nm, and the film was heated at 130°C for 10 minutes under a nitrogen gas atmosphere, to form a first layer (light emitting layer).

### (Formation of cathode)

The substrate carrying the first layer formed thereon was placed in a vapor deposition machine, and the internal pressure thereof was reduced to 1.0×10⁻⁴ Pa or less, then, as the cathode, sodium fluoride was vapor-deposited with a thickness of about 4 nm on the first layer, then, aluminum was vapor-deposited with a thickness of about 80 nm on the sodium fluoride layer. After vapor deposition, sealing was performed using a glass substrate, to fabricate a light emitting device D1.

### (Evaluation of light emitting device)

Voltage was applied to the light emitting device D1, to observe EL emission. The maximum light emission efficiency [cd/A] of the light emitting device D1, and the driving voltage [V] and the CIE chromaticity coordinate at the time were measured.

### <Comparative Example CD1> Fabrication and evaluation of light emitting device CD1

A light emitting device CD1 was fabricated in the same manner as in Example D1, except that "the polymer compound HTL-1 and the metal complex B-G2 (polymer compound HTL-1/metal complex B-G2 = 70% by mass/30% by mass) were dissolved at a concentration of 0.5% by mass in chlorobenzene" in (Formation of second layer) of Example D1 was changed to "the polymer compound HTL-1 was dissolved at a concentration of 0.7% by mass in xylene".

Voltage was applied to the light emitting device CD1, to observe EL emission. The maximum light emission efficiency [cd/A] of the light emitting device CD1, and the driving voltage [V] and the CIE chromaticity coordinate at the time were measured.

The results of Example D1 and Comparative Example CD1 are shown in Table 4. In Table 4, the difference in the maximum light emission efficiency [cd/A] denotes a difference in the maximum light emission efficiency of the light emitting device D1 with respect to the maximum light emission efficiency of the light emitting device CD1.

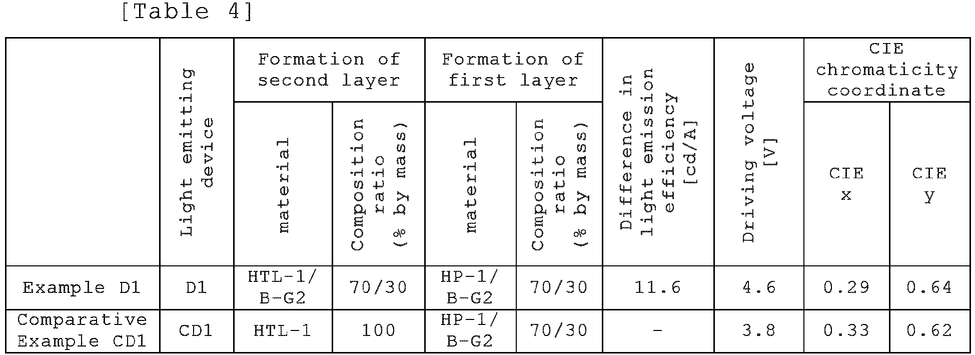

### <Comparative Example CD2> Fabrication and evaluation of light emitting device CD2

A light emitting device CD2 was fabricated in the same manner as in Example D1, except that "the polymer compound HTL-1 and the metal complex B-G2 (polymer compound HTL-1/metal complex B-G2 = 70% by mass/30% by mass) were dissolved at a concentration of 0.5% by mass in chlorobenzene" in (Formation of second layer) of Example D1 was changed to "the polymer compound HTL-1 and the metal complex B-G1 (polymer compound HTL-1/metal complex B-G1 = 70% by mass/30% by mass) were dissolved at a concentration of 0.6% by mass in xylene", and further, "the polymer compound HP-1 and the metal complex B-G2 (polymer compound HP-1/metal complex B-G2 = 70% by mass/30% by mass) were dissolved at a concentration of 2% by mass in chlorobenzene" in (Formation of first layer) of Example D1 was changed to "the polymer compound HP-1 and the metal complex B-G1 (polymer compound HP-1/metal complex B-G1 = 70% by mass/30% by mass) were dissolved at a concentration of 2.5% by mass in xylene".

Voltage was applied to the light emitting device CD2, to observe EL emission. The maximum light emission efficiency [cd/A] of the light emitting device CD2, and the driving voltage [V] and the CIE chromaticity coordinate at the time were measured.

### <Comparative Example CD3> Fabrication and evaluation of light emitting device CD3

A light emitting device CD3 was fabricated in the same manner as in Example D1, except that "the polymer compound HTL-1 and the metal complex B-G2 (polymer compound HTL-1/metal complex B-G2 = 70% by mass/30% by mass) were dissolved at a concentration of 0.5% by mass in chlorobenzene" in (Formation of second layer) of Example D1 was changed to "the polymer compound HTL-1 was dissolved at a concentration of 0.6% by mass in xylene", and further, "the polymer compound HP-1 and the metal complex B-G2 (polymer compound HP-1/metal complex B-G2 = 70% by mass/30% by mass) were dissolved at a concentration of 2% by mass in chlorobenzene" in (Formation of first layer) of Example D1 was changed to "the polymer compound HP-1 and the metal complex B-G1 (polymer compound HP-1/metal complex B-G1 = 70% by mass/30% by mass) were dissolved at a concentration of 2.5% by mass in xylene".

Voltage was applied to the light emitting device CD3, to observe EL emission. The maximum light emission efficiency [cd/A] of the light emitting device CD3, and the driving voltage [V] and the CIE chromaticity coordinate at the time were measured.

The results of Comparative Example CD2 and Comparative Example CD3 are shown in Table 5. In Table 5, the difference in the maximum light emission efficiency [cd/A] denotes a difference in the maximum light emission efficiency of the light emitting device CD2 with respect to the maximum light emission efficiency of the light emitting device CD3.

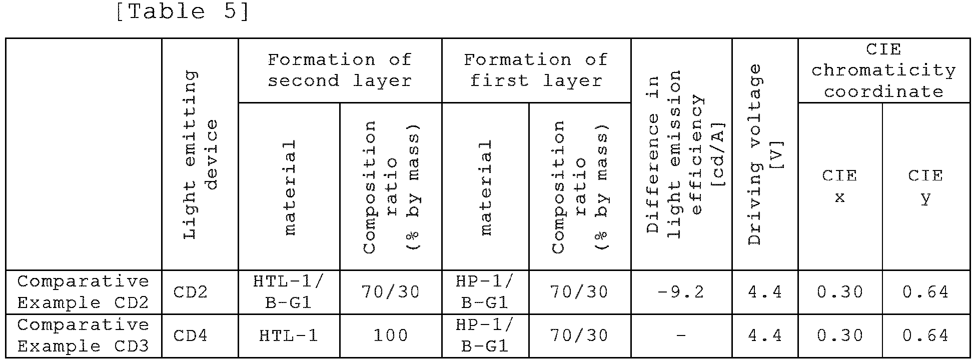

### <Example D2> Fabrication and evaluation of light emitting device D2

A light emitting device D2 was fabricated in the same manner as in Example D1, except that "the polymer compound HTL-1 and the metal complex B-G2 (polymer compound HTL-1/metal complex B-G2 = 70% by mass/30% by mass) were dissolved at a concentration of 0.5% by mass in chlorobenzene" in (Formation of second layer) of Example D1 was changed to "the polymer compound HTL-1 and the compound B-H1 (polymer compound HTL-1/compound B-H1 = 90% by mass/10% by mass) were dissolved at a concentration of 0.5% by mass in chlorobenzene", and further, "the polymer compound HP-1 and the metal complex B-G2 (polymer compound HP-1/metal complex B-G2 = 70% by mass/30% by mass) were dissolved at a concentration of 2% by mass in chlorobenzene" in (Formation of first layer) of Example D1 was changed to "the compound B-H1 and the metal complex B-G1 (compound B-H1/metal complex B-G1 = 70% by mass/30% by mass) were dissolved at a concentration of 3% by mass in chlorobenzene".

Voltage was applied to the light emitting device D2, to observe EL emission. The maximum light emission efficiency [cd/A] of the light emitting device D2, and the driving voltage [V] and the CIE chromaticity coordinate at the time were measured.

### <Example D3> Fabrication and evaluation of light emitting device D3

A light emitting device D3 was fabricated in the same manner as in Example D1, except that "the polymer compound HTL-1 and the metal complex B-G2 (polymer compound HTL-1/metal complex B-G2 = 70% by mass/30% by mass) were dissolved at a concentration of 0.5% by mass in chlorobenzene" in (Formation of second layer) of Example D1 was changed to "the polymer compound HTL-1 and the metal complex B-G1 (polymer compound HTL-1/metal complex B-G1 = 70% by mass/30% by mass) were dissolved at a concentration of 0.7% by mass in xylene", and further, "the polymer compound HP-1 and the metal complex B-G2 (polymer compound HP-1/metal complex B-G2 = 70% by mass/30% by mass) were dissolved at a concentration of 2% by mass in chlorobenzene" in (Formation of first layer) of Example D1 was changed to "the compound B-H1 and the metal complex B-G1 (compound B-H1/metal complex B-G1 = 70% by mass/30% by mass) were dissolved at a concentration of 2% by mass in chlorobenzene".

Voltage was applied to the light emitting device D3, to observe EL emission. The maximum light emission efficiency [cd/A] of the light emitting device D3, and the driving voltage [V] and the CIE chromaticity coordinate at the time were measured.

### <Example D4> Fabrication and evaluation of light emitting device D4

A light emitting device D4 was fabricated in the same manner as in Example D1, except that "the polymer compound HTL-1 and the metal complex B-G2 (polymer compound HTL-1/metal complex B-G2 = 70% by mass/30% by mass) were dissolved at a concentration of 0.5% by mass in chlorobenzene" in (Formation of second layer) of Example D1 was changed to "the polymer compound HTL-1 and the compound B-H1 (polymer compound HTL-1/compound B-H1 = 90% by mass/10% by mass) were dissolved at a concentration of 0.7% by mass in xylene", and further, "the polymer compound HP-1 and the metal complex B-G2 (polymer compound HP-1/metal complex B-G2 = 70% by mass/30% by mass) were dissolved at a concentration of 2% by mass in chlorobenzene" in (Formation of first layer) of Example D1 was changed to "the compound B-H1 and the metal complex B-G2 (compound B-H1/metal complex B-G2 = 70% by mass/30% by mass) were dissolved at a concentration of 2% by mass in chlorobenzene".

Voltage was applied to the light emitting device D4, to observe EL emission. The maximum light emission efficiency [cd/A] of the light emitting device D4, and the driving voltage [V] and the CIE chromaticity coordinate at the time were measured.

### <Example D5> Fabrication and evaluation of light emitting device D5

A light emitting device D5 was fabricated in the same manner as in Example D1, except that "the polymer compound HTL-1 and the metal complex B-G2 (polymer compound HTL-1/metal complex B-G2 = 70% by mass/30% by mass) were dissolved at a concentration of 0.5% by mass in chlorobenzene" in (Formation of second layer) of Example D1 was changed to "the polymer compound HTL-1 and the metal complex B-G2 (polymer compound HTL-1/metal complex B-G2 = 70% by mass/30% by mass) were dissolved at a concentration of 0.7% by mass in xylene", and further, "the polymer compound HP-1 and the metal complex B-G2 (polymer compound HP-1/metal complex B-G2 = 70% by mass/30% by mass) were dissolved at a concentration of 2% by mass in chlorobenzene" in (Formation of first layer) of Example D1 was changed to "the compound B-H1 and the metal complex B-G2 (compound B-H1/metal complex B-G2 = 70% by mass/30% by mass) were dissolved at a concentration of 2% by mass in chlorobenzene".

Voltage was applied to the light emitting device D5, to observe EL emission. The maximum light emission efficiency [cd/A] of the light emitting device D5, and the driving voltage [V] and the CIE chromaticity coordinate at the time were measured.

### <Comparative Example CD4> Fabrication and evaluation of light emitting device CD4

A light emitting device CD4 was fabricated in the same manner as in Example D1, except that "the polymer compound HTL-1 and the metal complex B-G2 (polymer compound HTL-1/metal complex B-G2 = 70% by mass/30% by mass) were dissolved at a concentration of 0.5% by mass in chlorobenzene" in (Formation of second layer) of Example D1 was changed to "the polymer compound HTL-1 was dissolved at a concentration of 0.5% by mass in chlorobenzene", and further, "the polymer compound HP-1 and the metal complex B-G2 (polymer compound HP-1/metal complex B-G2 = 70% by mass/30% by mass) were dissolved at a concentration of 2% by mass in chlorobenzene" in (Formation of first layer) of Example D1 was changed to "the compound B-H1 and the metal complex B-G1 (compound B-H1/metal complex B-G1 = 70% by mass/30% by mass) were dissolved at a concentration of 2% by mass in chlorobenzene".

Voltage was applied to the light emitting device CD4, to observe EL emission. The maximum light emission efficiency [cd/A] of the light emitting device CD4, and the driving voltage [V] and the CIE chromaticity coordinate at the time were measured.

The results of Examples D2 to D5 and Comparative Example CD4 are shown in Table 6. In Table 6, the difference in the maximum light emission efficiency [cd/A] denotes a difference in the maximum light emission efficiency of the light emitting devices D2 to D5 with respect to the maximum light emission efficiency of the light emitting device CD4.

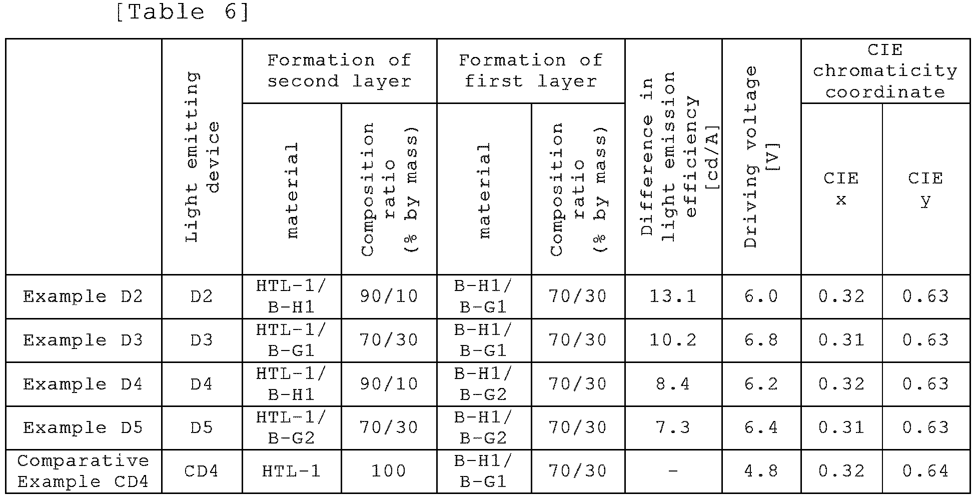

### <Example D6 and Comparative Example CD5> Fabrication and evaluation of light emitting devices D6 and CD5

Light emitting devices D6 and CD5 were fabricated in the same manner as in Example D2, except that "the polymer compound HTL-1 and the compound B-H1 (polymer compound HTL-1/compound B-H1 = 90% by mass/10% by mass)" in (Formation of second layer) of Example D2 were changed to the materials and the composition ratio (% by mass) described in Table 7, and further, "the compound B-H1 and the metal complex B-G1 (compound B-H1/metal complex B-G1 = 70% by mass/30% by mass)" in (Formation of first layer) of Example D2 were changed to the materials and the composition ratio (% by mass) described in Table 7.

Voltage was applied to the light emitting devices D6 and CD5, to observe EL emission. The maximum light emission efficiency [cd/A] of the light emitting devices D6 and CD5, and the driving voltage [V] and the CIE chromaticity coordinate at the time were measured.

The results of Example D6 and Comparative Example CD5 are shown in Table 7. In Table 7, the difference in the maximum light emission efficiency [cd/A] denotes a difference in the maximum light emission efficiency of the light emitting device D6 with respect to the maximum light emission efficiency of the light emitting device CD5.

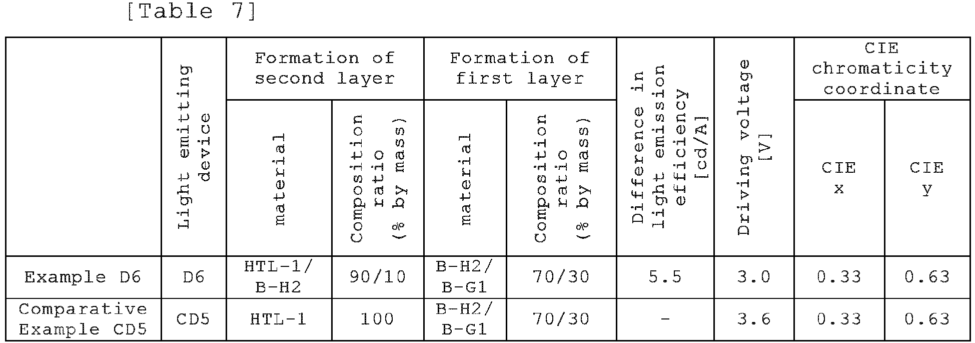

### <Example D7 and Comparative Example CD6> Fabrication and evaluation of light emitting devices D7 and CD6

Light emitting devices D7 and CD6 were fabricated in the same manner as in Example D6, except that "the polymer compound HTL-1 and the compound B-H2 (polymer compound HTL-1/compound B-H2 = 90% by mass/10% by mass)" in (Formation of second layer) of Example D6 were changed to the materials and the composition ratio (% by mass) described in Table 8.

Voltage was applied to the light emitting devices D7 and CD6, to observe EL emission. The maximum light emission efficiency [cd/A] of the light emitting devices D7 and CD6, and the driving voltage [V] and the CIE chromaticity coordinate at the time were measured.

The results of Examples D7 and Comparative Example CD6 are shown in Table 8. In Table 8, the difference in the maximum light emission efficiency [cd/A] denotes a difference in the maximum light emission efficiency of the light emitting device D7 with respect to the maximum light emission efficiency of the light emitting device CD6.

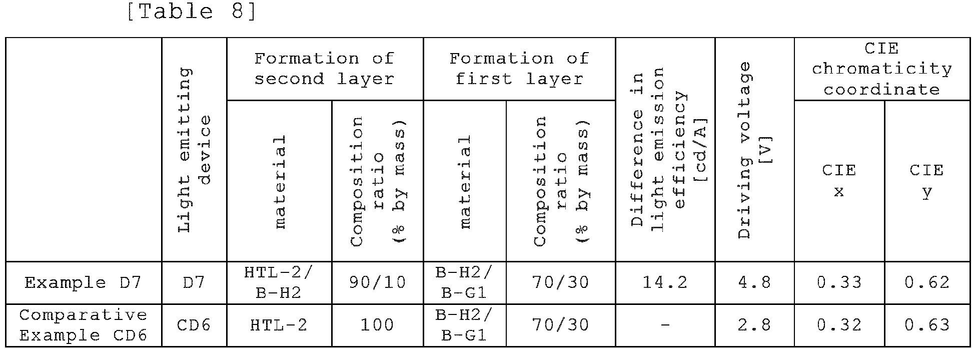

### <Example D8 and Comparative Example CD7> Fabrication and evaluation of light emitting devices D8 and CD7

Light emitting devices D8 and CD7 were fabricated in the same manner as in Example D6, except that "the polymer compound HTL-1 and the compound B-H2 (polymer compound HTL-1/compound B-H2 = 90% by mass/10% by mass)" in (Formation of second layer) of Example D6 were changed to the materials and the composition ratio (% by mass) described in Table 9.

Voltage was applied to the light emitting devices D8 and CD7, to observe EL emission. The maximum light emission efficiency [cd/A] of the light emitting devices D8 and CD7, and the driving voltage [V] and the CIE chromaticity coordinate at the time were measured.

The results of Example D8 and Comparative Example CD7 are shown in Table 9. In Table 9, the difference in the maximum light emission efficiency [cd/A] denotes a difference in the maximum light emission efficiency of the light emitting device D8 with respect to the maximum light emission efficiency of the light emitting device CD7.

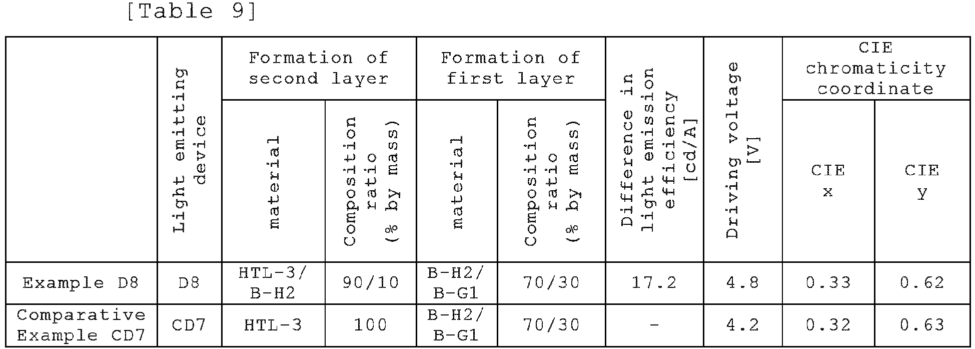

### <Examples D9 to D11 and Comparative Example CD8>

Fabrication and evaluation of light emitting devices D9 to D11 and CD8

Light emitting devices D9 to D11 and CD8 were fabricated in the same manner as in Example D6, except that "the polymer compound HTL-1 and the compound B-H2 (polymer compound HTL-1/compound B-H2 = 90% by mass/10% by mass)" in (Formation of second layer) of Example D6 were changed to the materials and the composition ratio (% by mass) described in Table 10.

Voltage was applied to the light emitting devices D9 to D11 and CD8, to observe EL emission. The maximum light emission efficiency [cd/A] of the light emitting devices D9 to D11 and CD8, and the driving voltage [V] and the CIE chromaticity coordinate at the time were measured.

The results of Examples D9 to D11 and Comparative Example CD8 are shown in Table 10. In Table 10, the difference in the maximum light emission efficiency [cd/A] denotes a difference in the maximum light emission efficiency of the light emitting devices D9 to D11 with respect to the maximum light emission efficiency of the light emitting device CD8.

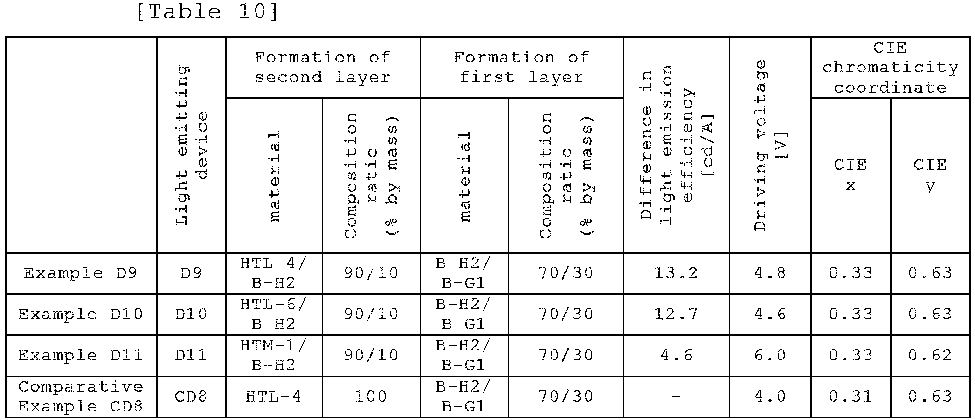

### <Example D12 and Comparative Example CD9> Fabrication and evaluation of light emitting devices D12 and CD9

Light emitting devices D12 and CD9 were fabricated in the same manner as in Example D6, except that "the polymer compound HTL-1 and the compound B-H2 (polymer compound HTL-1/compound B-H2 = 90% by mass/10% by mass)" in (Formation of second layer) of Example D6 were changed to the materials and the composition ratio (% by mass) described in Table 11.

Voltage was applied to the light emitting devices D12 and CD9, to observe EL emission. The maximum light emission efficiency [cd/A] of the light emitting devices D12 and CD9, and the driving voltage [V] and the CIE chromaticity coordinate at the time were measured.

The result of Example D12 and Comparative Example CD9 are shown in Table 11. In Table 11, the difference in the maximum light emission efficiency [cd/A] denotes a difference in the maximum light emission efficiency of the light emitting device D12 with respect to the maximum light emission efficiency of the light emitting device CD9.

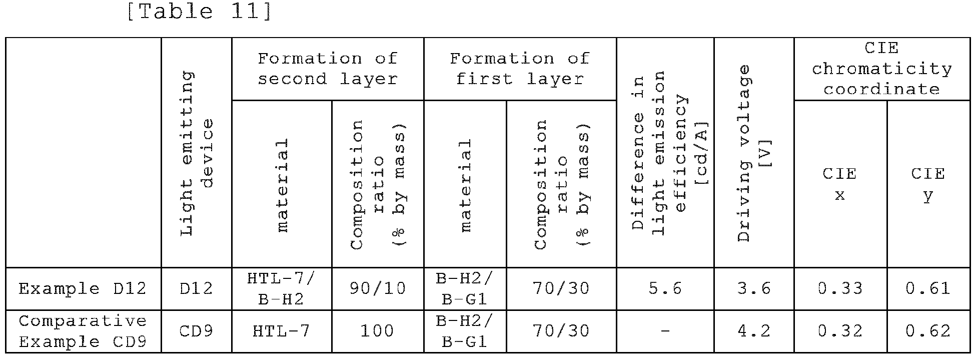

### <Example D13 and Comparative Example CD10> Fabrication and evaluation of light emitting devices D13 and CD10

Light emitting devices D13 and CD10 were fabricated in the same manner as in Example D6, except that "the polymer compound HTL-1 and the compound B-H2 (polymer compound HTL-1/compound B-H2 = 90% by mass/10% by mass)" in (Formation of second layer) of Example D6 were changed to the materials and the composition ratio (% by mass) described in Table 12.

Voltage was applied to the light emitting devices D13 and CD10, to observe EL emission. The maximum light emission efficiency [cd/A] of the light emitting devices D13 and CD10, and the driving voltage [V] and the CIE chromaticity coordinate at the time were measured.

The results of Example D13 and Comparative Example CD10 are shown in Table 12. In Table 12, the difference in the maximum light emission efficiency [cd/A] denotes a difference in the maximum light emission efficiency of the light emitting device D13 with respect to the maximum light emission efficiency of the light emitting device CD10.

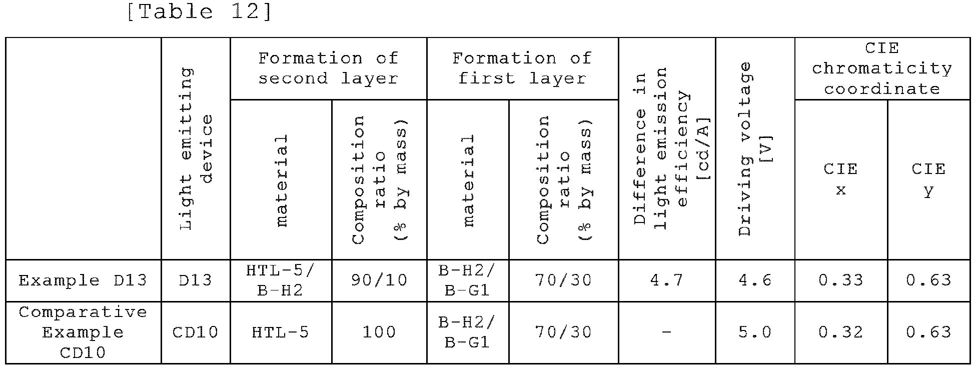

### <Example D14 and Comparative Example CD11> Fabrication and evaluation of light emitting devices D14 and CD11

Light emitting devices D14 and CD11 were fabricated in the same manner as in Example D6, except that "the polymer compound HTL-1 and the compound B-H2 (polymer compound HTL-1/compound B-H2 = 90% by mass/10% by mass)" in (Formation of second layer) of Example D6 were changed to the materials and the composition ratio (% by mass) described in Table 13.

Voltage was applied to the light emitting devices D14 and CD11, to observe EL emission. The maximum light emission efficiency [cd/A] of the light emitting devices D14 and CD11, and the driving voltage [V] and the CIE chromaticity coordinate at the time were measured.

The results of Example D14 and Comparative Example CD11 are shown in Table 13. In Table 13, the difference in the maximum light emission efficiency [cd/A] denotes a difference in the maximum light emission efficiency of the light emitting device D14 with respect to the maximum light emission efficiency of the light emitting device CD11.

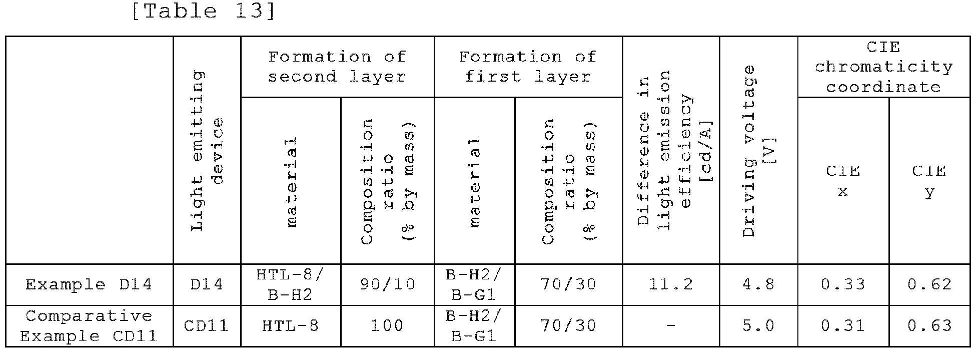

### <Examples D15 to D17 and Comparative Example CD12>

Fabrication and evaluation of light emitting devices D15 to D17 and CD12

Light emitting devices D15 to D17 and CD12 were fabricated in the same manner as in Example D2, except that "the polymer compound HTL-1 and the compound B-H1 (polymer compound HTL-1/compound B-H1 = 90% by mass/10% by mass)" in (Formation of second layer) of Example D2 were changed to the materials and the composition ratio (% by mass) described in Table 14, and further, "the compound B-H1 and the metal complex B-G1 (compound B-H1/metal complex B-G1 = 70% by mass/30% by mass)" in (Formation of first layer) of Example D2 were changed to the materials and the composition ratio (% by mass) described in Table 14.

Voltage was applied to the light emitting devices D15 to D17 and CD12, to observe EL emission. The maximum light emission efficiency [cd/A] of the light emitting devices D15 to D17 and CD12, and the driving voltage [V] and the CIE chromaticity coordinate at the time were measured.

The results of Examples D15 to D17 and Comparative Example CD12 are shown in Table 14. In Table 14, the difference in the maximum light emission efficiency [cd/A] denotes a difference in the maximum light emission efficiency of the light emitting devices D15 to D17 with respect to the maximum light emission efficiency of the light emitting device CD12.

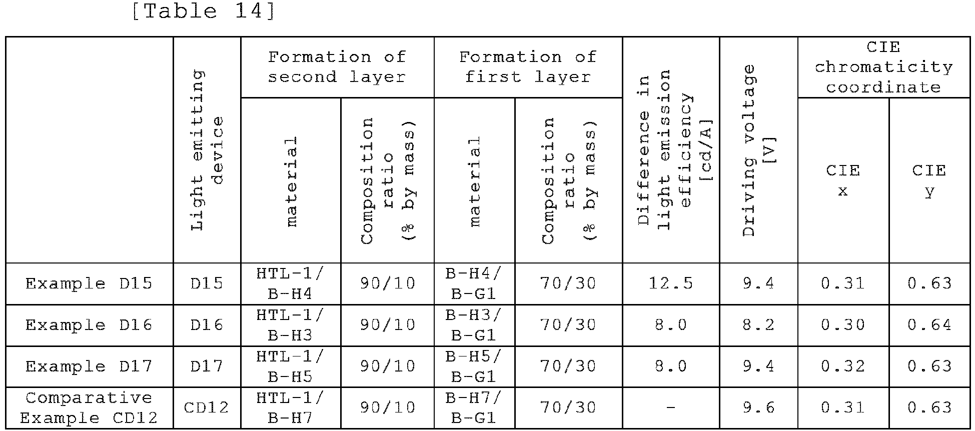

### <Example D18 and Comparative Example CD13> Fabrication and evaluation of light emitting devices D18 and CD13

Light emitting devices D18 and CD13 were fabricated in the same manner as in Example D2, except that "the polymer compound HTL-1 and the compound B-H1 (polymer compound HTL-1/compound B-H1 = 90% by mass/10% by mass)" in (Formation of second layer) of Example D2 were changed to the materials and the composition ratio (% by mass) described in Table 15, and further, "the compound B-H1 and the metal complex B-G1 (compound B-H1/metal complex B-G1 = 70% by mass/30% by mass)" in (Formation of first layer) of Example D2 were changed to the materials and the composition ratio (% by mass) described in Table 15.

Voltage was applied to the light emitting devices D18 and CD13, to observe EL emission. The maximum light emission efficiency [cd/A] of the light emitting devices D18 and CD13, and the driving voltage [V] and the CIE chromaticity coordinate at the time were measured.

The results of Example D18 and Comparative Example CD13 are shown in Table 15. In Table 15, the difference in the maximum light emission efficiency [cd/A] denotes a difference in the maximum light emission efficiency of the light emitting device D18 with respect to the maximum light emission efficiency of the light emitting device CD13.

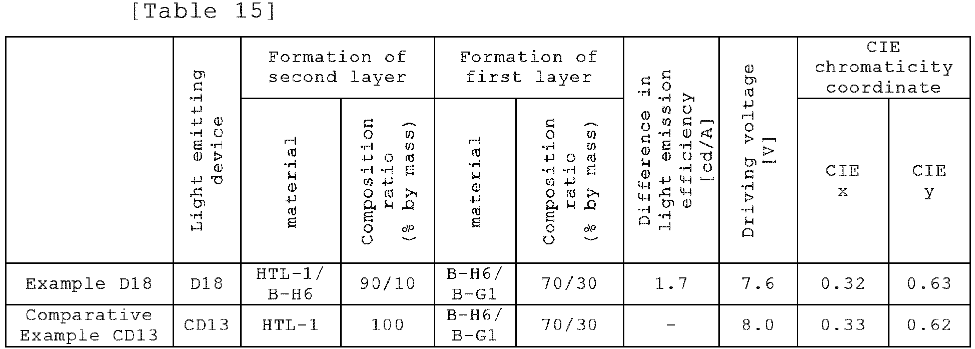

### <Comparative Examples CD14 and CD15> Fabrication and evaluation of light emitting devices CD14 and CD15

Light emitting devices CD14 and CD15 were fabricated in the same manner as in Example D2, except that "the polymer compound HTL-1 and the compound B-H1 (polymer compound HTL-1/compound B-H1 = 90% by mass/10% by mass)" in (Formation of second layer) of Example D2 were changed to the materials and the composition ratio (% by mass) described in Table 16, and further, "the compound B-H1 and the metal complex B-G1 (compound B-H1/metal complex B-G1 = 70% by mass/30% by mass)" in (Formation of first layer) of Example D2 were changed to the materials and the composition ratio (% by mass) described in Table 16.

Voltage was applied to the light emitting devices CD14 and CD15, to observe EL emission. The maximum light emission efficiency [cd/A] of the light emitting devices CD14 and CD15, and the driving voltage [V] and the CIE chromaticity coordinate at the time were measured.

The results of Comparative Examples CD14 and CD15 are shown in Table 16. In Table 16, the difference in the maximum light emission efficiency [cd/A] denotes a difference in the maximum light emission efficiency of the light emitting device CD14 with respect to the maximum light emission efficiency of the light emitting device CD15.

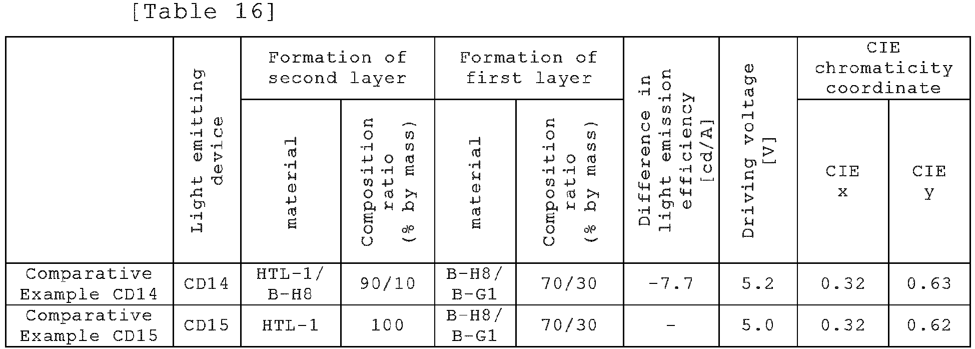

### <Example D19> Fabrication and evaluation of light emitting device D19

### (Formation of anode and hole injection layer)

An ITO film with a thickness of 45 nm was attached to a glass substrate by a sputtering method, to form an anode. On the anode, a polythiophene•sulfonic acid type hole injection agent AQ-1200 (manufactured by Plextronics) was spin-coated to form a film with a thickness of 65 nm, and the film was heated on a hot plate at 170°C for 15 minutes under an air atmosphere, to form a hole injection layer.

### (Formation of second layer)

The polymer compound HTL-1 and the compound B-H2 (polymer compound HTL-1/compound B-H2 = 90% by mass/10% by mass) were dissolved at a concentration of 0.5% by mass in chlorobenzene. The resultant chlorobenzene solution was spin-coated on the hole injection layer to form a film with a thickness of 20 nm, and the film was heated on a hot plate at 180°C for 60 minutes under a nitrogen gas atmosphere, to form a second layer (hole transporting layer). By this heating, the polymer compound HTL-1 became a cross-linked state (crosslinked body).

### (Formation of first layer)

The compound B-H2 and the metal complex B-R1 (compound B-H2/metal complex B-R1 = 90% by mass/10% by mass) were dissolved at a concentration of 3% by mass in chlorobenzene. The resultant chlorobenzene solution was spin-coated on the second layer to form a film with a thickness of 80 nm, and the film was heated at 130°C for 10 minutes under a nitrogen gas atmosphere, to form a first layer (light emitting layer).

### (Formation of cathode)

The substrate carrying the first layer formed thereon was placed in a vapor deposition machine, and the internal pressure thereof was reduced to 1.0×10⁻⁴ Pa or less, then, as the cathode, sodium fluoride was vapor-deposited with a thickness of about 4 nm on the first layer, then, aluminum was vapor-deposited with a thickness of about 80 nm on the sodium fluoride layer. After vapor deposition, sealing was performed using a glass substrate, to fabricate a light emitting device D19.

### (Evaluation of light emitting device)

Voltage was applied to the light emitting device D19, to observe EL emission. The maximum light emission efficiency [cd/A] of the light emitting device D19, and the driving voltage [V] and the CIE chromaticity coordinate at the time were measured.

### <Example D20 and Comparative Example CD16> Fabrication and evaluation of light emitting devices D20 and CD16

Light emitting devices D20 and CD16 were fabricated in the same manner as in Example D19, except that "the polymer compound HTL-1 and the compound B-H2 (polymer compound HTL-1/compound B-H2 = 90% by mass/10% by mass)" in (Formation of second layer) of Example D19 were changed to the materials and the composition ratio (% by mass) described in Table 17.

Voltage was applied to the light emitting devices D20 and CD16, to observe EL emission. The maximum light emission efficiency [cd/A] of the light emitting devices D20 and CD16, and the driving voltage [V] and the CIE chromaticity coordinate at the time were measured.

The results of Examples D19 to D20 and Comparative Example CD16 are shown in Table 17. In Table 17, the difference in the maximum light emission efficiency [cd/A] denotes a difference in the maximum light emission efficiency of the light emitting devices D19 and D20 with respect to the maximum light emission efficiency of the light emitting device CD16.

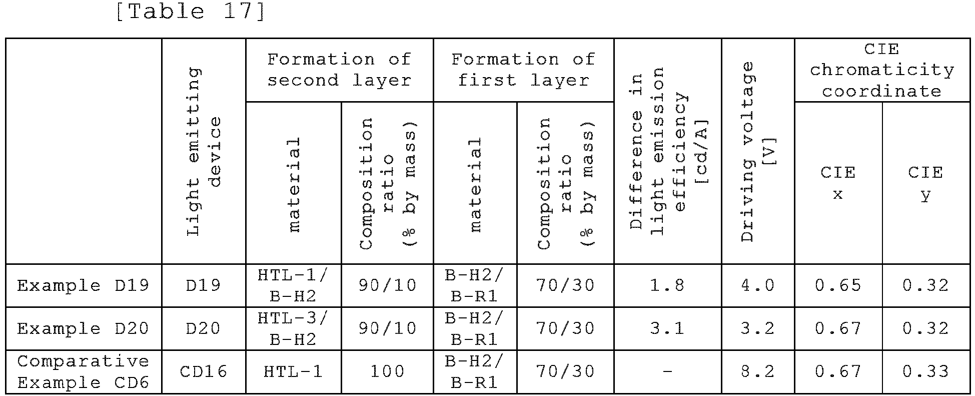

## Claims

1. A light emitting device having an anode and a cathode, and a first layer and a second layer disposed between the above-described anode and the above-described cathode, wherein
the above-described first layer is a layer containing at least one compound (B-1) selected from compounds represented by the formula (B),
the above-described second layer is a layer containing at least one compound (B-2) selected from compounds represented by the formula (B), and a crosslinked body of a compound having a cross-linkable group, and
at least one of the above-described compound (B-1) and the above-described compound (B-2) is a compound represented by the formula (B) in which n^{B1} is 1 or more: 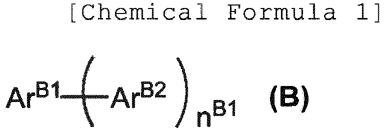 wherein,
n^{B1} represents an integer of 0 or more,
Ar^{B1} represents a group obtained by removing from an aromatic hydrocarbon n^{B1} hydrogen atoms bonding directly to carbon atoms constituting the ring, a group obtained by removing from a heterocyclic compound n^{B1} hydrogen atoms bonding directly to atoms constituting the ring or a group obtained by removing n^{B1} hydrogen atoms from a metal complex represented by the formula (1), and these groups optionally have a substituent, when a plurality of the substituents are present, they may be the same or different and may be combined together to form a ring together with atoms to which they are attached,
Ar^{B2} represents a group represented by the formula (D-A) or a group represented by the formula (D-B), when a plurality of Ar^{B2} are present, they may be the same or different, 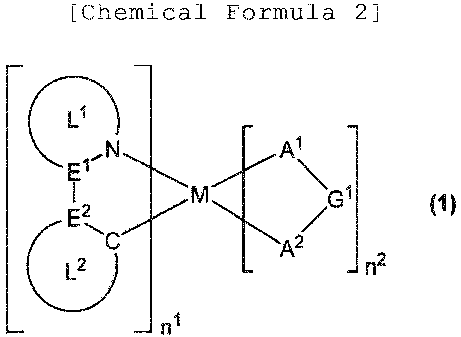 wherein,
M represents a rhodium atom, a palladium atom, an iridium atom or a platinum atom,
n¹ represents an integer of 1 or more, and n² represents an integer of 0 or more, providing n¹+n² is 3 when M is a rhodium atom or an iridium atom, while n¹+n² is 2 when M is a palladium atom or a platinum atom,
E¹ and E² each independently represent a carbon atom or a nitrogen atom, when a plurality of E¹ and E² are present, they may be the same or different at each occurrence,
Ring L¹ represents an aromatic hetero ring, and the ring optionally has a substituent, when a plurality of the substituents are present, they may be the same or different and may be combined together to form a ring together with atoms to which they are attached, when a plurality of Ring L¹ are present, they may be the same or different,
Ring L² represents an aromatic hydrocarbon ring or an aromatic hetero ring, and these rings optionally have a substituent, when a plurality of the substituents are present, they may be the same or different and may be combined together to form a ring together with atoms to which they are attached, when a plurality of Ring L² are present, they may be the same or different,
a substituent which Ring L¹ optionally has and a substituent which Ring L² optionally has may be the same or different and may be combined together to form a ring together with atoms to which they are attached,
A¹-G¹-A² represents an anionic bidentate ligand, A¹ and A² each independently represent a carbon atom, an oxygen atom or a nitrogen atom, and these atoms may be ring-constituent atoms, G¹ represents a single bond, or an atomic group constituting a bidentate ligand together with A¹ and A², when a plurality of A¹-G¹-A² are present, they may be the same or different, 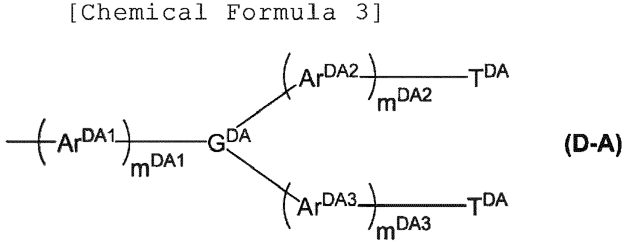 wherein,
m^{DA1} represents an integer of 1 or more,
m^{DA2} and m^{DA3} each independently represent an integer of 0 or more,
G^{DA} represents a nitrogen atom, an aromatic hydrocarbon group or a hetero ring group, and these groups optionally have a substituent, when a plurality of the substituents are present, they may be the same or different and may be combined together to form a ring together with atoms to which they are attached,
Ar^{DA1}, Ar^{DA2} and Ar^{DA3} each independently represent an arylene group or a divalent hetero ring group, and these groups optionally have a substituent, when a plurality of the substituents are present, they may be the same or different and may be combined together to form a ring together with atoms to which they are attached, when a plurality of Ar^{DA1}, Ar^{DA2} and Ar^{DA3} are present, they may be the same or different at each occurrence,
T^{DA} represents an aryl group or a monovalent hetero ring group, and these groups optionally have a substituent, when a plurality of the substituents are present, they may be the same or different and may be combined together to form a ring together with atoms to which they are attached, a plurality of T^{DA} may be the same or different, 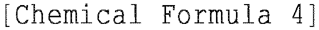 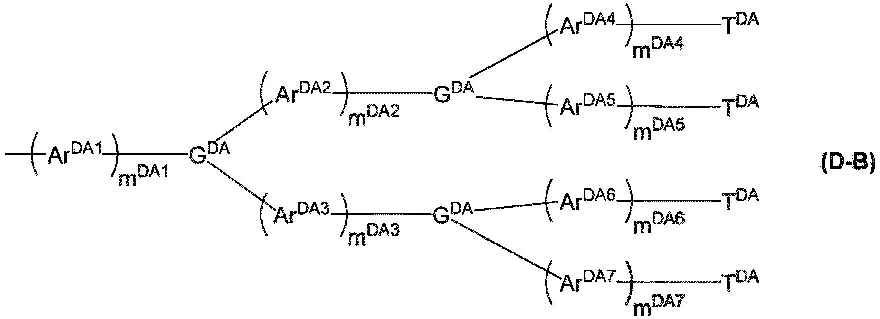 wherein,
m^{DA1} represents an integer of 1 or more,
m^{DA2} , m^{DA3}, m^{DA4}, m^{DA5}, m^{DA6} and m^{DA7} each independently represent an integer of 0 or more,
G^{DA} represents a nitrogen atom, an aromatic hydrocarbon group or a hetero ring group, and these groups optionally have a substituent, when a plurality of the substituents are present, they may be the same or different and may be combined together to form a ring together with atoms to which they are attached, a plurality of G^{DA} may be the same or different,
Ar^{DA1}, Ar^{DA2}, Ar^{DA3}, Ar^{DA4}, Ar^{DA5}, Ar^{DA6} and Ar^{DA7} each independently represent an arylene group or a divalent hetero ring group, and these groups optionally have a substituent, when a plurality of the substituents are present, they may be the same or different and may be combined together to form a ring together with atoms to which they are attached, when a plurality of Ar^{DA1}, Ar^{DA2}, Ar^{DA3}, Ar^{DA4}, Ar^{DA5}, Ar^{DA6} and Ar^{DA7} are present, they may be the same or different at each occurrence,
T^{DA} represents an aryl group or a monovalent hetero ring group, and these groups optionally have a substituent, when a plurality of the substituents are present, they may be the same or different and may be combined together to form a ring together with atoms to which they are attached, and a plurality of T^{DA} may be the same or different.

2. The light emitting device according to claim 1, wherein the above-described G^{DA} is an aromatic hydrocarbon group or a hetero ring group, and these groups optionally have a substituent.

3. The light emitting device according to claim 2, wherein the above-described G^{DA} is a group obtained by removing from a monocyclic, bicyclic or a tricyclic aromatic hydrocarbon three hydrogen atoms bonding directly to carbon atoms constituting the ring or a group obtained by removing from a monocyclic, bicyclic or a tricyclic heterocyclic compound three hydrogen atoms bonding directly to atoms constituting the ring, and these groups optionally have a substituent.

4. The light emitting device according to claim 3, wherein the above-described G^{DA} is a group obtained by removing from benzene, pyridine, diazabenzene, triazine or carbazole three hydrogen atoms bonding directly to atoms constituting the ring, and the group optionally has a substituent.

5. The light emitting device according to any one of claims 1 to 4, wherein the above-described Ar^{DA1} is a group obtained by removing from a monocyclic, bicyclic or a tricyclic aromatic hydrocarbon two hydrogen atoms bonding directly to carbon atoms constituting the ring or a group obtained by removing from a monocyclic, bicyclic or a tricyclic heterocyclic compound two hydrogen atoms bonding directly to atoms constituting the ring, and these groups optionally have a substituent.

6. The light emitting device according to any one of claims 1 to 4, wherein the above-described Ar^{DA1} is an arylene group, and the group optionally has a substituent.

7. The light emitting device according to claim 6, wherein the above-described Ar^{DA1} is a phenylene group optionally having a substituent.

8. The light emitting device according to any one of claims 1 to 7, wherein the above-described Ar^{B1} is a group obtained by removing from a monocyclic or bicyclic to pentacyclic aromatic hydrocarbon n^{B1} hydrogen atoms bonding directly to carbon atoms constituting the ring or a group obtained by removing from a monocyclic or bicyclic to pentacyclic heterocyclic compound n^{B1} hydrogen atoms bonding directly to atoms constituting the ring, and these groups optionally have a substituent.

9. The light emitting device according to claim 8, wherein the above-described Ar^{B1} is a group obtained by removing from a monocyclic or bicyclic to pentacyclic heterocyclic compound n^{B1} hydrogen atoms bonding directly to atoms constituting the ring, and the group optionally has a substituent.

10. The light emitting device according to any one of claims 1 to 7, wherein the above-described Ar^{B1} is a group obtained by removing n^{B1} hydrogen atoms from the above-described metal complex represented by the formula (1), and the group optionally has a substituent.

11. The light emitting device according to claim 10, wherein the above-described Ring L¹ is an aromatic hetero ring containing a 5-membered ring or an aromatic hetero ring containing a 6-membered ring, and the ring optionally has a substituent, and, the above-described Ring L² is an aromatic hydrocarbon ring containing a 5-membered ring or 6-membered ring or an aromatic hetero ring containing a 5-membered ring or 6-membered ring, and these rings optionally have a substituent.

12. The light emitting device according to claim 10 or 11, wherein the above-described Ring L¹ is a pyridine ring, a diazabenzene ring, an azanaphthalene ring, a diazanaphthalene ring, a diazole ring or a triazole ring, and these rings optionally have a substituent, and, the above-described Ring L² is a benzene ring, a pyridine ring or a diazabenzene ring, and these rings optionally have a substituent.

13. The light emitting device according to any one of claims 1 to 12, wherein the above-described compound having a cross-linkable group is a polymer compound containing a constitutional unit having a cross-linkable group.

14. The light emitting device according to claim 13, wherein the above-described constitutional unit having a cross-linkable group is a constitutional unit represented by the formula (Z) or a constitutional unit represented by the formula (Z'): 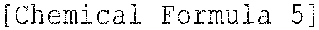 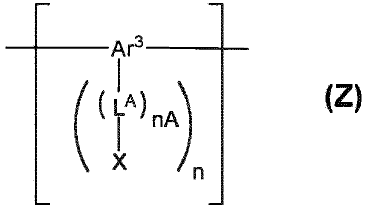 wherein,
n represents an integer of 1 or more,
nA represents an integer of 0 or more, when a plurality of nA are present, they may be the same or different,
Ar³ represents a hydrocarbon group, a hetero ring group, or a group in which at least one hydrocarbon group and at least one hetero ring group are bonded directly, and these groups optionally have a substituent, when a plurality of the substituents are present, they may be the same or different and may be combined together to form a ring together with atoms to which they are attached,
L^{A} represents an alkylene group, a cycloalkylene group, an arylene group, a divalent hetero ring group, a group represented by -N(R')-, an oxygen atom or a sulfur atom, and these groups optionally have a substituent, when a plurality of the substituents are present, they may be the same or different and may be combined together to form a ring together with atoms to which they are attached, R' represents a hydrogen atom, an alkyl group, a cycloalkyl group, an aryl group or a monovalent hetero ring group, and these groups optionally have a substituent, when a plurality of the substituents are present, they may be the same or different and may be combined together to form a ring together with atoms to which they are attached, when a plurality of L^{A} are present, they may be the same or different,
X represents a cross-linkable group, when a plurality of X are present, they may be the same or different, 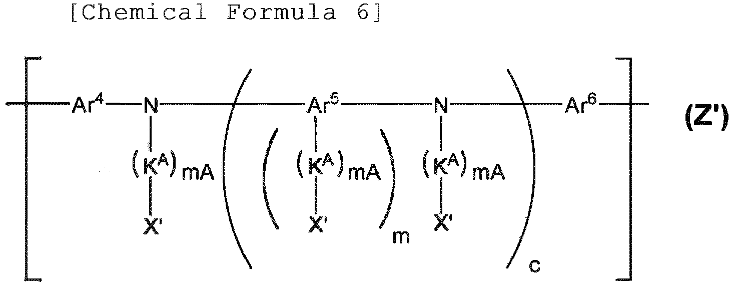 wherein,
mA, m and c each independently represent an integer of 0 or more, when a plurality of mA are present, they may be the same or different, when a plurality of m are present, they may be the same or different,
Ar⁵ represents a hydrocarbon group, a hetero ring group, or a group in which at least one hydrocarbon group and at least one hetero ring group are bonded directly, and these groups optionally have a substituent, when a plurality of the substituents are present, they may be the same or different and may be combined together to form a ring together with atoms to which they are attached, when a plurality of Ar⁵ are present, they may be the same or different,
Ar⁴ and Ar⁶ each independently represent an arylene group or a divalent hetero ring group, and these groups optionally have a substituent, when a plurality of the substituents are present, they may be the same or different and may be combined together to form a ring together with atoms to which they are attached,
K^{A} represents an alkylene group, a cycloalkylene group, an arylene group, a divalent hetero ring group, a group represented by -N(R")-, an oxygen atom or a sulfur atom, and these groups optionally have a substituent, when a plurality of the substituents are present, they may be the same or different and may be combined together to form a ring together with atoms to which they are attached, R" represents a hydrogen atom, an alkyl group, a cycloalkyl group, an aryl group or a monovalent hetero ring group, and these groups optionally have a substituent, when a plurality of the substituents are present, they may be the same or different and may be combined together to form a ring together with atoms to which they are attached, when a plurality of K^{A} are present, they may be the same or different,
X' represents a hydrogen atom, a cross-linkable group, an alkyl group, a cycloalkyl group, an aryl group or a monovalent hetero ring group, and these groups optionally have a substituent, when a plurality of the substituents are present, they may be the same or different and may be combined together to form a ring together with atoms to which they are attached, when a plurality of X' are present, they may be the same or different, providing at least one X' is a cross-linkable group.

15. The light emitting device according to claim 13 or 14, wherein the above-described polymer compound further contains at least one constitutional unit selected from the group consisting of a constitutional unit represented by the formula (X) and a constitutional unit represented by the formula (Y): 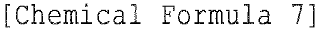 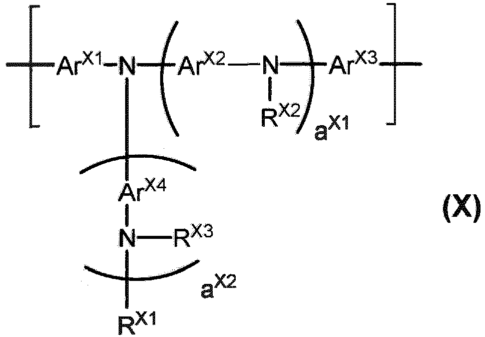 wherein,
a^{X1} and a^{X2} each independently represent an integer of 0 or more,
Ar^{X1} and Ar^{X3} each independently represent an arylene group or a divalent hetero ring group, and these groups optionally have a substituent, when a plurality of the substituents are present, they may be the same or different and may be combined together to form a ring together with atoms to which they are attached,
Ar^{X2} and Ar^{X4} each independently represent an arylene group, a divalent hetero ring group, or a divalent group in which at least one arylene group and at least one divalent hetero ring group are bonded directly, and these groups optionally have a substituent, when a plurality of the substituents are present, they may be the same or different and may be combined together to form a ring together with atoms to which they are attached, when a plurality of Ar^{X2} are present, they may be the same or different at each occurrence, when a plurality of Ar^{X4} are present, they may be the same or different at each occurrence,
R^{X1}, R^{X2} and R^{X3} each independently represent a hydrogen atom, an alkyl group, a cycloalkyl group, an aryl group or a monovalent hetero ring group, and these groups optionally have a substituent, when a plurality of the substituents are present, they may be the same or different and may be combined together to form a ring together with atoms to which they are attached, when a plurality of R^{X2} are present, they may be the same or different, when a plurality of R^{X3} are present, they may be the same or different, 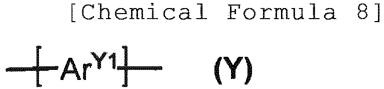 wherein, Ar^{Y1} represents an arylene group, a divalent hetero ring group, or a divalent group in which at least one arylene group and at least one divalent hetero ring group are bonded directly, and these groups optionally have a substituent, when a plurality of the substituents are present, they may be the same or different and may be combined together to form a ring
together with atoms to which they are attached.

16. The light emitting device according to any one of claims 1 to 12, wherein the above-described compound having a cross-linkable group is a compound represented by the formula (Z"): 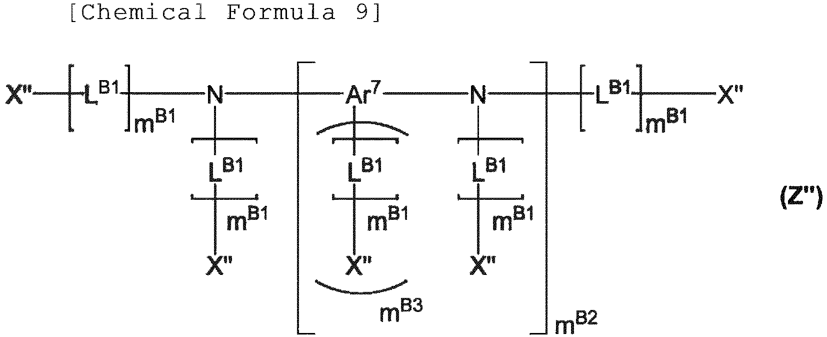 wherein,
m^{B1}, m^{B2} and m^{B3} each independently represent an integer of 0 or more, when a plurality of m^{B1} are present, they may be the same or different, when a plurality of m^{B3} are present, they may be the same or different,
Ar⁷ represents a hydrocarbon group, a hetero ring group, or a group in which at least one hydrocarbon group and at least one hetero ring group are bonded directly, and these groups optionally have a substituent, when a plurality of the substituents are present, they may be the same or different and may be combined together to form a ring together with atoms to which they are attached, when a plurality of Ar⁷ are present, they may be the same or different,
L^{B1} represents an alkylene group, a cycloalkylene group, an arylene group, a divalent hetero ring group, a group represented by -N(R‴)-, an oxygen atom or a sulfur atom, and these groups optionally have a substituent, when a plurality of the substituents are present, they may be the same or different and may be combined together to form a ring together with atoms to which they are attached, R‴ represents a hydrogen atom, an alkyl group, a cycloalkyl group, an aryl group or a monovalent hetero ring group, and these groups optionally have a substituent, when a plurality of the substituents are present, they may be the same or different and may be combined together to form a ring together with atoms to which they are attached, when a plurality of L^{B1} are present, they may be the same or different, X" represents a cross-linkable group, a hydrogen atom, an alkyl group, a cycloalkyl group, an aryl group or a monovalent hetero ring group, and these groups optionally have a substituent, when a plurality of the substituents are present, they may be the same or different and may be combined together to form a ring together with atoms to which they are attached, a plurality of X" may be the same or different, providing at least one of a plurality of X" is a cross-linkable group.

17. The light emitting device according to any one of claims 1 to 16, wherein the above-described cross-linkable group is at least one cross-linkable group selected from Group A of cross-linkable group:
(Group A of cross-linkable group) 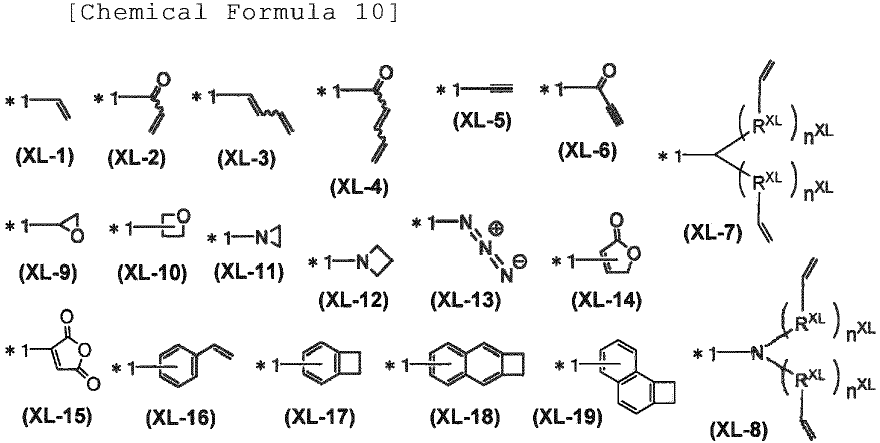 wherein, R^{XL} represents a methylene group, an oxygen atom or a sulfur atom, and n^{XL} represents an integer of 0 to 5, when a plurality of R^{XL} are present, they may be the same or different, when a plurality of n^{XL} are present, they may be the same or different, *1 represents a bonding position, these cross-linkable groups optionally have a substituent, and when a plurality of the substituents are present, they may be the same or different and may be combined together to form a ring together with atoms to which they are attached.

18. The light emitting device according to any one of claims 1 to 17, wherein the above-described first layer contains two or more of the above-described compounds (B-1).

19. The light emitting device according to any one of claims 1 to 18, wherein the above-described first layer further contains a polymer compound containing at least one constitutional unit selected from the group consisting of a constitutional unit represented by the formula (X) and a constitutional unit represented by the formula (Y): 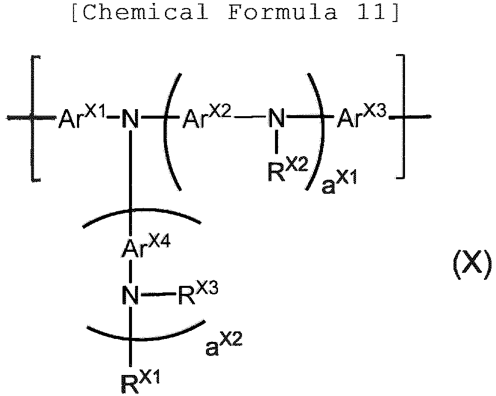 wherein,
a^{X1} and a^{X2} each independently represent an integer of 0 or more,
Ar^{X1} and Ar^{X3} each independently represent an arylene group or a divalent hetero ring group, and these groups optionally have a substituent, when a plurality of the substituents are present, they may be the same or different and may be combined together to form a ring together with atoms to which they are attached,
Ar^{X2} and Ar^{X4} each independently represent an arylene group, a divalent hetero ring group, or a divalent group in which at least one arylene group and at least one divalent hetero ring group are bonded directly, and these groups optionally have a substituent, when a plurality of the substituents are present, they may be the same or different and may be combined together to form a ring together with atoms to which they are attached, when a plurality of Ar^{X2} are present, they may be the same or different at each occurrence, when a plurality of Ar^{X4} are present, they may be the same or different at each occurrence,
R^{X1}, R^{X2} and R^{X3} each independently represent a hydrogen atom, an alkyl group, a cycloalkyl group, an aryl group or a monovalent hetero ring group, and these groups optionally have a substituent, when a plurality of the substituents are present, they may be the same or different and may be combined together to form a ring together with atoms to which they are attached, when a plurality of R^{X2} are present, they may be the same or different, when a plurality of R^{X3} are present, they may be the same or different, 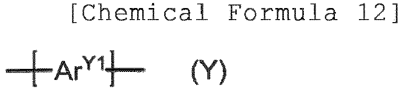 wherein, Ar^{Y1} represents an arylene group, a divalent hetero ring group, or a divalent group in which at least one arylene group and at least one divalent hetero ring group are bonded directly, and these groups optionally have a substituent, when a plurality of the substituents are present, they may be the same or different and may be combined together to form a ring together with atoms to which they are attached.

20. The light emitting device according to any one of claims 1 to 19, wherein at least one of the above-described compound (B-1) and at least one of the above-described compound (B-2) are the same.

21. The light emitting device according to any one of claims 1 to 20, wherein at least one of the above-described compound (B-1) is the above-described compound in which n^{B1} is 1 or more.

22. The light emitting device according to any one of claims 1 to 21, wherein the above-described first layer further contains at least one selected from the group consisting of a hole transporting material, a hole injection material, an electron transporting material, an electron injection material, a light emitting material and an antioxidant.

23. The light emitting device according to any one of claims 1 to 22, wherein the above-described first layer and the above-described second layer are adjacent.

24. The light emitting device according to any one of claims 1 to 23, wherein the above-described second layer is disposed between the above-described anode and the above-described first layer.

25. A composition containing at least one compound (B-2) selected from compounds represented by the formula (B), and a crosslinked body of a compound having a cross-linkable group: 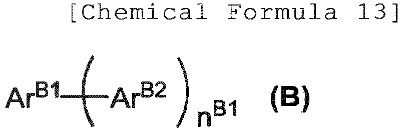 wherein,
n^{B1} represents an integer of 1 or more,
Ar^{B1} represents a group obtained by removing from an aromatic hydrocarbon n^{B1} hydrogen atoms bonding directly to carbon atoms constituting the ring, a group obtained by removing from a heterocyclic compound n^{B1} hydrogen atoms bonding directly to atoms constituting the ring or a group obtained by removing n^{B1} hydrogen atoms from a metal complex represented by the formula (1), and these groups optionally have a substituent, when a plurality of the substituents are present, they may be the same or different and may be combined together to form a ring together with atoms to which they are attached,
Ar^{B2} represents a group represented by the formula (D-A) or a group represented by the formula (D-B), when a plurality of Ar^{B2} are present, they may be the same or different, 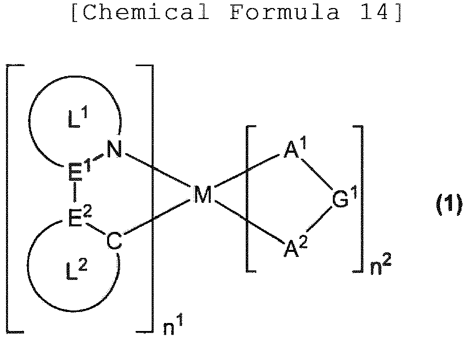 wherein,
M represents a rhodium atom, a palladium atom, an iridium atom or a platinum atom,
n¹ represents an integer of 1 or more, and n² represents an integer of 0 or more, providing n¹+n² is 3 when M is a rhodium atom or an iridium atom, while n¹+n² is 2 when M is a palladium atom or a platinum atom,
E¹ and E² each independently represent a carbon atom or a nitrogen atom, when a plurality of E¹ and E² are present, they may be the same or different at each occurrence,
Ring L¹ represents an aromatic hetero ring, and the ring optionally has a substituent, when a plurality of the substituents are present, they may be the same or different and may be combined together to form a ring together with atoms to which they are attached, when a plurality of Ring L¹ are present, they may be the same or different,
Ring L² represents an aromatic hydrocarbon ring or an aromatic hetero ring, and these rings optionally have a substituent, when a plurality of the substituents are present, they may be the same or different and may be combined together to form a ring together with atoms to which they are attached, when a plurality of Ring L² are present, they may be the same or different,
a substituent which Ring L¹ optionally has and a substituent which Ring L² optionally has may be the same or different and may be combined together to form a ring together with atoms to which they are attached,
A¹-G¹-A² represents an anionic bidentate ligand, A¹ and A² each independently represent a carbon atom, an oxygen atom or a nitrogen atom, and these atoms may be ring-constituent atoms, G¹ represents a single bond, or an atomic group constituting a bidentate ligand together with A¹ and A², when a plurality of A¹-G¹-A² are present, they may be the same or different, 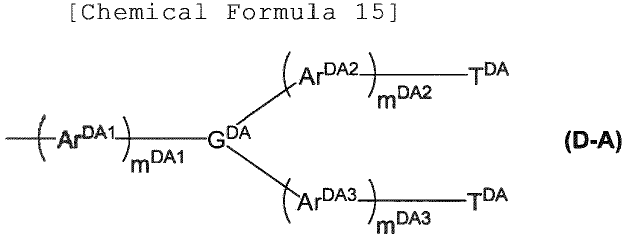 wherein,
m^{DA1} represents an integer of 1 or more,
m^{DA2} and m^{DA3} each independently represent an integer of 0 or more,
G^{DA} represents a nitrogen atom, an aromatic hydrocarbon group or a hetero ring group, and these groups optionally have a substituent, when a plurality of the substituents are present, they may be the same or different and may be combined together to form a ring together with atoms to which they are attached,
Ar^{DA1}, Ar^{DA2} and Ar^{DA3} each independently represent an arylene group or a divalent hetero ring group, and these groups optionally have a substituent, when a plurality of the substituents are present, they may be the same or different and may be combined together to form a ring together with atoms to which they are attached, when a plurality of Ar^{DA1}, Ar^{DA2} and Ar^{DA3} are present, they may be the same or different at each occurrence,
T^{DA} represents an aryl group or a monovalent hetero ring group, and these groups optionally have a substituent, when a plurality of the substituents are present, they may be the same or different and may be combined together to form a ring together with atoms to which they are attached, a plurality of T^{DA} may be the same or different, 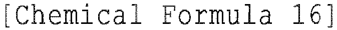 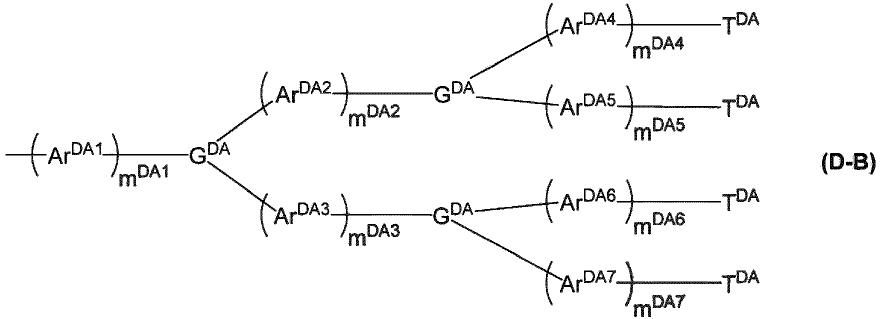 wherein,
m^{DA1} represents an integer of 1 or more,
m^{DA2}, m^{DA3}, m^{DA4}, m^{DA5}, m^{DA6} and m^{DA7} each independently represent an integer of 0 or more,
G^{DA} represents a nitrogen atom, an aromatic hydrocarbon group or a hetero ring group, and these groups optionally have a substituent, when a plurality of the substituents are present, they may be the same or different and may be combined together to form a ring together with atoms to which they are attached, a plurality of G^{DA} may be the same or different,
Ar^{DA1}, Ar^{DA2}, Ar^{DA3}, Ar^{DA4}, Ar^{DA5}, Ar^{DA6} and Ar^{DA7} each independently represent an arylene group or a divalent hetero ring group, and these groups optionally have a substituent, when a plurality of the substituents are present, they may be the same or different and may be combined together to form a ring together with atoms to which they are attached, when a plurality of Ar^{DA1}, Ar^{DA2}, Ar^{DA3}, Ar^{DA4} , Ar^{DA5}, Ar^{DA6} and Ar^{Da7} are present, they may be the same or different at each occurrence,
T^{DA} represents an aryl group or a monovalent hetero ring group, and these groups optionally have a substituent, when a plurality of the substituents are present, they may be the same or different and may be combined together to form a ring together with atoms to which they are attached, and a plurality of T^{DA} may be the same or different; wherein
when Ar^{B1} is a group obtained by removing from an aromatic hydrocarbon n^{B1} hydrogen atoms bonding directly to carbon atoms constituting the ring or a group obtained by removing from a heterocyclic compound n^{B1} hydrogen atoms bonding directly to atoms constituting the ring, the compound represented by the formula (B) is a compound constituted of typical elements.
